(19) Europäisches Patentamt
European Patent Office
Office européen des brevets

(11) EP 4 509 128 A1

(12) **EUROPEAN PATENT APPLICATION**
published in accordance with Art. 153(4) EPC

(43) Date of publication:
**19.02.2025 Bulletin 2025/08**

(21) Application number: **23788606.4**

(22) Date of filing: **12.04.2023**

(51) International Patent Classification (IPC):
***A61K 35/28*** *(2015.01)* ***A61K 31/7105*** *(2006.01)*
***A61K 38/19*** *(2006.01)* ***A61K 38/20*** *(2006.01)*
***A61K 45/06*** *(2006.01)* ***A61P 35/00*** *(2006.01)*
***C12N 5/073*** *(2010.01)*

(52) Cooperative Patent Classification (CPC):
**A61K 31/7105; A61K 35/28; A61K 38/19; A61K 38/20; A61K 45/06; A61P 35/00; C12N 5/06**

(86) International application number:
**PCT/KR2023/004969**

(87) International publication number:
**WO 2023/200257 (19.10.2023 Gazette 2023/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.04.2022 KR 20220045356**

(71) Applicant: **Inexoplat, Inc.**
**Incheon 21984 (KR)**

(72) Inventors:
- **KIM, Sung Hwan**
**Incheon 21986 (KR)**
- **KIM, Yoon Young**
**Incheon 22000 (KR)**
- **LEE, Jae Hwan**
**Incheon 21986 (KR)**
- **YU, Ho Sung**
**Incheon 22009 (KR)**
- **KO, Min Sung**
**Incheon 21670 (KR)**
- **LEE, Hyeon Ji**
**Incheon 21997 (KR)**
- **HONG, Eun Be**
**Yongin-si Gyeonggi-do 16874 (KR)**

(74) Representative: **HGF**
**HGF Limited**
**1 City Walk**
**Leeds LS11 9DX (GB)**

Remarks:
The complete document including Reference Table(s) and the Sequence Listing(s) can be downloaded from the EPO website

# (54) EXTRACELLULAR VESICLES ISOLATED FROM STEM CELLS, AND USES THEREOF

(57) The present invention provides extracellular vesicles isolated from stem cells treated with zymosan, Poly LC, and monophosphoryl lipid A (MPLA), their use for treatment of cancer, and a method for preparing the same. The extracellular vesicles according to the present invention contain various miRNAs and cytokines related to anticancer effects, and have tumor growth inhibitory effects. In addition, since the extracellular vesicles exhibit a synergistic effect in cancer treatment when administered in combination with an immune checkpoint inhibitor, a pharmaceutical composition for cancer treatment containing the extracellular vesicles as an active ingredient is highly likely to be effectively useful in cancer treatment by increasing immune activity in the body.

[Figure 7]

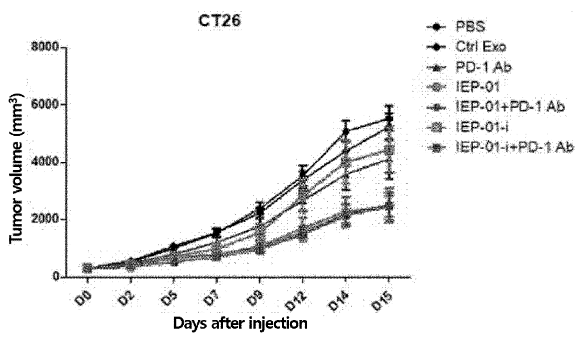


EP 4 509 128 A1

## Description

### Technical Field

The present invention relates to novel extracellular vesicles isolated from stem cells treated with chemical substances, their use for treatment of cancer, and a method for preparing the same.

### Background Art

Cancer is one of the major causes of death and morbidity worldwide, and various types of cancer treatment methods such as chemotherapy, surgery, and radiation therapy are being developed. However, these treatment methods have disadvantages such as causing serious side effects such as killing healthy cells or developing resistance. Another major disadvantage is that there is no effective therapeutic agent for solid cancer.

Recently, methods for treating cancer by activating the body's immune response have been reported. For example, various immune anticancer agents such as an immune checkpoint inhibitor, a chimeric antigen receptor-T cell (CAR-T), a T cell receptor modified T cell (TCR-T), a tumor infiltrating lymphocyte (TIL), a bispecific T-cell engager (BiTE), a tumor vaccine, and an oncolytic virus are being developed. However, unlike blood cancers, the effects of existing immune anticancer agents are insufficient in various solid cancers, because the tumor microenvironment surrounding cancer cells limits the function of immune cells.

Accordingly, methods for enhancing anticancer function by remodeling the tumor microenvironment are being developed, and these can be used alone or in combination with existing immune anticancer agents. In addition, these immune anticancer agents can be used in combination with various cancer treatment methods such as existing chemotherapy, surgery, and radiation therapy.

Meanwhile, extracellular vesicles are nanometer-sized vesicles composed of lipid bilayers, and comprise various types of physiologically active substances such as proteins, lipids, RNA (including microRNA, mRNA, and lncRNA) that exist inside the parent cell, and are known to play a very important role in the transmission of information between cells. In particular, extracellular vesicles isolated from stem cells are attracting attention as an alternative that can overcome various shortcomings of cell therapeutic agents because they have the therapeutic efficacy of stem cells while being cell-free.

Extracellular vesicles are released or secreted from various cells of animals, plants, fungi, algae, etc., and are dispersed, suspended, precipitated, floating, or mixed in biological solutions such as cell culture solution, cell culture supernatant, stem cell culture solution, stem cell culture supernatant, whole blood, serum, umbilical cord blood, plasma, ascites fluid, brain and cerebrospinal fluid, placental extract, and bone marrow aspirate. As such, extracellular vesicles present in biological solutions can be separated and purified by various separation methods.

Currently known methods for isolating extracellular vesicles include ultracentrifugation, density gradient centrifugation, ultrafiltration, tangential flow filtration, size exclusion chromatography, ion exchange chromatography, immunoaffinity capture, microfluidics-based isolation, exosome precipitation, total exosome isolation kit, or polymer-based precipitation.

In addition, it is known that the characteristics and functions of extracellular vesicles differ depending on the origin and type of the cell, the state of cell growth, proliferation, differentiation, death, immortalization, etc., and the characteristics and functions may also vary depending on the culture state of the cell, culture conditions, and the isolation method and conditions of the extracellular vesicles, etc. Therefore, specific culture conditions can be used to prepare extracellular vesicles with specific physiological activities.

The incorporation of a specific substance into extracellular vesicles can be achieved by culturing cells genetically engineered to produce recombinant extracellular vesicles in a cell culture medium in which a drug is dissolved, or by placing the isolated recombinant extracellular vesicles in a solvent in which a therapeutic agent for a specific disease is dissolved and subjecting them to ultrasonification to reconstruct the extracellular vesicles. When selectively loading a drug into extracellular vesicles, the isolated extracellular vesicles can be simply mixed with the drug in an appropriate solvent or medium and stirred for an appropriate amount of time (Sun et al., Mol. Ther. 18(9): 1606-1614, 2010). In the case of a hydrophilic drug such as nucleic acids, they can be incorporated into extracellular vesicles using electroporation.

Meanwhile, extracellular vesicles have the advantage of being safe because they do not induce immune responses and have a very low risk of tumorigenesis because they cannot self-replicate, unlike cells. In particular, extracellular vesicles secreted from mesenchymal stem cells have the characteristic of effectively reaching tumor cells and being less toxic even after repeated administration. In addition, extracellular vesicles are much smaller than the cell size of 10,000 nm to 20,000 nm, so they can be administered without the risk of embolism, a potential risk factor for cell therapeutic agents. Accordingly, attempts are being made to apply the extracellular vesicles to cancer treatment.

Therefore, there is an urgent need to develop a method for preparing extracellular vesicles that can effectively apply the characteristics of these extracellular vesicles to anticancer agents.

## Detailed Description of Invention

### Technical Problem

Accordingly, the present inventors conducted research to develop extracellular vesicles as an anticancer agent. As a result, the present inventors found that extracellular vesicles isolated from cells treated with zymosan, Poly I:C, and monophosphoryl lipid A (MPLA) comprise various miRNAs, mRNAs, and cytokines and exhibit excellent anticancer effects. Based on the above, the present inventors completed the present invention.

### Solution to Problem

In order to achieve the above object, in one aspect of the present invention, there is provided an extracellular vesicle comprising various miRNAs, mRNAs, and cytokines.

In another aspect of the present invention, there is provided an extracellular vesicle isolated from a human-derived cell treated with zymosan, Poly I:C, and monophosphoryl lipid A (MPLA).

In another aspect of the present invention, there is provided a pharmaceutical composition for preventing or treating cancer, comprising the extracellular vesicle as an active ingredient.

In another aspect of the present invention, there is provided a pharmaceutical composition for combination administration for preventing or treating cancer, comprising the extracellular vesicle and an immune anticancer agent as active ingredients.

### Effects of Invention

The extracellular vesicles isolated from cells treated with zymosan, Poly I:C, and monophosphoryl lipid A (MPLA) comprise various miRNAs, mRNAs, and cytokines related to anticancer effects, and have tumor growth inhibitory effects. In addition, the extracellular vesicles exhibit a synergistic effect in cancer treatment when administered in combination with an immune checkpoint inhibitor. Therefore, a pharmaceutical composition for treating cancer, comprising the extracellular vesicles as an active ingredient can be effectively utilized in the prevention or treatment of various cancers.

### Brief Description of Drawings

Figure 1 is a graph showing the results obtained by measuring the number of particles by size of the exosomes isolated from mesenchymal stem cells treated with the chemical substance cocktail of the present invention (CHEM Exo) and the exosomes isolated from untreated mesenchymal stem cells (CTRL Exo) using an NTA equipment.

Figure 2 shows the result obtained by performing Western blot to confirm the expression pattern of extracellular vesicle-specific markers of the exosomes isolated from mesenchymal stem cells treated with the chemical substance cocktail of the present invention (CHEM Exo).

Figure 3 shows the result obtained by performing ELISA to analyze cytokines present in the exosomes isolated from mesenchymal stem cells treated with the chemical substance cocktail of the present invention (CHEM Exo).

Figure 4 shows the result obtained by performing microarray to confirm miRNAs with a specifically increased expression level in the exosomes isolated from mesenchymal stem cells treated with the chemical substance cocktail of the present invention (CHEM Exo).

Figure 5 shows the results obtained by measuring the amount of nitric oxide production in a mouse leukemia cell line (RAW 264.7) following the treatment of the exosomes isolated from mesenchymal stem cells treated with the chemical substance cocktail of the present invention. The data represent the mean ± standard error of three samples, and ***$p < 0.001$ is a value derived from one-way ANOVA after multiple comparisons with Student's two-tailed t test.

Figure 6 shows the result obtained by performing RT-qPCR to measure the intensity of expression of proinflammatory cytokines and inflammation-related proteins in a mouse leukemia cell line (RAW 264.7) following the treatment of the exosomes isolated from mesenchymal stem cells treated with the chemical substance cocktail of the present invention. The data represent the mean ± standard error of three samples, and ***$p < 0.001$ is a value derived from one-way ANOVA after multiple comparisons with Student's two-tailed t test.

Figure 7 shows the results obtained by measuring the change in tumor volume by group in a mouse colorectal cancer model following the administration of the exosomes isolated from mesenchymal stem cells or mesenchymal stem cells differentiated from induced pluripotent stem cells treated with the chemical substance cocktail of the present invention (IEP-01 or IEP-01-i) alone or the administration in combination with an anti-PD-1 antibody. The error bars in the graph represent the Standard Error Mean (SEM).

Figure 8 shows the results obtained by measuring the change in tumor volume by subject in a mouse colorectal cancer model following the administration of the exosomes isolated from mesenchymal stem cells or mesenchymal stem cells differentiated from induced pluripotent stem cells treated with the chemical substance cocktail of the present invention (IEP-01 or IEP-01-i) alone or the administration in combination with an anti-PD-1 antibody.

Figure 9 shows the results obtained by measuring the body weight by subject in a mouse colorectal cancer model following the administration of the exosomes isolated from mesenchymal stem cells or mesenchymal stem cells differentiated from induced pluripotent stem cells treated with the chemical substance cocktail of the present invention (IEP-01 or IEP-01-i) alone or the administration in combination with an anti-PD-1 antibody.

Figure 10 shows the results obtained by measuring the change in tumor weight by group in a mouse colorectal cancer model following the administration of the exosomes isolated from mesenchymal stem cells or mesenchymal stem cells differentiated from induced pluripotent stem cells treated with the chemical substance cocktail of the present invention (IEP-01 or IEP-01-i) alone or the administration in combination with an anti-PD-1 antibody. The error bars in the graph represent the Standard Error Mean (SEM). *$p < 0.05$, **$p < 0.01$ are values derived from one-way ANOVA after multiple comparisons with Student's one-tailed t test

Figure 11 is a photograph of extracted tumors by subject in a mouse colorectal cancer model following the administration of the exosomes isolated from mesenchymal stem cells or mesenchymal stem cells differentiated from induced pluripotent stem cells treated with the chemical substance cocktail of the present invention (IEP-01 or IEP-01-i) alone or the administration in combination with an anti-PD-1 antibody.

Figure 12 shows the results obtained by measuring the level of IL-6 mRNA expression in a mouse leukemia cell line (RAW 264.7) by qRT-PCR after treatment with the exosomes isolated from mesenchymal stem cells treated with the chemical substance cocktail of the present invention alone (IEP-01) or after treatment with POLY I:C alone.

Figure 13a is a graph showing the level of IL-6 expression after treating Raw264.7 cells with exosomes obtained from untreated WJ-MSCs (naive), exosomes obtained from WJ-MSCs treated with 3-chem or 2-chem (IEP-01 or IEP-01'), or LPS for 24 hours.

Figure 13b is a graph showing the level of IL-1β expression after treating Raw264.7 cells with exosomes obtained from untreated WJ-MSCs (naive), exosomes obtained from WJ-MSCs treated with 3-chem or 2-chem (IEP-01 or IEP-01'), or LPS for 24 hours.

Figure 13c is a graph showing the level of iNOS2 expression after treating Raw264.7 cells with exosomes obtained from untreated WJ-MSCs (naive), exosomes obtained from WJ-MSCs treated with 3-chem or 2-chem (IEP-01 or IEP-01'), or LPS for 24 hours.

Figure 13d is a graph showing the level of IL-6 expression after treating Raw264.7 cells with exosomes obtained from untreated ciMSCs (naive), exosomes obtained from ciMSCs treated with 3-chem or 2-chem (IEP-01i or IEP-01i'), or LPS for 24 hours.

Figure 13e is a graph showing the level of iNOS2 expression after treating Raw264.7 cells with exosomes obtained from untreated ciMSCs (naive), exosomes obtained from ciMSCs treated with 3-chem or 2-chem (IEP-01i or IEP-01i'), or LPS for 24 hours.

Figure 13f is a graph showing the level of IL-6 expression after treating Raw264.7 cells with exosomes obtained from untreated HEK293T cell line (naive), exosomes obtained from HEK293T cell line treated with 3-chem or 2-chem (IEP-01h or IEP-01h'), or LPS for 24 hours.

Figure 13g is a graph showing the level of IL-1β expression after treating Raw264.7 cells with exosomes obtained from untreated HEK293T cell line (naive), exosomes obtained from HEK293T cell line treated with 3-chem or 2-chem (IEP-01h or IEP-01h'), or LPS for 24 hours.

Figure 14a is a graph showing the degree of uptake of ciMSC-derived exosomes by PBMC and immune cell types.

Figure 14b is a graph showing the degree of uptake of ciMSC-derived exosomes by PBMC and immune cell types as measured by PKH67 MFI.

Figure 14c is a graph showing the degree of each immune cell that was positive for PKH67 as %.

Figure 15a shows the results of flow cytometry analysis for the degree of macrophage activation as measured by the level of CD80 expression after treating Raw264.7 cells with IEP-01i'.

Figure 15b is a graph showing the degree of macrophage activation as measured by the level of CD80 expression after treating Raw264.7 cells with IEP-01i'.

Figure 15c is a graph showing the results obtained by confirming the expression level of CD80, an M1 macrophage marker, by flow cytometry analysis when Raw264.7 cells were treated with IL-4 to induce differentiation into M2 macrophages and then treated with IEP-01i'.

Figure 15d is a graph showing the results obtained by confirming the expression level of CD80, an M1 macrophage marker, when Raw264.7 cells were treated with IL-4 to induce differentiation into M2 macrophages and then treated with IEP-01i'.

Figure 16a is a graph showing the ratio of dendritic cells in each experimental group as a ratio of CD11c+ cells.

Figure 16b shows that PKH-positive dendritic cells were observed in dendritic cells treated with PKH-labeled

exosomes.

Figure 16c confirms PKH-positive dendritic cells in dendritic cells treated with PKH-labeled naive exosomes.

Figure 16d confirms PKH-positive dendritic cells in dendritic cells treated with PKH-labeled IEP-01i'.

Figure 16e is a graph showing the expression level of CD80, an activation marker of dendritic cells, in each experimental group.

Figure 16f is a graph showing the expression level of CD80, an activation marker of dendritic cells, and the PKH-positive ratio in each experimental group.

Figure 16g is a graph showing the expression level of CD86, an activation marker of dendritic cells, in each experimental group.

Figure 16h is a graph showing the expression level of CD86, an activation marker of dendritic cells, and the PKH-positive ratio in each experimental group.

Figure 16i is a graph showing the expression level of MHC class I of dendritic cells in each experimental group.

Figure 16j is a graph showing the expression level of MHC class I of dendritic cells and the PKH-positive ratio in each experimental group.

Figure 16k is a graph showing the expression level of MHC class II of dendritic cells in each experimental group.

Figure 16l is a graph showing the expression level of MHC class II of dendritic cells and the PKH-positive ratio in each experimental group.

Figure 17a is a graph showing the results obtained by analyzing IP-10 included in naive exosomes or IEP-01i' obtained from ciMSCs.

Figure 17b is a graph showing the results obtained by analyzing IL-6 included in naive exosomes or IEP-01i' obtained from ciMSCs.

Figure 17c is a graph showing the results obtained by analyzing MCP-1 included in naive exosomes or IEP-01i' obtained from ciMSCs.

Figure 17d is a graph showing the results obtained by analyzing IL-8 included in naive exosomes or IEP-01i' obtained from ciMSCs.

Figure 17e is a graph showing the results obtained by analyzing TGF-$\beta$1 included in naive exosomes or IEP-01i' obtained from ciMSCs.

Figure 18a is a schematic diagram of an experiment to confirm the anticancer activity of IEP-01i' against colorectal cancer.

Figure 18b is a graph showing the change in tumor size when IEP-01i' was administered at a low dose (LD), a mid dose (MD), or a high dose (HD) to a CT26 colorectal cancer animal model.

Figure 18c is a graph showing the tumor growth of each subject in a group treated with PBS in a CT26 colorectal cancer animal model.

Figure 18d is a graph showing the tumor growth of each subject in a group treated with IEP-01i' at a low dose once every two days in a CT26 colorectal cancer animal model.

Figure 18e is a graph showing the tumor growth of each subject in a group treated with IEP-01i' at a mid dose once every two days in a CT26 colorectal cancer animal model.

Figure 18f is a graph showing the tumor growth of each subject in a group treated with IEP-01i' at a high dose once every two days in a CT26 colorectal cancer animal model.

Figure 18g is a graph showing the tumor growth of each subject in a group treated with IEP-01i' at a low dose every day in a CT26 colorectal cancer animal model.

Figure 18h is a graph showing the tumor growth of each subject in a group treated with IEP-01i' at a mid dose every day in a CT26 colorectal cancer animal model.

Figure 18i is a graph showing the tumor growth of each subject in a group treated with IEP-01 i' at a high dose every day in a CT26 colorectal cancer animal model.

Figure 19a is a schematic diagram of an experiment to confirm the anticancer activity of IEP-01 and IEP-01i against colorectal cancer.

Figure 19b is a graph showing the results obtained by analyzing the tumor size of each subject by date after treatment with an anti-PD-1 antibody, IEP-01, or IEP-01i alone or in combination in a CT26 colorectal cancer animal model.

Figure 19c is a graph showing the results obtained by analyzing the tumor size of each subject by date after treatment with PBS in a CT26 colorectal cancer animal model.

Figure 19d is a graph showing the results obtained by analyzing the tumor size of each subject by date after treatment with Ctrl Exo in a CT26 colorectal cancer animal model.

Figure 19e is a graph showing the results obtained by analyzing the tumor size of each subject by date after treatment with an anti-PD-1 antibody in a CT26 colorectal cancer animal model.

Figure 19f is a graph showing the results obtained by analyzing the tumor size of each subject by date after treatment with IEP-01 in a CT26 colorectal cancer animal model.

Figure 19g is a graph showing the results obtained by analyzing the tumor size of each subject by date after treatment

with IEP-01 and an anti-PD-1 antibody in combination in a CT26 colorectal cancer animal model.

Figure 19h is a graph showing the results obtained by analyzing the tumor size of each subject by date after treatment with IEP-01i in a CT26 colorectal cancer animal model.

Figure 19i is a graph showing the results obtained by analyzing the tumor size of each subject by date after treatment with IEP-01i and an anti-PD-1 antibody in combination in a CT26 colorectal cancer animal model.

Figure 20a is a schematic diagram of an experiment to confirm the anticancer activity of IEP-01i in a B 16F 10 melanoma model.

Figure 20b is a graph showing the tumor size of B 16F 10 by date after treatment with an anti-PD-1 antibody or IEP-01i alone or in combination, respectively, three times a week for a total of two weeks in a B 16F 10 melanoma animal model.

Figure 20c is a graph showing the results obtained by analyzing the tumor size of each subject by date after treatment with PBS in a B 16F 10 melanoma animal model.

Figure 20d is a graph showing the results obtained by analyzing the tumor size of each subject by date after treatment with an anti-PD-1 antibody in a B16F10 melanoma animal model.

Figure 20e is a graph showing the results obtained by analyzing the tumor size of each subject by date after treatment with IEP-01i in a B 16F 10 melanoma animal model.

Figure 20f is a graph showing the results obtained by analyzing the tumor size of each subject by date after treatment with IEP-01i and an anti-PD-1 antibody in combination in a B 16F 10 melanoma animal model.

Figure 21a is a schematic diagram of an experiment to confirm the anticancer activity of IEP-01i' in a Pan02 pancreatic cancer animal model.

Figure 21b is a graph showing the results obtained by analyzing the tumor size by date after treatment with an anti-PD-1 antibody or IEP-01i' alone or in combination, respectively, three times a week for a total of two weeks in a Pan02 pancreatic cancer animal model.

Figure 21c is a graph showing the results obtained by analyzing the tumor size of each subject by date after treatment with the control group in a Pan02 pancreatic cancer animal model.

Figure 21d is a graph showing the results obtained by analyzing the tumor size of each subject by date after treatment with an anti-PD-1 antibody in a Pan02 pancreatic cancer animal model.

Figure 21e is a graph showing the results obtained by analyzing the tumor size of each subject by date after treatment with IEP-01i' in a Pan02 pancreatic cancer animal model.

Figure 21f is a graph showing the results obtained by analyzing the tumor size of each subject by date after treatment with IEP-01i' and an anti-PD-1 antibody in combination in a Pan02 pancreatic cancer animal model.

Figure 22a is a graph showing the results obtained by analyzing whether poly IC is detected in samples obtained before and after purifying the cell culture medium spiked with polyI:C, using HPLC.

Figure 22b is a graph showing the results obtained by analyzing whether poly IC is detected in samples before and after the IEP-01 purification process, using HPLC.

Figure 22c is a graph showing HPLC analysis results for whether poly IC is detected in IEP-01 or naive exosomes.

Figure 23 is a heat map showing miRNAs that are specifically abundant in IEP-01i or IEP-01i' compared to naive exosomes by analyzing miRNAs included in naive exosomes (Ctrl Exo), IEP-01i (3 chem Exo), or IEP-01i' (2 chem Exo) using microarray.

Mode for Carrying out the Invention

**Novel extracellular vesicles isolated from mesenchymal stem cells**

In one aspect of the present invention, there is provided an extracellular vesicle isolated from a human-derived cell, wherein the extracellular vesicle

i) comprises at least one miRNA selected from the group consisting of NovelmiRNA-67, hsa-miR-199a-3p, NovelmiRNA-298, hsa-miR-145-5p, NovelmiRNA-281, NovelmiRNA-109, hsa-miR-432-5p, NovelmiRNA-230, NovelmiRNA-88, hsa-miR-200b-3p, NovelmiRNA-169, hsa-miR-200a-3p, hsa-miR-487b-3p, hsa-miR-214-3p, NovelmiRNA-208, NovelmiRNA-164, NovelmiRNA-225, NovelmiRNA-174, hsa-miR-708-5p, hsa-miR-124-3p, NovelmiRNA-150, hsa-miR-135a-5p, NovelmiRNA-64, NovelmiRNA-92, NovelmiRNA-168, NovelmiRNA-22, NovelmiRNA-35, NovelmiRNA-161, hsa-miR-3940-3p, hsa-miR-219a-3p, hsa-miR-212-3p, NovelmiRNA-76, hsa-miR-323a-3p, hsa-miR-654-3p, hsa-miR-204-5p, hsa-miR-136-3p, NovelmiRNA-39, NovelmiRNA-175, hsa-miR-134-5p, hsa-miR-454-3p, NovelmiRNA-286, hsa-miR-542-3p, hsa-miR-433-3p, hsa-miR-574-5p, hsa-miR-6086, hsa-miR-4701-3p, hsa-miR-3149, hsa-miR-4743-5p, hsa-miR-8075, hsa-miR-6880-5p, hsa-miR-3148, hsa-miR-6780b-5p, hsa-miR-6846-5p, hsa-mir-320e, hsa-miR-32-3p, hsa-miR-642a-3p, hsa-miR-4505, hsa-miR-595, hsa-miR-4487, hsa-miR-4721, hsa-miR-7151-3p, hsa-miR-4484, hsa-miR-642b-3p, ENSG00000239154,

hsa-miR-4706, hsa-miR-6126, hsa-miR-378h, hsa-miR-6849-5p, hsa-miR-7844-5p, hsa-miR-4535, hsa-mir-9-1, hsa-miR-4459, hsa-miR-4443, hsa-miR-6732-5p, hsa-miR-3064-5p, hsa-miR-3178, hsa-miR-3135b, hsa-miR-4717-3p, hsa-miR-206, hsa-miR-4529-3p, and hsa-miR-3613-5p, and

ii) comprises at least one mRNA selected from the group consisting of MED13L, lnc-TOMM20L-1, lnc-CDK5R1-1, ETV3, SLC35G1, SMARCA4, ACAT2, ADAM12, ARFGEF1, CELF3, DNAJB5, lnc-WRNIP1-2, ACTL8, lnc-TM2D3-2, GCN1L1, LOC101929337, FAM217A, XLOC_l2_013467, SNHG4, NUDT13, MCTS1, ATG4D, NPEPL1, YEATS2, SPEN, GGT6, lnc-RP11-770J1.4.1-1, MIAT, lnc-HMCN1-2, RETN, lnc-EXT1-2, LIN54, C17orf74, SLC12A2, TTC28, ADRA2A, NLGN2, lnc-FBXO25-4, LOC100506603, LOC399886, RCC2, KIAA0040, HOXB9, JMJD8, LINC01146, lnc-GIT1-1, VRTN, NHP2, KIF26A, HAUS5, lnc-SOD2-2, APOL3, lnc-POLR2L-1, lnc-SUPT3H-1, lnc-XRN2-3, RTP2, TERT, T'NFRSF 12A, LOC645427, lnc-CTR9-5, LOC100507420, OPA3, XLOC_l2_014245, lnc-NR5A1-1, DMTN, SMPD4, TXLNB, SPRR1A, DISP1, LOC100130285, lnc-SLC17A9-1, lnc-SELV.1-1, XLOC_l2_000018, lnc-ROM1-2, lnc-RABEPK-1, lnc-RRP8-1, lnc-RP11-234B24.6.1-1, lnc-TMEM189-UBE2V1-2, LAMTOR2, BTK, lnc-BSND-1, C2orf71, lnc-C17orf63-2, lnc-CHGA-1, ABCD1, NCAN, C9orf106, AGAP2, EMC6, CD86, LOC100129175, lnc-PHYHD1-1, GIGYF2, RNF151, BAGE, ADRA2C, lnc-SLC43A1-1, ENTPD8, NYAP1, lnc-GLIPR1L1-1, CCNT2-AS1, NAVI, TBX21, GNAZ, DNM3OS, PRKAG2-AS1, lnc-MFSD9-4, lnc-RPGRIP1-2, ABCC6, LRFN3, lnc-COL1A1-2, lnc-PPAPDC3-2, CTU1, lnc-PABPC4-1, LOC100507165, LOC102724861, SPTSSB, lnc-C20orf96-4, DCDC5, lnc-CRYL1-1, LOC100506114, SHARPIN, ASMTL-AS1, HMOX1, SHARPIN, LOC101928207, TRAIP, lnc-ANTXRL-2, LINC01135, BZRAP1, LOC101926983, WDR6, C19orf81, NAMA, LIM2, FAM155A-IT1, lnc-RPP30-2, ZNF264, MALAT1, HTT-AS, ZDHHC22, SEPHS2, ABTB1, lnc-RP11-791J7.2.1-1, CCDC28B, LOC284263, lnc-PPIC-2, CDC42BPB, C9orf173-AS1, C9orf139, FGD5, SP6, RCOR2, lnc-AC016251.1-2, lnc-PRKAA2-1, RANBP3, lnc-SH2B3-1, XLOC_l2_011901, CACNA1H, LINC01314, lnc-VAPB-1, ARHGAP23, ATG9A, GM2A, XLOC_l2_013547, lnc-SLC25A26-1, TMEM240, CLSTN3, ATG16L2, MMP11, TSSK3, lnc-SOX6-1, EDEM3, LOC101927210, C14orf169, EBLN2, lnc-XRN1-1, lnc-FTSJD2-1, MDFI, LOC102546226, SLC5A10, SENP7, lnc-C17orf97-4, ARNTL, lnc-RTL1-1, ZNF713, XLOC_l2_005871, QSOX1, SP2-AS1, ARSA, PHF7, GS1-24F4.2, LRIT1, PLLP, SLC4A11, C11orf95, FAM110B, IER5, LINC01508, lnc-GTPBP8-1, LOC400958, WFDC21P, lnc-CPXM2-2, P2RX6, SLC22A7, lnc-PPP2R2C-1, lnc-GATA5-2, MARVELD1, lnc-TRIM41-3, PRSS8, SEMA6C, SLC48A1, RPL28, BGN, BZW1, lnc-C6orf221-2, SLC20A2, HYALP1, SNAR-G1, SEC22A, TIMM8B, ATAD1, FNDC7, B3GNT8, STARD7-AS1, TMEM100, LARP6, HELZ2, TMX2, lnc-EPHB6-1, PSMC2, lnc-LINC00273-1, TNFRSF25, BCL3, LOC101929450, LOC102724416, FLII, WAC, PPP6R1, LOC100507377, LRRFIP2, CCNT2-AS1, lnc-SCAMP5-1, BRD1, lnc-ELAVL4-3, CHRDL1, lnc-FUT8-1, lnc-DNAJC7-1, LINC01264, LMO2, LINC01197, LOC101927533, REP15, C1orf226, lnc-STEAP1B-1, SNORD3B-1, lnc-TMEM135-2, lnc-FAM160A1-1, GOLGA2P5, TMEM132C, GPX3, lnc-DCAF4L2-2, XLOC_l2_014217, PPT1, RPA4, KCTD5, lnc-UQCRFS1-9, MTUS2-AS1, CHKA, TBC1D31, SNORD38A, lnc-RP11-322L20.1.1-7, RAD51, MEIOB, lnc-ADAMTS14-1, NOS2, and PCDHA13, and

iii) comprises at least one cytokine selected from the group consisting of IL-6, IL-8, MCP-3, MCP-1, IP-10, IL-1$\beta$, and RANTES.

The human-derived cell may include a cell, a cell line, or a stem cell derived from human tissue, but is not limited thereto.

The cell derived from human tissue may be a cell derived from the human heart, stomach, colorectal cancer, small intestine, lung, liver, kidney or uterus. In addition, the cell derived from human tissue may be a human-derived immortalized cell line.

The stem cell may be a mesenchymal stem cell, a hematopoietic stem cell, a neural stem cell, an embryonic stem cell, or an induced pluripotent stem cell. Specifically, the stem cell may be a mesenchymal stem cell.

As used herein, the term "mesenchymal stem cell (MSC)" refers to stem cells that exist in cartilage, bone tissue, adipose tissue, the stroma of bone marrow, and the like, differentiated from the mesoderm formed by division of a fertilized egg, and may include mesenchymal stem cells of animals, including mammals, for example humans. Mesenchymal stem cells maintain sternness and self-renewal and have the ability to differentiate into various cells, including chondrocytes, osteoblasts, muscle cells, and adipocytes, and can be extracted from Wharton's jelly, bone marrow, adipose tissue, umbilical cord blood, synovial membrane, bone tissue (trabecular bone), muscle, infrapatellar fat pad, peripheral blood, liver, teeth, hair follicles, and the like. Mesenchymal stem cells have immunomodulatory abilities that suppress the activity and proliferation of T lymphocytes and B lymphocytes, inhibit the activity of natural killer cells (NK cells), and regulate the functions of dendritic cells and macrophages, and are therefore cells capable of allotransplantation and xenotransplantation.

The mesenchymal stem cell may be derived from umbilical cord, umbilical cord blood, Wharton's jelly, bone marrow, fat, muscle, nerve, skin, amniotic membrane, or placenta, or may be differentiated from an induced pluripotent stem cell, but is not limited thereto. Specifically, the mesenchymal stem cell may be derived from Wharton's jelly.

As used herein, the term "derived cell", for example, "derived mesenchymal stem cell", etc., refers to a cell

substantially isolated from the tissue from which the cell originates, for example, Wharton's jelly, or a cell differentiated from an induced stem cell.

As used herein, the term "induced pluripotent stem cell (iPSC)" refers to a cell that has been induced to have pluripotency like a stem cell by introducing a specific factor into a differentiated somatic cell to dedifferentiate.

As used herein, the term "differentiation" refers to a phenomenon in which structures or functions become specialized while cells divide and proliferate and grow, that is, cells, tissues, and the like of a living organism change in shape or function to perform their respective tasks.

As used herein, the term "extracellular vesicle" refers to a nano-sized particle naturally secreted by living cells, packaged in a lipid bilayer, and may be a concept that encompasses all vesicles (exosomes, microvesicles, multivesicles, and extracellular vesicle-like vesicles) that serve as information transfer mechanisms between cells and have a composition similar to that of extracellular vesicles.

As used herein, the term "isolated extracellular vesicle" refers to an extracellular vesicle substantially isolated from a cell from which an extracellular vesicle originates, for example, a mesenchymal stem cell.

In one embodiment, the extracellular vesicles may be isolated from a culture solution of mesenchymal stem cells derived from Wharton's jelly.

In one embodiment, the extracellular vesicles may be isolated from a culture solution of mesenchymal stem cells differentiated from induced pluripotent stem cells. The mesenchymal stem cells may be chemically induced to differentiate.

In one embodiment, the extracellular vesicles may be isolated from a culture solution of a HEK293T cell line.

In one embodiment, as a result of analyzing the miRNAs of extracellular vesicles isolated from mesenchymal stem cells cultured in a medium supplemented with at least one selected from the group consisting of zymosan, polyinosinic-polycytidylic acid (Poly I:C), and monophosphoryl lipid A (MPLA), the extracellular vesicle may comprise at least one miRNA selected from the group consisting of NovelmiRNA-67, hsa-miR-199a-3p, NovelmiRNA-298, hsa-miR-145-5p, NovelmiRNA-281, NovelmiRNA-109, hsa-miR-432-5p, NovelmiRNA-230, NovelmiRNA-88, hsa-miR-200b-3p, NovelmiRNA-169, hsa-miR-200a-3p, hsa-miR-487b-3p, hsa-miR-214-3p, NovelmiRNA-208, NovelmiRNA-164, NovelmiRNA-225, NovelmiRNA-174, hsa-miR-708-5p, hsa-miR-124-3p, NovelmiRNA-150, hsa-miR-135a-5p, NovelmiRNA-64, NovelmiRNA-92, NovelmiRNA-168, NovelmiRNA-22, NovelmiRNA-35, NovelmiRNA-161, hsa-miR-3940-3p, hsa-miR-219a-3p, hsa-miR-212-3p, NovelmiRNA-76, hsa-miR-323a-3p, hsa-miR-654-3p, hsa-miR-204-5p, hsa-miR-136-3p, NovelmiRNA-39, NovelmiRNA-175, hsa-miR-134-5p, hsa-miR-454-3p, NovelmiRNA-286, hsa-miR-542-3p,hsa-miR-433-3p, hsa-miR-574-5p, hsa-miR-6086, hsa-miR-4701-3p, hsa-miR-3149, hsa-miR-4743-5p, hsa-miR-8075, hsa-miR-6880-5p, hsa-miR-3148, hsa-miR-6780b-5p, hsa-miR-6846-5p, hsa-mir-320e, hsa-miR-32-3p, hsa-miR-642a-3p, hsa-miR-4505, hsa-miR-595, hsa-miR-4487, hsa-miR-4721, hsa-miR-7151-3p, hsa-miR-4484, hsa-miR-642b-3p, ENSG00000239154, hsa-miR-4706, hsa-miR-6126, hsa-miR-378h, hsa-miR-6849-5p, hsa-miR-7844-5p, hsa-miR-4535, hsa-mir-9-1, hsa-miR-4459, hsa-miR-4443, hsa-miR-6732-5p, hsa-miR-3064-5p, hsa-miR-3178, hsa-miR-3135b, hsa-miR-4717-3p, hsa-miR-206, hsa-miR-4529-3p, and hsa-miR-3613-5p. For example, the extracellular vesicle may comprise 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more or all miRNAs, among the above 43 types of miRNAs.

In one embodiment, as a result of analyzing the miRNAs of extracellular vesicles isolated from Wharton's jelly-derived mesenchymal stem cells cultured in a medium supplemented with at least one selected from the group consisting of zymosan, polyinosinic-polycytidylic acid (Poly I:C), and monophosphoryl lipid A (MPLA), the extracellular vesicle may comprise at least one miRNA selected from the group consisting of NovelmiRNA-67, hsa-miR-199a-3p, NovelmiRNA-298, hsa-miR-145-5p, NovelmiRNA-281, NovelmiRNA-109, hsa-miR-432-5p, NovelmiRNA-230, NovelmiRNA-88, hsa-miR-200b-3p, NovelmiRNA-169, hsa-miR-200a-3p, hsa-miR-487b-3p, hsa-miR-214-3p, NovelmiRNA-208, NovelmiRNA-164, NovelmiRNA-225, NovelmiRNA-174, hsa-miR-708-5p, hsa-miR-124-3p, NovelmiRNA-150, hsa-miR-135a-5p, NovelmiRNA-64, NovelmiRNA-92, NovelmiRNA-168, NovelmiRNA-22, NovelmiRNA-35, NovelmiRNA-161, hsa-miR-3940-3p, hsa-miR-219a-3p, hsa-miR-212-3p, NovelmiRNA-76, hsa-miR-323a-3p, hsa-miR-654-3p, hsa-miR-204-5p, hsa-miR-136-3p, NovelmiRNA-39, NovelmiRNA-175, hsa-miR-134-5p, hsa-miR-454-3p, NovelmiRNA-286, hsa-miR-542-3p, and hsa-miR-433-3p. In addition, it may significantly comprise the miRNAs compared to the comparative exosomes that have not been treated with chemical substances.

hsa-miR-199a-3p and hsa-miR-200b-3p can inhibit cell proliferation and induce apoptosis; hsa-miR-145-5p, hsa-miR-432-5p and hsa-miR-200a-3p can inhibit cell migration and invasion; hsa-miR-214-3p and hsa-miR-708-5p can inhibit cell proliferation and cell cycle progression; hsa-miR-487b-3p can inhibit chemoresistance and metastasis of osteosarcoma; hsa-miR-124-3p can function as a tumor suppressor; and hsa-miR-135a-5p can inhibit tumor invasion.

In one embodiment, as a result of analyzing the miRNAs of extracellular vesicles isolated from mesenchymal stem cells differentiated from induced pluripotent stem cells cultured in a medium supplemented with at least one selected from the group consisting of zymosan, polyinosinic-polycytidylic acid (Poly I:C), and monophosphoryl lipid A (MPLA), the extracellular vesicle may comprise at least one miRNA selected from the group consisting of hsa-miR-574-5p, hsa-miR-6086, hsa-miR-4701-3p, hsa-miR-3149, hsa-miR-4743-5p, hsa-miR-8075, hsa-miR-6880-5p, hsa-miR-3148, hsa-miR-6780b-5p, hsa-miR-6846-5p, hsa-mir-320e, hsa-miR-32-3p, hsa-miR-642a-3p, hsa-miR-4505, hsa-miR-595, hsa-miR-4487, hsa-miR-4721, hsa-miR-7151-3p, hsa-miR-4484, hsa-miR-642b-3p, ENSG00000239154, hsa-miR-4706, hsa-miR-6126, hsa-miR-378h, hsa-miR-6849-5p, hsa-miR-7844-5p, hsa-miR-4535, hsa-mir-9-1, hsa-miR-4459, hsa-miR-4443, hsa-miR-6732-5p, hsa-miR-3064-5p, hsa-miR-3178, hsa-miR-3135b, hsa-miR-4717-3p, hsa-miR-206, hsa-miR-4529-3p, and hsa-miR-3613-5p. In addition, it may significantly comprise the miRNAs compared to the comparative exosomes that have not been treated with chemical substances.

In another embodiment, as a result of analyzing the mRNAs of extracellular vesicles isolated from mesenchymal stem cells cultured in a medium supplemented with at least one selected from the group consisting of zymosan, polyinosinic-polycytidylic acid (Poly I:C), and monophosphoryl lipid A (MPLA), the extracellular vesicle may comprise at least one mRNA selected from the group consisting of MED13L, lnc-TOMM20L-1, lnc-CDK5R1-1, ETV3, SLC35G1, SMARCA4, ACAT2, ADAM12, ARFGEF1, CELF3, DNAJB5, lnc-WRNIP1-2, ACTL8, lnc-TM2D3-2, GCN1L1, LOC101929337, FAM217A, XLOC_l2_013467, SNHG4, NUDT13, MCTS1, ATG4D, NPEPL1, YEATS2, SPEN, GGT6, lnc-RP11-770J1.4.1-1, MIAT, lnc-HMCN1-2, RETN, lnc-EXT1-2, LIN54, C17orf74, SLC12A2, TTC28, ADRA2A, NLGN2, lnc-FBXO25-4, LOC100506603, LOC399886, RCC2, KIAA0040, HOXB9, JMJD8, LINC01146, lnc-GIT1-1, VRTN, NHP2, KIF26A, HAUS5, lnc-SOD2-2, APOL3, lnc-POLR2L-1, lnc-SUPT3H-1, lnc-XRN2-3, RTP2, TERT, TNFRSF12A, LOC645427, lnc-CTR9-5, LOC100507420, OPA3, XLOC_l2_014245, lnc-NR5A1-1, DMTN, SMPD4, TXLNB, SPRR1A, DISP1, LOC100130285, lnc-SLC17A9-1, lnc-SELV.1-1, XLOC_l2_000018, lnc-ROM1-2, lnc-RABEPK-1, lnc-RRP8-1, lnc-RP11-234B24.6.1-1, lnc-TMEM189-UBE2V1-2, LAMTOR2, BTK, lnc-BSND-1, C2orf71, lnc-C17orf63-2, lnc-CHGA-1, ABCD1, NCAN, C9orf106, AGAP2, EMC6, CD86, LOC100129175, lnc-PHYHD1-1, GIGYF2, RNF151, BAGE, ADRA2C, lnc-SLC43A1-1, ENTPD8, NYAP1, lnc-GLIPR1L1-1, CCNT2-AS1, NAVI, TBX21, GNAZ, DNM3OS, PRKAG2-AS1, lnc-MFSD9-4, lnc-RPGRIP1-2, ABCC6, LRFN3, lnc-COL1A1-2, lnc-PPAPDC3-2, CTU1, lnc-PABPC4-1, LOC100507165, LOC102724861, SPTSSB, lnc-C20orf96-4, DCDC5, lnc-CRYL1-1, LOC100506114, SHARPIN, ASMTL-AS1, HMOX1, SHARPIN, LOC101928207, TRAIP, lnc-ANTXRL-2, LINC01135, BZRAP1, LOC101926983, WDR6, C19orf81, NAMA, LIM2, FAM155A-IT1, lnc-RPP30-2, ZNF264, MALAT1, HTT-AS, ZDHHC22, SEPHS2, ABTB1, lnc-RP11-791J7.2.1-1, CCDC28B, LOC284263, lnc-PPIC-2, CDC42BPB, C9orf173-AS1, C9orf139, FGD5, SP6, RCOR2, lnc-AC016251.1-2, lnc-PRKAA2-1, RANBP3, lnc-SH2B3-1, XLOC_l2_011901, CACNA1H, LINC01314, lnc-VAPB-1, ARHGAP23, ATG9A, GM2A, XLOC_l2_013547, lnc-SLC25A26-1, TMEM240, CLSTN3, ATG16L2, MMP11, TSSK3, lnc-SOX6-1, EDEM3, LOC101927210, C14orf169, EBLN2, lnc-XRN1-1, lnc-FTSJD2-1, MDFI, LOC102546226, SLC5A10, SENP7, lnc-C17orf97-4, ARNTL, lnc-RTL1-1, ZNF713, XLOC_l2_005871, QSOX1, SP2-AS1, ARSA, PHF7, GS1-24F4.2, LRIT1, PLLP, SLC4A11, C11orf95, FAM110B, IER5, LINC01508, lnc-GTPBP8-1, LOC400958, WFDC21P, lnc-CPXM2-2, P2RX6, SLC22A7, lnc-PPP2R2C-1, lnc-GATA5-2, MARVELD1, lnc-TRIM41-3, PRSS8, SEMA6C, SLC48A1, RPL28, BGN, BZW1, lnc-C6orf221-2, SLC20A2, HYALP1, SNAR-G1, SEC22A, TIMM8B, ATAD1, FNDC7, B3GNT8, STARD7-AS1, TMEM100, LARP6, HELZ2, TMX2, lnc-EPHB6-1, PSMC2, lnc-LINC00273-1, TNFRSF25, BCL3, LOC101929450, LOC102724416, FLII, WAC, PPP6R1, LOC100507377, LRRFIP2, CCNT2-AS1, lnc-SCAMP5-1, BRD1, lnc-ELAVL4-3, CHRDL1, lnc-FUT8-1, lnc-DNAJC7-1, LINC01264, LMO2, LINC01197, LOC101927533, REP15, C1orf226, lnc-STEAP1B-1, SNORD3B-1, lnc-TMEM135-2, lnc-FAM160A1-1, GOLGA2P5, TMEM132C, GPX3, lnc-DCAF4L2-2, XLOC_l2_014217, PPT1, RPA4, KCTD5, lnc-UQCRFS1-9, MTUS2-AS1, CHKA, TBC1D31, SNORD38A, lnc-RP11-322L20.1.1-7, RAD51, MEIOB, lnc-ADAMTS14-1, NOS2, and PCDHA13. For example, the extracellular vesicle may comprise 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, 30 or more or all mRNAs, among the above 275 types of mRNAs.

In another embodiment, as a result of analyzing the cytokines of extracellular vesicles isolated from mesenchymal stem cells cultured in a medium supplemented with at least one selected from the group consisting of zymosan, polyinosinic-polycytidylic acid (Poly I:C), and monophosphoryl lipid A (MPLA), the extracellular vesicle may comprise at least one cytokine selected from the group consisting of IL-6, IL-8, MCP-3, MCP-1, IP-10, and RANTES. For example, the extracellular vesicle may comprise 2 or more, 3 or more, 4 or more, 5 or more or all cytokines, among the above 6 types of cytokines.

The extracellular vesicle may comprise at least one selected from the group consisting of zymosan, Poly I:C, and monophosphoryl lipid A (MPLA).

As used herein, the term "zymosan" is a protein and carbohydrate complex obtained from yeast cell walls, and refers to a glucan in which glucose units linked by β-1,3-glycosidic bonds are repeated. Zymosan may be a compound represented by the following Formula 1.

[Formula 1]

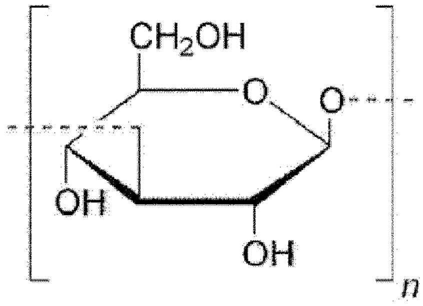

β-1,3

As used herein, the term "polyinosinic-polycytidylic acid (Poly I:C)" refers to an immunostimulant. Poly I:C is known to interact with toll-like receptor 3 (TLR3) expressed on the endosomal membrane of macrophages and dendritic cells, and may be a compound represented by the following Formula 2.

[Formula 2]

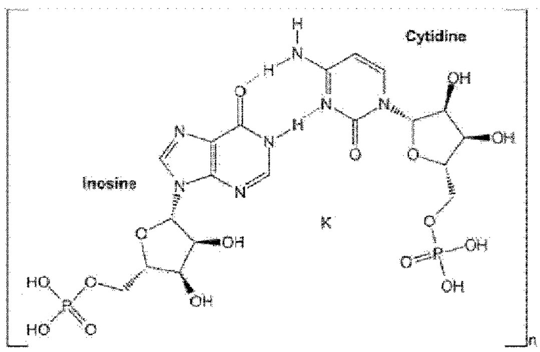

As used herein, the term "monophosphoryl lipid A (MPLA)" is a modified form of lipid A and refers to an agonist of toll-like receptor 4. Monophosphoryl lipid A (MPLA) is derived from the cell wall of non-pathogenic Salmonella and is known to have immunostimulating activity and may be a compound represented by the following Formula 3.

[Formula 3]

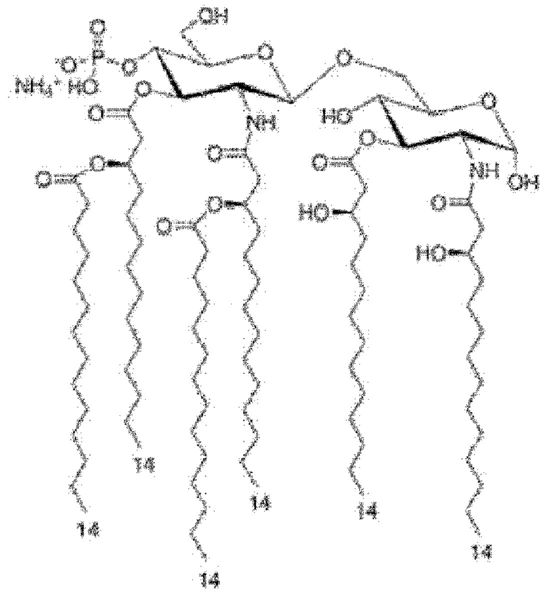

The extracellular vesicle i) may be positive for at least one marker selected from the group consisting of CD9, CD63, CD81, and TSG101, and ii) may be negative for at least one marker selected from the group consisting of GM130 and Calnexin.

As used herein, the term "positive" may mean, with respect to a specific marker, that the marker is present in a greater amount or at a higher concentration compared to a reference control. That is, the extracellular vesicle according to the present invention is positive for a marker if the marker is present on the surface or inside the extracellular vesicles and the extracellular vesicles can be distinguished from one or more non-extracellular vesicles or other extracellular vesicles using the marker. For example, the extracellular vesicle of the present invention can be detected with an antibody specific for CD81, an extracellular vesicle-specific surface antigen, and if the signal intensity from this antibody is detectably greater than that of the control group (for example, background value), the extracellular vesicle is "$CD81^+$ (CD81 positive)".

As used herein, the term "negative" means that the marker is not detectable compared to background values even when using a specific antibody. For example, if the extracellular vesicle of the present invention cannot be detectably labeled with an antibody specific for GM130, the extracellular vesicle is "$GM130^-$ (GM130 negative)".

The above immunological characteristics can be determined by conventional methods known in the art to which the present invention pertains. For example, various methods such as flow cytometry, immunocytochemical staining, Western blot, or RT-PCR can be used.

In another aspect of the present invention, there is provided an extracellular vesicle isolated from mesenchymal stem cells treated with at least one selected from the group consisting of zymosan, Poly I:C, and monophosphoryl lipid A (MPLA).

The extracellular vesicle i) may comprise at least one miRNA selected from the group consisting of NovelmiRNA-67, hsa-miR-199a-3p, NovelmiRNA-298, hsa-miR-145-5p, NovelmiRNA-281, NovelmiRNA-109, hsa-miR-432-5p, NovelmiRNA-230, NovelmiRNA-88, hsa-miR-200b-3p, NovelmiRNA-169, hsa-miR-200a-3p, hsa-miR-487b-3p, hsa-miR-214-3p, NovelmiRNA-208, NovelmiRNA-164, NovelmiRNA-225, NovelmiRNA-174, hsa-miR-708-5p, hsa-miR-124-3p, NovelmiRNA-150, hsa-miR-135a-5p, NovelmiRNA-64, NovelmiRNA-92, NovelmiRNA-168, NovelmiRNA-22, NovelmiRNA-35, NovelmiRNA-161, hsa-miR-3940-3p, hsa-miR-219a-3p, hsa-miR-212-3p, NovelmiRNA-76, hsa-miR-323a-3p, hsa-miR-654-3p, hsa-miR-204-5p, hsa-miR-136-3p, NovelmiRNA-39, NovelmiRNA-175, hsa-miR-134-5p, hsa-miR-454-3p, NovelmiRNA-286, hsa-miR-542-3p, hsa-miR-433-3p, hsa-miR-574-5p, hsa-miR-6086, hsa-miR-4701-3p, hsa-miR-3149, hsa-miR-4743-5p, hsa-miR-8075, hsa-miR-6880-5p, hsa-miR-3148, hsa-miR-6780b-5p, hsa-miR-6846-5p, hsa-mir-320e, hsa-miR-32-3p, hsa-miR-642a-3p, hsa-miR-4505, hsa-miR-595, hsa-miR-4487, hsa-miR-4721, hsa-miR-7151-3p, hsa-miR-4484, hsa-miR-642b-3p, ENSG00000239154, hsa-miR-4706, hsa-miR-6126, hsa-miR-378h, hsa-miR-6849-5p, hsa-miR-7844-5p, hsa-miR-4535, hsa-mir-9-1, hsa-miR-4459, hsa-miR-4443, hsa-miR-6732-5p, hsa-miR-3064-5p, hsa-miR-3178, hsa-miR-3135b, hsa-miR-4717-3p, hsa-miR-206, hsa-miR-4529-3p, and hsa-miR-3613-5p, and

ii) may comprise at least one mRNA selected from the group consisting of MED13L, lnc-TOMM20L-1, lnc-CDK5R1-1, ETV3, SLC35G1, SMARCA4, ACAT2, ADAM12, ARFGEF1, CELF3, DNAJB5, lnc-WRNIP1-2, ACTL8, lnc-TM2D3-2, GCN1L1, LOC101929337, FAM217A, XLOC_l2_013467, SNHG4, NUDT13, MCTS1, ATG4D, NPEPL1, YEATS2, SPEN, GGT6, lnc-RP11-770J1.4.1-1, MIAT, lnc-HMCN1-2, RETN, lnc-EXT1-2, LIN54, C17orf74,

SLC12A2, TTC28, ADRA2A, NLGN2, lnc-FBXO25-4, LOC100506603, LOC399886, RCC2, KIAA0040, HOXB9, JMJD8, LINC01146, lnc-GIT1-1, VRTN, NHP2, KIF26A, HAUS5, lnc-SOD2-2, APOL3, lnc-POLR2L-1, lnc-SUPT3H-1, lnc-XRN2-3, RTP2, TERT, TNFRSF12A, LOC645427, lnc-CTR9-5, LOC100507420, OPA3, XLOC_l2_014245, lnc-NR5A1-1, DMTN, SMPD4, TXLNB, SPRR1A, DISP1, LOC100130285, lnc-SLC17A9-1, lnc-SELV.1-1, XLOC_l2_000018, lnc-ROM1-2, lnc-RABEPK-1, lnc-RRP8-1, lnc-RP11-234B24.6.1-1, lnc-TMEM189-UBE2V1-2, LAMTOR2, BTK, lnc-BSND-1, C2orf71, lnc-C17orf63-2, lnc-CHGA-1, ABCD1, NCAN, C9orf106, AGAP2, EMC6, CD86, LOC100129175, lnc-PHYHD1-1, GIGYF2, RNF151, BAGE, ADRA2C, lnc-SLC43A1-1, ENTPD8, NYAP1, lnc-GLIPR1L1-1, CCNT2-AS1, NAVI, TBX21, GNAZ, DNM3OS, PRKAG2-AS1, lnc-MFSD9-4, lnc-RPGRIP1-2, ABCC6, LRFN3, lnc-COL1A1-2, lnc-PPAPDC3-2, CTU1, lnc-PABPC4-1, LOC100507165, LOC102724861, SPTSSB, lnc-C20orf96-4, DCDC5, lnc-CRYL1-1, LOC100506114, SHARPIN, ASMTL-AS1, HMOX1, SHARPIN, LOC101928207, TRAIP, lnc-ANTXRL-2, LINC01135, BZRAP1, LOC101926983, WDR6, C19orf81, NAMA, LIM2, FAM155A-IT1, lnc-RPP30-2, ZNF264, MALAT1, HTT-AS, ZDHHC22, SEPHS2, ABTB1, lnc-RP11-791J7.2.1-1, CCDC28B, LOC284263, lnc-PPIC-2, CDC42BPB, C9orf173-AS1, C9orf139, FGD5, SP6, RCOR2, lnc-AC016251.1-2, lnc-PRKAA2-1, RANBP3, lnc-SH2B3-1, XLOC_l2_011901, CACNA1H, LINC01314, lnc-VAPB-1, ARHGAP23, ATG9A, GM2A, XLOC_l2_013547, lnc-SLC25A26-1, TMEM240, CLSTN3, ATG16L2, MMP11, TSSK3, lnc-SOX6-1, EDEM3, LOC101927210, C14orf169, EBLN2, lnc-XRN1-1, lnc-FTSJD2-1, MDFI, LOC102546226, SLC5A10, SENP7, lnc-C17orf97-4, ARNTL, lnc-RTL1-1, ZNF713, XLOC_l2_005871, QSOX1, SP2-AS1, ARSA, PHF7, GS1-24F4.2, LRIT1, PLLP, SLC4A11, C11orf95, FAM110B, IER5, LINC01508, lnc-GTPBP8-1, LOC400958, WFDC21P, lnc-CPXM2-2, P2RX6, SLC22A7, lnc-PPP2R2C-1, lnc-GATA5-2, MARVELD1, lnc-TRIM41-3, PRSS8, SEMA6C, SLC48A1, RPL28, BGN, BZW1, lnc-C6orf221-2, SLC20A2, HYALP1, SNAR-G1, SEC22A, TIMM8B, ATAD1, FNDC7, B3GNT8, STARD7-AS1, TMEM100, LARP6, HELZ2, TMX2, lnc-EPHB6-1, PSMC2, lnc-LINC00273-1, TNFRSF25, BCL3, LOC101929450, LOC102724416, FLII, WAC, PPP6R1, LOC100507377, LRRFIP2, CCNT2-AS1, lnc-SCAMP5-1, BRD1, lnc-ELAVL4-3, CHRDL1, lnc-FUT8-1, lnc-DNAJC7-1, LINC01264, LMO2, LINC01197, LOC101927533, REP15, C1orf226, lnc-STEAP1B-1, SNORD3B-1, lnc-TMEM135-2, lnc-FAM160A1-1, GOLGA2P5, TMEM132C, GPX3, lnc-DCAF4L2-2, XLOC_l2_014217, PPT1, RPA4, KCTD5, lnc-UQCRFS1-9, MTUS2-AS1, CHKA, TBC1D31, SNORD38A, lnc-RP11-322L20.1.1-7, RAD51, MEIOB, lnc-ADAMTS14-1, NOS2, and PCDHA13, and

iii) may comprise at least one cytokine selected from the group consisting of IL-6, IL-8, MCP-3, MCP-1, IP-10, IL-1$\beta$, and RANTES.

In one embodiment, the extracellular vesicle may be treated with Poly I:C and monophosphoryl lipid A (MPLA); or zymosan, Poly I:C, and monophosphoryl lipid A (MPLA).

In one embodiment, the extracellular vesicle may be treated with Poly I:C and monophosphoryl lipid A (MPLA); or zymosan, Poly I:C, and monophosphoryl lipid A (MPLA).

The extracellular vesicle is as described above.

**Pharmaceutical use of novel extracellular vesicles isolated from mesenchymal stem cells**

In another aspect of the present invention, there is provided a pharmaceutical composition for treating or preventing cancer, comprising the extracellular vesicle as an active ingredient.

The pharmaceutical composition for preventing or treating cancer, comprising as an active ingredient the extracellular vesicle isolated from mesenchymal stem cells treated with at least one selected from the group consisting of zymosan, Poly I:C, and monophosphoryl lipid A (MPLA), according to the present invention, can increase the efficacy of cancer treatment and/or prevention.

The extracellular vesicle is as described above.

In one embodiment, the immune stimulating activity and tumor growth inhibitory effect of the extracellular vesicles isolated from Wharton's jelly-derived mesenchymal stem cells treated with zymosan, Poly I:C, and monophosphoryl lipid A (MPLA) against cancer cells can be confirmed.

In another embodiment, the tumor growth inhibitory effect of the extracellular vesicles isolated after culturing mesenchymal stem cells differentiated from induced pluripotent stem cells by treating them with zymosan, Poly I:C, and monophosphoryl lipid A (MPLA) can be confirmed.

The cancer may be any one selected from the group consisting of gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, and lymphoma, but is not limited thereto.

The preferred dosage of the pharmaceutical composition varies depending on the patient's condition and body weight, the severity of the disease, the drug form, and the route and period of administration, but can be appropriately selected by those of ordinary skill in the art. In the pharmaceutical composition for preventing or treating cancer of the

present invention, the active ingredient may be included in any amount (effective amount) depending on the use, formulation, compounding purpose, and the like, as long as it can exhibit anticancer activity. The typical effective amount of extracellular vesicles will be determined within the range of 0.001 wt% to 20.0 wt% based on the total weight of the composition. Here, the "effective amount" refers to an amount of the active ingredient capable of inducing an anticancer effect. Such an effective amount can be experimentally determined within the normal capabilities of those of ordinary skill in the art.

As used herein, the term "treatment" may be used to mean both therapeutic treatment and preventive treatment. Prevention may be used to mean alleviating or reducing a pathological condition or disease in a subject. In one embodiment, the term "treatment" includes all applications or any form of medication for treating a disease in mammals, including humans. In addition, the above term includes inhibiting or slowing down a disease or the progression of a disease; restoring or repairing damaged or defective functions, thereby partially or completely alleviating a disease; or stimulating an inefficient process; or alleviating a serious disease.

As used herein, the term "efficacy" can be determined by one or more parameters, such as survival or disease-free survival over a period of time, such as 1 year, 5 years, or 10 years. In addition, the parameters can include suppression of the size of at least one tumor in a subject.

Pharmacokinetic parameters, such as bioavailability, and underlying parameters, such as clearance rate, can also affect efficacy. Therefore, "enhanced efficacy" (for example, improved efficacy) can be due to enhanced pharmacokinetic parameters and enhanced efficacy, and can be measured by comparing clearance rates and tumor growth in test animals or human subjects, or by comparing parameters such as survival, relapse rate, or disease-free survival.

Here, the term "therapeutically effective amount" or "pharmaceutically effective amount" refers to an amount of a compound or composition effective for preventing or treating a target disease, which is an amount that is sufficient to treat the disease with a reasonable benefit/risk ratio applicable to medical treatment and that does not cause side effects. The level of the effective amount may be determined according to factors including the health condition of the patient, the type and severity of the disease, the activity of the drug, the sensitivity to the drug, administration method, administration time, the route of administration and excretion rate, treatment period, the drug to be combined or concurrently used, and other factors well known in the medical field. In one embodiment, a therapeutically effective amount refers to an amount of a drug effective for treating cancer.

The pharmaceutical composition may be administered as an individual therapeutic agent or in combination with another therapeutic agent, may be administered sequentially or simultaneously with a conventional therapeutic agent, and may be administered in one dose or in multiple doses. It is important to administer an amount that can achieve the maximum effect with the minimum amount without side effects by considering all of the above factors, and this can be easily determined by those of ordinary skill in the art.

At this time, the pharmaceutical composition may further comprise a pharmaceutically acceptable carrier. The pharmaceutically acceptable carrier may be any carrier as long as it is non-toxic material suitable for delivery to a patient. Distilled water, alcohol, fats, waxes and inert solids may be included as a carrier. In addition, a pharmacologically acceptable adjuvant (buffering agent, dispersing agent) may be included in the pharmaceutical composition.

Specifically, the pharmaceutical composition may be prepared as a parenteral formulation according to the route of administration by a conventional method known in the art, including a pharmaceutically acceptable carrier in addition to the active ingredient. Here, "pharmaceutically acceptable" means that it does not inhibit the activity of the active ingredient and does not have toxicity beyond what the subject for application (prescription) can tolerate.

When the pharmaceutical composition is prepared as a parenteral formulation, it may be formulated in the form of an injection, a transdermal preparation, a nasal inhalant, and a suppository together with suitable carriers according to methods known in the art. When it is formulated as an injection, as a suitable carrier, sterile water, ethanol, polyol such as glycerol or propylene glycol, or a mixture thereof may be used, and Ringer's solution, PBS (Phosphate Buffered Saline) containing triethanolamine, or sterile water for injection, an isotonic solution such as 5% dextrose, and the like may be preferably used. Methods for formulating pharmaceutical compositions are known in the art, and specific references can be made to literature [Remington's Pharmaceutical Sciences (19th ed., 1995)], etc. The above literature is considered a part of the present specification.

The preferred dosage of the pharmaceutical composition may be in the range of 0.01 ug/kg to 10 g/kg per day, or in the range of 0.01 mg/kg to 1 g/kg per day, depending on the patient's condition, body weight, sex, age, the severity of the patient, and the route of administration. The administration may be performed once a day or divided into several times per day. This dosage may be increased or decreased depending on the route of administration, the severity of the disease, gender, body weight, age, and the like, and therefore should not be construed as limiting the scope of the present invention in any way.

The subject to which the pharmaceutical composition can be applied (prescribed) is a mammal and a human, and particularly, the subject is preferably a human. In addition to the active ingredient, the pharmaceutical composition of the present invention may further comprise any compound or natural extract that has already been verified as safe and known to have anticancer activity in order to increase or enhance anticancer activity.

In another aspect of the present invention, there is provided a pharmaceutical composition for combination administration for treating or preventing cancer, comprising the extracellular vesicle and an immune anticancer agent as active ingredients.

Since the extracellular vesicles increase immune activity in the body, they can be used in combination with an immune checkpoint inhibitor, a conventionally-used anticancer agent.

The immune anticancer agent may be any one selected from the group consisting of an immune checkpoint inhibitor, a chimeric antigen receptor-T cell (CAR-T), a T cell receptor modified T cell (TCR-T), a tumor infiltrating lymphocyte (TIL), a bispecific T-cell engager (BiTE), a tumor vaccine, and an oncolytic virus, but is not limited thereto.

The immune checkpoint inhibitor may be any one selected from the group consisting of an anti-PD-1 antibody, an anti-PD-L1 antibody, an anti-PD-L2 antibody, an anti-CTLA-4 antibody, an anti-B7-H4 antibody, an anti-HVEM antibody, an anti-TIM3 antibody, an anti-GAL9 antibody, an anti-LAG3 antibody, an anti-VISTA antibody, an anti-KIR antibody, an anti-BTLA antibody, and an anti-TIGIT antibody, but is not limited thereto. Specifically, it may be an anti-PD-1 antibody. In one embodiment, the immune checkpoint inhibitor may be Ipilimumab, Pembrolizumab, Nivolumab, Cemiplimab, Atezolizumab, Avelumab, Duralumab, or a combination thereof, but is not limited thereto.

As used herein, the term "immune checkpoint inhibitor" refers to a substance that inhibits the activity of an immune checkpoint protein that inhibits the differentiation, proliferation, and activity of immune cells, and is known to eliminate cancer cells by preventing cancer cells from evading the immune system.

In one embodiment, when comparing the administration of the extracellular vesicle alone with the administration of the extracellular vesicle in combination with an anti-PD-1 antibody, it was confirmed that the tumor growth inhibitory effect was significantly increased by combination administration.

**Method for preparing novel extracellular vesicles**

In another aspect of the present invention, there is provided a method for preparing extracellular vesicles, comprising: a) culturing human-derived cells in a serum-free cell culture medium supplemented with Poly I:C and monophosphoryl lipid A (MPLA); b) obtaining a culture solution during the culturing process; and c) filtering the culture solution obtained during the above process to remove particles larger than 200 nm and then isolating the extracellular vesicles.

In one embodiment, the serum-free cell culture medium may be additionally supplemented with zymosan. Specifically, the serum-free cell culture medium may be supplemented with Poly I:C and monophosphoryl lipid A (MPLA); or zymosan, Poly I:C, and monophosphoryl lipid A (MPLA).

Overlapping contents are omitted in consideration of the complexity of the present specification, and terms not otherwise defined in the present specification have meanings commonly used in the art to which the present invention pertains.

Hereinafter, the present invention will be described in more detail through examples. These examples are intended only to illustrate the present invention, and it will be obvious to those of ordinary skill in the art that the scope of the present invention is not to be construed as being limited by these examples.

**Preparation Example 1. Culturing of Wharton's jelly-derived mesenchymal stem cells**

The human Wharton's jelly-derived mesenchymal stem cells (WJ-MSC) were cultured in minimal essential media alpha modification ($\alpha$-MEM) (Welgene, South Korea) medium supplemented with 10% fetal bovine serum (Corning, USA) and 1% penicillin-streptomycin at 37 °C and 5% $CO_2$ conditions. The human Wharton's jelly-derived mesenchymal stem cells were obtained from Samsung Medical Center with IRB approval and used under research consent.

**Manufacturing Example 1. Isolation of exosomes from Wharton's jelly-derived mesenchymal stem cells treated with chemical substance cocktail**

When the WJ-MSCs of Preparation Example 1 were passaged 7 to 13 times and the confluency of the WJ-MSCs reached 80% to 90%, the culture medium was removed and then washed with PBS (Welgene, South Korea). Thereafter, the cells were cultured in a serum-free $\alpha$-MEM medium supplemented with a chemical substance cocktail at 37 °C and 5% $CO_2$ conditions for 24 hours to obtain a culture solution. The chemical substance cocktail consisted of 1 $\mu$g/mL zymosan A (isolated from Saccharomyces cerevisiae) (Sigma-Aldrich, USA), 30 ng/mL polyinosinic-polycytidylic acid (Poly I:C) (Sigma-Aldrich, USA), and 1 $\mu$g/mL monophosphoryl lipid A (MPLA) (InvivoGen, USA).

The obtained WJ-MSC culture solution was centrifuged at 4 °C and 3,000 xg conditions for 20 minutes to remove cells and large cell debris. The supernatant obtained through centrifugation was passed through a 0.2 $\mu$m Minisart™ NML syringe filter (Sartorious, Germany) to remove particles larger than 200 nm. Thereafter, exosomes were isolated using a Labspinner (Live Cell Instrument, South Korea) equipment. The exosomes were obtained in a state existing in PBS and

used directly for experiments or stored frozen at 80 °C.

**Comparative Example 1. Isolation of exosomes from Wharton's jelly-derived mesenchymal stem cells**

When the WJ-MSCs of Preparation Example 1 were passaged 7 to 13 times and the confluency of the WJ-MSCs reached 80% to 90%, the culture medium was removed and then washed with PBS (Welgene, South Korea). Thereafter, the cells were cultured in a serum-free α-MEM medium at 37 °C and 5% $CO_2$ conditions for 24 hours to obtain a culture solution.

Exosomes were isolated from the obtained culture solution in the same manner as described in Manufacturing Example 1.

**Comparative Example 2. Isolation of exosomes from Wharton's jelly-derived mesenchymal stem cells treated with Poly I:C**

When the WJ-MSCs of Preparation Example 1 were passaged 7 to 13 times and the confluency of the WJ-MSCs reached 80% to 90%, the culture medium was removed and then washed with PBS (Welgene, South Korea). Thereafter, the cells were cultured in a serum-free α-MEM medium supplemented with 30 ng/mL of Poly I:C (Sigma-Aldrich, USA) at 37 °C and 5% $CO_2$ conditions for 24 hours to obtain a culture solution.

Exosomes were isolated from the obtained culture solution in the same manner as described in Manufacturing Example 1.

**Comparative Example 3. Isolation of exosomes from HeLa cells treated with chemical substance cocktail**

Exosomes were isolated in the same manner as described in Manufacturing Example 1 using HeLa cells instead of WJ-MSCs. The HeLa cells were purchased from the ATCC and used.

**Manufacturing Example 2. Isolation in bulk of exosomes from Wharton's jelly-derived mesenchymal stem cells treated with chemical substance cocktail**

When the WJ-MSCs of Preparation Example 1 were passaged 7 to 13 times and the confluency of the WJ-MSCs reached 80% to 90%, the culture medium was removed and then washed with PBS (Welgene, South Korea). Thereafter, the cells were cultured in a serum-free α-MEM medium supplemented with a chemical substance cocktail at 37 °C and 5% $CO_2$ conditions for 24 hours to obtain a culture solution. The chemical substance cocktail consisted of 1 μg/mL zymosan A (isolated from Saccharomyces cerevisiae) (Sigma-Aldrich, USA), 30 ng/mL polyinosinic-polycytidylic acid (Poly I:C) (Sigma-Aldrich, USA), and 1 μg/mL monophosphoryl lipid A (MPLA) (InvivoGen, USA).

The obtained WJ-MSC culture solution was subjected to tangential flow filtration (TFF) to remove cells and large cell debris (0.65 μm). The solution obtained by passing through the filter was concentrated in the same manner (TFF) using a MWCO 300,000 or 500,000 Da (Dalton) filter to remove impurities below the MWCO. Thereafter, buffer exchange (dialysis) with PBS, etc. was performed using the same method (TFF) to isolate exosomes.

**Comparative Example 4. Isolation in bulk of exosomes from Wharton's jelly-derived mesenchymal stem cells**

When the WJ-MSCs of Preparation Example 1 were passaged 7 to 13 times and the confluency of the WJ-MSCs reached 80% to 90%, the culture medium was removed and then washed with PBS (Welgene, South Korea). Thereafter, the cells were cultured in a serum-free α-MEM medium at 37 °C and 5% $CO_2$ conditions for 24 hours to obtain a culture solution.

Exosomes were isolated from the obtained culture solution in the same manner as described in Manufacturing Example 2.

**Comparative Example 5. Isolation in bulk of exosomes from iPSC-derived mesenchymal stem cells treated with chemical substance cocktail**

The induced pluripotent stem cells purchased from the ATCC were placed in a 12-well plate coated with vitronectin, and cultured for 5 days in α-MEM medium supplemented with 10% fetal bovine serum (FBS) and 1% penicillin-streptomycin (PS) by adding a chemical substance cocktail comprising SB4, SB431542, Y27632, PD0325901, SB202190, SP600125, Go6983, and CHIR99021. Cells differentiated into mesoderm were obtained, and then the cells isolated from the medium by treating with 0.25% Trypsin-EDTA were centrifuged at 300 RCF for 5 minutes. Thereafter, a chemical substance cocktail comprising Y27632, PD0325901, SB202190, SP600125, Go6983 and CHIR99021 was

added to α-MEM medium supplemented with 10% FBS and 1% PS, and the collected cells were suspended, and then 1 × $10^6$ cells per well were placed in a 6-well plate and cultured for 5 days to induce the differentiation into mesenchymal stem cells. The culture solution was obtained during the process of culturing the differentiated iPSC-derived mesenchymal stem cells (ciMSC) in a low-concentration DMEM medium supplemented with 10% FBS and 1% PS.

Exosomes were isolated from the obtained culture solution in the same manner as described in Manufacturing Example 2.

**Experimental Example 1. Analysis of characteristics of exosomes isolated from Wharton's jelly-derived mesenchymal stem cells treated with chemical substance cocktail**

**Experimental Example 1.1. Measurement of exosome size**

The size and size distribution of exosomes isolated from human Wharton's jelly-derived mesenchymal stem cells in Comparative Example 1 (CTRL Exo) and exosomes isolated from human Wharton's jelly-derived mesenchymal stem cells treated with a chemical substance cocktail in Manufacturing Example 1 (CHEM Exo) were measured using nanoparticle tracking analysis (NTA; NS300, Malvern Panalytical, UK), and the results are shown in Figure 1.

As shown in Figure 1, it was confirmed that the sizes of CTRL Exo and CHEM Exo were distributed between 50 and 200 nm.

**Experimental Example 1.2. Confirmation of marker expression of exosomes**

In order to confirm whether exosomes isolated from human Wharton's jelly-derived mesenchymal stem cells in Comparative Example 1 (CTRL Exo) and exosomes isolated from human Wharton's jelly-derived mesenchymal stem cells treated with a chemical substance cocktail in Manufacturing Example 1 (CHEM Exo) express exosome-specific markers, Western blot was performed. Specifically, as primary antibodies, the exosome-positive markers, CD63 monoclonal antibodies (mAbs; Abcam, UK), CD81 mAbs (Bioss Antibodies, USA), CD9 mAbs (Cell Signaling Technology, USA) and TSG101 mAbs (Abcam, UK), and the exosome-negative markers, GM130 polyclonal antibodies (pAbs; Bioss Antibodies, USA) and Calnexin mAbs (Bioss Antibodies, USA), were used. For detection of primary antibodies, goat anti-mouse or goat anti-rabbit IgG H&L (Abcam, UK) conjugated with horseradish peroxidase (HRP) was used as a secondary antibody. The results of Western blot are shown in Figure 2.

As shown in Figure 2, it was confirmed that both CTRL Exo and CHEM Exo expressed CD9, CD63, CD81 proteins belonging to the Tetraspanin family, which are membrane proteins of exosomes, and TSG101, a multivesicular body protein. In addition, it was confirmed that calnexin, a membrane-resident protein present in the endoplasmic reticulum, and GM130, a Golgi marker, were not expressed. This confirmed that the exosomes isolated in Manufacturing Example 1 were pure exosomes that did not comprise cellular organelles other than exosomes.

**Experimental Example 2. Analysis of cytokines present in exosomes**

A cytokine ELISA was performed to analyze cytokines present in exosomes. Cytokine analysis was performed through Creative Biolabs' Exosomal cytokine profiling service, which is an analysis using the Bio-Plex Pro human cytokine screening 48-plex panel (Bio-Rad, #1200283) that can check the expression of multiple cytokines at once. Data analysis was performed from August to September 2021.

Specifically, S1 to S8 are samples for creating a standard curve, and the concentrations were set to decrease in order from S1, and PBS was used as a negative control. CTRL Exo is an exosome isolated from human Wharton's jelly-derived mesenchymal stem cells in Comparative Example 1, CHEM Exo is an exosome isolated from human Wharton's jelly-derived mesenchymal stem cells treated with a chemical substance cocktail in Manufacturing Example 1, POLYI:C Exo is an exosome isolated from human Wharton's jelly-derived mesenchymal stem cells treated with Poly I:C in Comparative Example 2, and HeLa CHEM Exo is an exosome isolated from HeLa cells treated with a chemical substance cocktail in Comparative Example 3.

In order to confirm whether the analysis results of cytokines present in the exosomes isolated from human Wharton's jelly-derived mesenchymal stem cells in Manufacturing Example 1 are specific to mesenchymal stem cells, HeLa cells, the most commonly used cell line, were treated with the chemical substance cocktail described in Manufacturing Example 1, and then exosomes were isolated to confirm the expression of cytokines in Comparative Example 3, and the results are shown in Figure 3 .

As shown in Figure 3, it was confirmed that the expression of IL-6, IL-8, MCP-3, MCP-1, IP-10, and RANTES was strongly increased in the exosomes isolated from human Wharton's jelly-derived mesenchymal stem cells treated with a chemical substance cocktail (CHEM Exo) compared to the exosomes that were not treated with the chemical substance cocktail (CTRL Exo) and the exosomes that were treated with only Poly I:C (POLYI:C Exo). In addition, it was confirmed

that when treated with the chemical substance cocktail having the same composition, the expression of IL-6, IL-8, MCP-3, MCP-1, IP-10, and RANTES was strongly increased in the exosome isolated from human Wharton's jelly-derived mesenchymal stem cells (CHEM Exo), whereas the expression of the corresponding cytokines was not increased in the exosomes isolated from HeLa cells (HeLa CHEM Exo).

**Experimental Example 3. Analysis of miRNAs present in exosomes isolated from Wharton's jelly-derived mesenchymal stem cells treated with chemical substance cocktail**

In order to analyze miRNAs present in exosomes, the Extracellular vesicle characterization service of Creative Biolabs was used. Specifically, miRNAs contained in exosome samples were extracted, the library was constructed, and then sequencing was performed. By comparing the resulting sequence information with the existing miRbase database, miRNAs that were relatively more expressed in each exosome sample were analyzed, and the results are shown in Figure 4. Data analysis was performed from August to September 2021.

Comparative Example 1 is an exosome isolated from human Wharton's jelly-derived mesenchymal stem cells (CTRL Exo), Manufacturing Example 1 is an exosome isolated from human Wharton's jelly-derived mesenchymal stem cells treated with a chemical substance cocktail (CHEM Exo), Comparative Example 2 is an exosome isolated from human Wharton's jelly-derived mesenchymal stem cells treated with Poly I:C (POLY IC Exo), and Comparative Example 3 is an exosome isolated from HeLa cells treated with a chemical substance cocktail (HeLa CHEM Exo). The results of measuring the types of miRNAs present in each exosome and their expression intensities are shown in Table 1 below.

[Table 1]

| | Manufacturing Example 1 | Comparative Example 2 | Comparative Example 1 | Comparative Example 3 |
|---|---|---|---|---|
| **NovelmiRNA-67** | 792 | 292 | 0 | 0 |
| **hsa-miR-199a-3p** | 322 | 383 | 54 | 6 |
| **NovelmiRNA-298** | 199 | 0 | 0 | 0 |
| **hsa-miR-145-5p** | 106 | 80 | 5 | 2 |
| **NovelmiRNA-281** | 103 | 0 | 0 | 0 |
| **NovelmiRNA-109** | 88 | 0 | 0 | 0 |
| **hsa-miR-432-5p** | 81 | 27 | 0 | 0 |
| **NovelmiRNA-230** | 68 | 0 | 0 | 0 |
| **NovelmiRNA-88** | 66 | 0 | 0 | 0 |
| **hsa-miR-200b-3p** | 63 | 47 | 0 | 0 |
| **NovelmiRNA-169** | 56 | 0 | 0 | 0 |
| **hsa-miR-200a-3p** | 41 | 21 | 0 | 0 |
| **hsa-miR-487b-3p** | 39 | 35 | 0 | 0 |
| **hsa-miR-214-3p** | 36 | 53 | 3 | 0 |
| **NovelmiRNA-208** | 35 | 0 | 0 | 0 |
| **NovelmiRNA-164** | 34 | 0 | 0 | 0 |
| **NovelmiRNA-225** | 32 | 0 | 0 | 0 |
| **NovelmiRNA-174** | 30 | 0 | 0 | 0 |
| **hsa-miR-708-5p** | 22 | 0 | 2 | 0 |
| **hsa-miR-124-3p** | 21 | 0 | 2 | 1 |
| **NovelmiRNA-150** | 19 | 0 | 0 | 0 |
| **hsa-miR-135a-5p** | 17 | 27 | 0 | 0 |
| **NovelmiRNA-64** | 16 | 0 | 0 | 0 |
| **NovelmiRNA-92** | 16 | 0 | 0 | 0 |

(continued)

| | Manufacturing Example 1 | Comparative Example 2 | Comparative Example 1 | Comparative Example 3 |
|---|---|---|---|---|
| **NovelmiRNA-168** | 16 | 0 | 0 | 0 |
| **NovelmiRNA-22** | 14 | 0 | 0 | 0 |
| **NovelmiRNA-35** | 13 | 0 | 0 | 0 |
| **NovelmiRNA-161** | 12 | 0 | 0 | 0 |
| **hsa-miR-3940-3p** | 11 | 0 | 0 | 0 |
| **hsa-miR-219a-3p** | 11 | 0 | 0 | 0 |
| **hsa-miR-212-3p** | 11 | 0 | 0 | 5 |
| **NovelmiRNA-76** | 10 | 0 | 0 | 0 |
| **hsa-miR-323a-3p** | 9 | 6 | 0 | 1 |
| **hsa-miR-654-3p** | 7 | 8 | 0 | 0 |
| **hsa-miR-204-5p** | 7 | 1 | 0 | 1 |
| **hsa-miR-136-3p** | 7 | 1 | 0 | 0 |
| **NovelmiRNA-39** | 7 | 0 | 0 | 0 |
| **NovelmiRNA-175** | 7 | 0 | 0 | 0 |
| **hsa-miR-134-5p** | 6 | 23 | 2 | 0 |
| **hsa-miR-454-3p** | 5 | 0 | 1 | 0 |
| **NovelmiRNA-286** | 5 | 3 | 0 | 0 |
| **hsa-miR-542-3p** | 4 | 0 | 0 | 0 |
| **hsa-miR-433-3p** | 4 | 0 | 0 | 0 |
| **hsa-miR-376a-2-3p** | 1 | 35 | 0 | 0 |
| **hsa-miR-376a-3p** | 1 | 35 | 0 | 0 |
| **hsa-miR-664a-5p** | 1 | 8 | 0 | 0 |
| **hsa-miR-150-3p** | 0 | 4 | 0 | 0 |
| **hsa-miR-3909** | 0 | 5 | 0 | 0 |
| **hsa-miR-618** | 0 | 6 | 0 | 0 |
| **NovelmiRNA-52** | 0 | 29 | 0 | 0 |
| **NovelmiRNA-172** | 0 | 550 | 0 | 0 |
| **NovelmiRNA-218** | 0 | 42 | 0 | 0 |
| **NovelmiRNA-220** | 0 | 11 | 0 | 0 |
| **NovelmiRNA-222** | 0 | 53 | 0 | 0 |
| **NovelmiRNA-266** | 0 | 19 | 0 | 0 |
| **NovelmiRNA-283** | 0 | 532 | 0 | 0 |

As shown in Table 1, it was confirmed that the expression intensities of NovelmiRNA-67, hsa-miR-199a-3p, NovelmiRNA-298, hsa-miR-145-5p, NovelmiRNA-281, NovelmiRNA-109, hsa-miR-432-5p, NovelmiRNA-230, NovelmiRNA-88, hsa-miR-200b-3p, NovelmiRNA-169, hsa-miR-200a-3p, hsa-miR-487b-3p, hsa-miR-214-3p, NovelmiRNA-208, NovelmiRNA-164, NovelmiRNA-225, NovelmiRNA-174, hsa-miR-708-5p, hsa-miR-124-3p, NovelmiRNA-150, hsa-miR-135a-5p, NovelmiRNA-64, NovelmiRNA-92, NovelmiRNA-168, NovelmiRNA-22, NovelmiRNA-35, NovelmiRNA-161, hsa-miR-3940-3p, hsa-miR-219a-3p, hsa-miR-212-3p, NovelmiRNA-76, hsa-miR-323a-3p, hsa-miR-654-3p, hsa-miR-204-5p, hsa-miR-136-3p, NovelmiRNA-39, NovelmiRNA-175, hsa-miR-134-5p, hsa-miR-454-3p, NovelmiRNA-286, hsa-miR-542-3p, and hsa-miR-433-3p were specifically increased in the exosomes isolated in Manufacturing

Example 1 (CHEM Exo) compared to the exosomes isolated in Comparative Examples 1 to 3.

Specifically, it was confirmed that the expression intensities of hsa-miR-199a-3p and hsa-miR-200b-3p, known to inhibit cell proliferation and induce apoptosis, hsa-miR-145-5p, hsa-miR-432-5p and hsa-miR-200a-3p, known to inhibit cell migration and invasion, hsa-miR-214-3p and hsa-miR-708-5p, known to inhibit cell proliferation and cell cycle progression, hsa-miR-487b-3p, known to inhibit chemoresistance and metastasis of osteosarcoma, hsa-miR-124-3p, known to function as a tumor suppressor, and hsa-miR-135a-5p, known to inhibit tumor invasion, were specifically increased.

Meanwhile, it was confirmed that the expression intensities of hsa-miR-376a-2-3p, hsa-miR-376a-3p, hsa-miR-664a-5p, hsa-miR-150-3p, hsa-miR-3909, hsa-miR-618, NovelmiRNA-52, NovelmiRNA-172, NovelmiRNA-218, NovelmiRNA-220, NovelmiRNA-222, NovelmiRNA-266, and NovelmiRNA-283 were relatively decreased in the exosomes isolated from human Wharton's jelly-derived mesenchymal stem cells treated with zymosan, Poly I:C, and monophosphoryl lipid A (MPLA) in Manufacturing Example 1 (CHEM Exo) compared to the exosomes isolated from human Wharton's jelly-derived mesenchymal stem cells treated with Poly I:C in Comparative Example 2 (POLY IC Exo).

### Experimental Example 4. Analysis of mRNAs present in exosomes isolated from Wharton's jelly-derived mesenchymal stem cells treated with chemical substance cocktail

In order to compare the mRNA expression patterns of exosomes isolated from human Wharton's jelly-derived mesenchymal stem cells treated with a chemical substance cocktail in Manufacturing Example 1 and exosomes isolated from human Wharton's jelly-derived mesenchymal stem cells in Comparative Example 1, a microarray experiment was performed. Specifically, using the Extracellular vesicle characterization service of Creative Biolabs, mRNAs contained in each exosome sample were extracted, and mRNAs that were relatively more expressed were analyzed through microarray. Data analysis was performed from August to September 2021. As a result of performing microarray, it was confirmed that the expression levels of a total of 275 types of mRNA, including MED13L, were significantly increased in human Wharton's jelly-derived mesenchymal stem cells treated with a chemical substance cocktail. Table 2 below lists 275 types of mRNA.

[Table 2]

| | | | | |
|---|---|---|---|---|
| MED13L | lnc-EXT1-2 | LOC100507420 | lnc-PHYHD1-1 | SHARPIN |
| lnc-TOMM20L-1 | LIN54 | OPA3 | GIGYF2 | ASMTL-AS1 |
| lnc-CDKSR1-1 | C17orf74 | XLOC_l2_014245 | RNF151 | HMOX1 |
| ETV3 | SLC12A2 | lnc-NRSA1-1 | BAGE | SHARPIN |
| SLC35G1 | TTC28 | DMTN | ADRA2C | LOC101928207 |
| SMARCA4 | ADRA2A | SMPD4 | lnc-SLC43A1-1 | TRAIP |
| ACAT2 | NLGN2 | TXLNB | ENTPD8 | lnc-ANTXRL-2 |
| ADAM12 | lnc-FBXO25-4 | SPRR1A | NYAP1 | LINC01135 |
| ARFGEF1 | LOC100506603 | DISP1 | lnc-GLIPR1L1-1 | BZRAP1 |
| CELF3 | LOC399886 | LOC100130285 | CCNT2-AS1 | LOC101926983 |
| DNAJB5 | RCC2 | lnc-SLC17A9-1 | NAV1 | WDR6 |
| lnc-WRNIP1-2 | KIAA0040 | lnc-SELV.1-1 | TBX21 | C19orf81 |
| ACTL8 | HOXB9 | lnc-ROM1-2 | GNAZ | NAMA |
| lnc-TM2D3-2 | JMJD8 | lnc-RABEPK-1 | DNM3OS | LIM2 |
| GCN1L1 | LINC01146 | lnc-RRP8-1 | PRKAG2-AS1 | FAM155A-IT1 |
| LOC101929337 | lnc-GIT1-1 | lnc-RP11-234B24.6.1-1 | lnc-MFSD9-4 | lnc-RPP30-2 |
| FAM217A | VRTN | lnc-TMEM189-UBE2V1-2 | lnc-RPGRIP1-2 | ZNF264 |
| XLOC_l2_013467 | NHP2 | LAMTOR2 | ABCC6 | MALAT1 |
| SNHG4 | KIF26A | BTK | LRFN3 | HTT-AS |
| NUDT13 | HAUS5 | lnc-BSND-1 | lnc-COL1A1-2 | ZDHHC22 |
| MCTS1 | lnc-SOD2-2 | C2orf71 | lnc-PPAPDC3-2 | SEPHS2 |

(continued)

| | | | | |
|---|---|---|---|---|
| ATG4D | APOL3 | lnc-C17orf63-2 | CTU1 | ABTB1 |
| NPEPL1 | lnc-POLR2L-1 | lnc-CHGA-1 | lnc-PABPC4-1 | lnc-RP11-791J7.2.1-1 |
| YEATS2 | lnc-SUPT3H-1 | ABCD1 | LOC100507165 | CCDC28B |
| SPEN | lnc-XRN2-3 | NCAN | LOC102724861 | LOC284263 |
| GGT6 | RTP2 | C9orf106 | SPTSSB | lnc-PPIC-2 |
| lnc-RP11-770J1.4.1-1 | TERT | AGAP2 | lnc-C20orf96-4 | CDC42BPB |
| MIAT | TNFRSF12A | EMC6 | DCDC5 | C9orf173-AS1 |
| lnc-HMCN1-2 | LOC645427 | CD86 | lnc-CRYL1-1 | C9orf139 |
| RETN | lnc-CTR9-5 | LOC100129175 | LOC100506114 | FGD5 |
| SP6 | SENP7 | SLC48A1 | CCNT2-AS1 | lnc-RP11-322L20.1.1-7 |
| RCOR2 | lnc-C17orf97-4 | RPL28 | lnc-SCAMP5-1 | RAD51 |
| lnc-AC016251.1-2 | ARNTL | BGN | BRD1 | MEIOB |
| lnc-PRKAA2-1 | lnc-RTL1-1 | BZW1 | lnc-ELAVL4-3 | lnc-ADAMTS14-1 |
| RANBP3 | ZNF713 | lnc-C6orf221-2 | CHRDL1 | PCDHA13 |
| lnc-SH2B3-1 | XLOC_-l2_005871 | SLC20A2 | lnc-FUT8-1 | |
| XLOC_l2_011901 | QSOX1 | HYALP1 | lnc-DNAJC7-1 | |
| CACNA1H | SP2-AS 1 | SNAR-G1 | LINC01264 | |
| LINC01314 | ARSA | SEC22A | LMO2 | |
| lnc-VAPB-1 | PHF7 | TIMM8B | LINC01197 | |
| ARHGAP23 | GS1-24F4.2 | ATAD1 | LOC101927533 | |
| ATG9A | LRIT1 | FNDC7 | REP15 | |
| GM2A | PLLP | B3GNT8 | C1orf226 | |
| XLOC_l2_013547 | SLC4A11 | STARD7-AS1 | lnc-STEAP1B-1 | |
| lnc-SLC25A26-1 | C11orf95 | TMEM100 | SNORD3B-1 | |
| TMEM240 | FAM110B | LARP6 | lnc-TMEM135-2 | |
| CLSTN3 | IER5 | HELZ2 | lnc-FAM160A1-1 | |
| ATG16L2 | LINC01508 | TMX2 | GOLGA2P5 | |
| MMP11 | lnc-GTPBP8-1 | lnc-EPHB6-1 | TMEM132C | |
| TSSK3 | LOC400958 | PSMC2 | GPX3 | |
| lnc-SOX6-1 | WFDC21P | lnc-LINC00273-1 | lnc-DCAF4L2-2 | |
| EDEM3 | lnc-CPXM2-2 | TNFRSF25 | XLOC_-l2_014217 | |
| LOC101927210 | P2RX6 | BCL3 | PPT1 | |
| C14orf169 | SLC22A7 | LOC101929450 | RPA4 | |
| EBLN2 | lnc-PPP2R2C-1 | LOC102724416 | KCTD5 | |
| lnc-XRN1-1 | lnc-GATA5-2 | FLII | lnc-UQCRFS1-9 | |
| lnc-FTSJD2-1 | MARVELD1 | WAC | MTUS2-AS1 | |
| MDFI | lnc-TRIM41-3 | PPP6R1 | CHKA | |
| LOC102546226 | PRSS8 | LOC100507377 | TBC1D31 | |
| SLC5A10 | SEMA6C | LRRFIP2 | SNORD38A | |

**Experimental Example 5. Confirmation of *in vitro* effect of exosomes isolated from Wharton's jelly-derived mesenchymal stem cells treated with chemical substance cocktail**

**Experimental Example 5.1. Culturing of mouse macrophage cell line and exosome treatment**

RAW 264.7 (ATCC, TIB-71), a mouse macrophage cell line, was cultured in Dulbecco's modified Eagle medium with high glucose (DMEM; Welgene, South Korea) medium supplemented with 10% heat-treated fetal bovine serum (Corning, USA) and 1% penicillin-streptomycin at 37 °C and 5% $CO_2$ conditions.

RAW 264.7 cells were seeded at $1 \times 10^5$ cells per well in a 48-well plate (Corning, USA) and cultured for 24 hours in serum free DMEM medium. The medium was supplemented with either no treatment(CTRL), $1 \times 10^8$ exosomes isolated from human Wharton's jelly-derived mesenchymal stem cells in Comparative Example 1 (CTRL Exo), $1 \times 10^8$ exosomes isolated from human Wharton's jelly-derived mesenchymal stem cells treated with a chemical substance cocktail in Manufacturing Example 1 (CHEM Exo), or 10 ng/mL of lipopolysaccharide (Sigma-Aldrich, USA) (LPS).

**Experimental Example 5.2. Analysis of amount of nitric oxide production**

In order to analyze the amount of nitric oxide (NO) production of RAW 264.7 following CTRL, CTRL Exo, CHEM Exo or LPS treatment, the culture media obtained from cells treated with CTRL, CTRL Exo, CHEM Exo, and LPS, respectively, were transferred to new 96-well plates (VWR, USA), and then the NO concentration was measured using a Nitric Oxide plus detection kit (iNtRON Biotech, South Korea), and the results are shown in Figure 5.

As shown in Figure 5, it was confirmed that the amount of nitric oxide production was significantly increased when treated with CHEM Exo compared to when an inflammatory response was induced by treatment with LPS. This confirmed that the exosomes isolated from human Wharton's jelly-derived mesenchymal stem cells treated with a chemical substance cocktail in Manufacturing Example 1 induce macrophage activation through the secretion of nitric oxide, thereby enhancing the inflammatory response.

**Experimental Example 5.3. Measurement of expression level of inflammation-related mRNA**

In order to analyze the expression levels of cytokines and chemokines in RAW 264.7 following the treatment with CTRL, CTRL Exo, CHEM Exo, or LPS, real-time polymerase chain reaction (quantitative reverse transcription PCR, RT-qPCR) was performed. Specifically, the cells treated with CTRL, CTRL Exo, CHEM Exo, and LPS, respectively, were lysed, and then RNAs were extracted and purified using TaKaRa MiniBEST universal RNA extraction kit (Takara Bio Inc., Japan). Thereafter, complementary DNAs (cDNA) of the RNAs were synthesized using the PrimeScript 1st strand cDNA synthesis kit (Takara Bio Inc., Japan). The expression levels of mouse interleukin-6 (IL-6), IL-1β, tumor necrosis factor-α (TNF-α), nitric oxide synthase 2 (NOS2), and cyclooxygenase 2 (COX2) were measured by performing RT-qPCR with the synthesized cDNAs, and the results are shown in Figure 6. GAPDH was used as a reference gene for normalization of gene expression levels, and the sequence information of the primers used for RT-qPCR is as shown in Table 3 below.

[Table 3]

| Gene | Primer | | SEQ ID NO | Annealing Temperature (°C) | Size (bp) |
|---|---|---|---|---|---|
| GAPDH | Forward | CAT CAC TGC CAC CCA GAA GAC TG | 1 | 63.3 | 153 |
| | Reverse | ATG CCA GTG AGC TTC CCG AG | 2 | 65.2 | |
| IL-6 | Forward | AGT TGC CTT CTT GGG ACT GAT | 3 | 59.3 | 159 |
| | Reverse | TCC ACG ATT TCC CAG AGA ACA | 4 | 59 | |
| IL-12 | Forward | AGG TCA CAC TGG ACC AAA GG | 5 | 60.5 | 173 |
| | Reverse | TGG TTT GAT GAT GTC CCT GA | 6 | 56.4 | |
| IL-1β | Forward | TTC CTG TTG TCT ACA CCAATG C | 7 | 60.3 | 162 |
| | Reverse | CGG GCT TTA AGT GAG TAG GAG A | 8 | 62.1 | |
| TNF-α | Forward | ACG GCA TGG ATC TCA AAG AC | 9 | 60.1 | 116 |
| | Reverse | GTG GGT GAG GAG CAC GTA GT | 10 | 60.2 | |
| NOS2 | Forward | GAA CTG TAG CAC AGC ACA GGA AAT | 11 | 61.6 | 158 |
| | Reverse | CGT ACC GGA TGA GCT GTG AAT | 12 | 60.2 | |

(continued)

| Gene | Primer | | SEQ ID NO | Annealing Temperature (°C) | Size (bp) |
|---|---|---|---|---|---|
| COX2 | Forward | CAG TTT ATG TTG TCT GTC CAG AGT TTC | 13 | 60.5 | 127 |
| | Reverse | CCA GCA CTT CAC CCA TCA GTT | 14 | 60.6 | |

As shown in Figure 6, it was confirmed that the expression intensities of the inflammatory cytokines IL-6, IL-12, TNF-$\alpha$, and IL-1$\beta$ and the inflammation-related enzymes NOS2 and COX2 were significantly increased when treated with CHEM exosomes compared to when treated with CTRL or CTRL Exo. In addition, it was confirmed that the expression of IL-6, IL-12, IL-1$\beta$, NOS2, and COX2 was significantly increased compared to treatment with LPS, which is generally known to induce an inflammatory response, indicating that the intensity of the immune response induced by CHEM Exo is significant. All of the increased cytokines are pro-inflammatory cytokines, which activate dendritic cells, macrophages, T cells, and B cells. Therefore, these experimental results suggest that CHEM Exo of Manufacturing Example 1 can induce immune cell activation. In addition, the results showing increased secretion of proinflammatory cytokines compared to LPS suggest that the immune activation effect of CHEM Exo of Manufacturing Example 1 is very excellent.

**Experimental Example 6. Confirmation of *in vivo* effect of exosomes isolated in bulk from Wharton's jelly-derived mesenchymal stem cells treated with chemical substance cocktail**

**Experimental Example 6.1. Preparation of mouse colorectal cancer cancer cell line**

The mouse colorectal cancer cancer cell line CT26 (ATCC, CRL-2638) was suspended in RPMI1640 medium supplemented with 10% heat-treated fetal bovine serum (Gibco, 10082-147) and 1% penicillin-streptomycin, placed in a cell culture flask, and cultured in an incubator at 37 °C and 5% $CO_2$ conditions. After the culture was completed, the cells were washed with PBS, and then 10-fold diluted 2.5% Trypsin-EDTA (Gibco, 15090) was added to detach the cells. Thereafter, centrifugation was performed at 1,000 rpm for 5 minutes, the supernatant was removed, and the resulting cell suspension was transferred to a fresh medium. The cell viability was confirmed using a microscope, and then the cell line was prepared by diluting it in DPBS to a concentration of $5.0 \times 10^6$ cells/mL.

**Experimental Example 6.2. Preparation of mouse colorectal cancer cancer model**

BALB/c mice were acclimatized to the breeding environment for 7 days, and then the day before transplantation of the CT26 cell line, the dorsal skin of the mice was shaved using an electric razor. When transplanting the cell line, the injection site was disinfected with 70% alcohol, the skin on the right dorsal side of the mouse was pulled with the thumb and index finger to create a space between the skin and muscle, and then the cells were administered subcutaneously using an insulin syringe into the subcutaneous space between the thumb and index finger from the anterior aspect of the animal at a dose of $5.0 \times 10^5$ cells/0.1 mL/head. After cell line inoculation, when the tumor size at the transplantation site reached about 300 $mm^3$, the mice were divided into seven groups of six mice per group so that the tumor sizes in each group were evenly distributed.

**Experimental Example 6.3. Administration of exosomes to a mouse colorectal cancer cancer model**

The seven groups divided above were administered with PBS, exosomes isolated in bulk from human Wharton's j elly-derived mesenchymal stem cells in Comparative Example 4 (Ctrl Exo), anti-PD-1 antibody (BioXCell, #BP0273), exosomes isolated in bulk from human Wharton's jelly-derived mesenchymal stem cells treated with a chemical substance cocktail in Manufacturing Example 2 (IEP-01), exosomes isolated from iPSC-derived mesenchymal stem cells treated with a chemical substance cocktail in Comparative Example 5 (IEP-01-i), a combination of IEP-01 and an anti-PD-1 antibody, and a combination of IEP-01-i and an anti-PD-1 antibody, respectively. The substances were administered three times a week for a total of six times over two weeks. CTRL Exo, IEP-01, and IEP-01-i were administered intravenously at a dose of $5 \times 10^8$ particles/head, and the anti-PD-1 antibody was administered intraperitoneally at a dose of 5 mg/kg.

**Experimental Example 6.4. Measurement of body weight and tumor volume of mouse colorectal cancer cancer model**

The body weight and tumor volume were measured three times a week from the start date of administration of the above substances. The tumor volume was calculated by measuring the long axis and short axis of the tumor using a caliper

and calculating the tumor volume using the following equation, and the results are shown in Figures 7 and 8. In addition, the body weight of each mouse was measured, and the results are shown in Figure 9.

[Equation 1]

Tumor volume (㎣) = $ab^2/2$ (a: long axis length, b: short axis length)

As shown in Figures 7 and 8, when administered alone, the tumor growth inhibitory effect was the most excellent in the group administered with exosomes isolated in bulk from human Wharton's jelly-derived mesenchymal stem cells treated with a chemical substance cocktail in Manufacturing Example 2 (IEP-01), and it was confirmed that the tumor growth inhibitory effect was increased when the exosomes isolated from mesenchymal stem cells treated with a chemical substance cocktail (IEP-01 or IEP-01-i) were administered in combination with the anti-PD-1 antibody rather than when administered alone. Meanwhile, as shown in Figure 9, it was confirmed that there was almost no change in the body weight of the mice depending on each administered substance.

**Experimental Example 6.5. Measurement of tumor weight of mouse colorectal cancer cancer model**

Fifteen days after the start date of administration of the above substances, all surviving mice were anesthetized, then blood was collected from the posterior vena cava using a syringe, the abdominal aorta and posterior vena cava were cut to exsanguineate and kill the mice, and then the tumors were extracted. The extracted tumors were lightly washed with PBS, dried, and photographed. The results are shown in Figure 11. The weights were measured, and the results are shown in Figure 10.

As shown in Figures 10 and 11, the tumor weight of the group administered with exosomes isolated in bulk from human Wharton's jelly-derived mesenchymal stem cells treated with a chemical substance cocktail in Manufacturing Example 2 (IEP-01) was the lowest, and it was confirmed that the tumor reduction effect was increased when the exosomes isolated from iPSC-derived mesenchymal stem cells treated with a chemical substance cocktail in Comparative Example 5 (IEP-01-i) were administered in combination with the anti-PD-1 antibody rather than when administered alone.

**Experimental Example 6.6. Functional Comparison of exosomes isolated from human Wharton's jelly-derived mesenchymal stem cells treated with Poly I:C alone and treated with chemical substance cocktail**

In order to confirm whether the proinflammatory cytokine secretion effect of exosomes isolated in bulk from human Wharton's jelly-derived mesenchymal stem cells treated with a chemical substance cocktail in Manufacturing Example 2 (IEP-01) results from the exosomes themselves or the chemical substance cocktail used in Manufacturing Example 2, the following *in vitro* experiment was performed.

Specifically, the RAW 264.7 cell line was treated with exosomes isolated in bulk in Manufacturing Example 2 (IEP-01) or with Poly I:C alone, and then RT-PCR was performed in the same manner as described in Experimental Example 5.3. to measure the expression level of IL-6, a proinflammatory cytokine, and the results are shown in Figure 12.

As shown in Figure 12, when the concentration of Poly I:C was calculated based on the number of the treated IEP-01, it was confirmed that the expression of IL-6 was increased at a low concentration when treated with IEP-01 compared to when treated with Poly I:C alone. Specifically, it was confirmed that when the expression level of IL-6 mRNA was 4,000, 35 μg of Poly I:C was required, but in the case of IEP-01, 13.8 μg of Poly I:C was used. These results demonstrate that IEP-01 can achieve the same effect with Poly I:C at a concentration 2.5 times lower than when Poly I:C is used alone. In other words, it was confirmed that the proinflammatory cytokine secretion of IEP-01 was not the effect of Poly I:C alone, but rather the result of the combined action of cytokines, miRNA, and mRNA present in IEP-01.

**Experimental Example 7. Comparative experiment of exosomes by cell type obtained**

**Experimental Example 7.1. Isolation of exosomes from cells treated with a chemical substance cocktail**

The WJ-MSC-derived exosomes, the exosomes derived from ciMSC differentiated from the iPSCs, and the exosomes obtained from the HEK293T cell line were compared by the treated chemical substance.

One day before obtaining the cell culture solution, the culture solution of the cells being grown was removed, washed with PBS, and the culture medium containing no FBS was used as the naive exosome group. In addition, exosomes obtained from the 3-chem group treated with poly I:C 30 μg/ml, MPLA 1 μg/ml, and Zymosan 1 μg/ml were designated as IEP-01. In addition, exosomes obtained from the 2-chem group treated with poly I:C 30 μg/ml and MPLA 1 μg/ml were designated as IEP-01'. Exosomes obtained from ciMSCs were additionally indicated with "i", and exosomes obtained from HEK293T were additionally indicated with "h". The cell culture solution was collected from each experimental group after culturing for 24 hours.

The exosome samples from the cell culture solution were isolated using tangential flow filtration. In order to label the exosomes, the exosome samples were cultured with PKH67 lipophilic fluorescent dye for 5 minutes at room temperature, and then washed twice with PBS containing a sufficient amount of 10% BSA. The labeled exosomes were concentrated using an Amicon 100 kDa MWCO filter column and quantified by NTA.

**Experimental Example 7.2. Analysis of mRNAs present in each exosome**

Raw264.7 cells, a mouse-derived macrophage cell line, were treated with the exosome 1000 ptc/cell that had been obtained from each cell group treated with compounds, or 1 ng/mL of LPS for 24 hours. Thereafter, mRNA was isolated from Raw264.7 cells, and then cDNA was synthesized through reverse transcription. qRT-PCR was performed using TaqMan probes to confirm the expression of IL-6, iNOS2, and IL-1β using the synthesized cDNA. LPS was used as a positive control group to activate Raw264.7. The expression of each gene was quantified as a relative value based on GAPDH.

As shown in Figures 13a to 13c, it was confirmed that the expression of IL-6, IL-1β, or iNOS2 was not induced in Raw264.7 cells from the group treated with naive exosomes among the WJ-MSC-derived exosomes, whereas the expression of IL-6, IL-1β, or iNOS2 was induced more strongly in the exosomes obtained from the groups treated with 2-chem and 3-chem than that induced by LPS.

In addition, as shown in Figures 13d and 13e, similar to the exosomes obtained from WJ-MSCs, it was confirmed that the expression of IL-6 or iNOS was not induced in the group treated with naive exosomes among the ciMSC-derived exosomes, whereas the expression of IL-6 or iNOS2 was induced more strongly in the exosomes obtained from the the group treated with 2-chem and 3-chem than that induced by LPS.

In addition, as shown in Figures 13f and 13g, similar to the exosomes obtained from WJ-MSCs and ciMSCs, the expression of IL-6 and IL-1β was not induced in the group treated with naive exosomes among the exosomes derived from HEK293T cell line, whereas a significant increase in the expression of IL-6 or IL-1β was observed in the exosomes obtained from the groups treated with 2-chem and 3-chem.

These results suggest that treatment of various cell lines with polyI:C and MPLA can secrete exosomes capable of activating Raw264.7 macrophages.

**Experimental Example 8. Confirmation of degree of uptake of ciMSC-derived exosomes by cell types**

The degree of uptake of ciMSC-derived exosomes was compared by PBMC and immune cell types.

For exosome uptake experiment, human peripheral blood mononuclear cells (CTLs) were cultured with labeled ciMSC-derived exosomes or unlabeled ciMSC-derived exosomes prepared above at a concentration of 3000 particles/cell in a $CO_2$ incubator at 37 °C for 4 hours and washed twice with PBS. For immunotyping, the cells were cultured with fluorescence-labeled antibodies at 4 °C for 3 minutes, then washed with PBS containing 2% FBS, and analyzed using a Cytoflex flow cytometer. All data were analyzed using Kaluza software.

At this time, for immunotyping, APC anti-human CD3 (Biolegend, 300312), APC anti-human CD19 (Biolegend, 302212), Brilliant Violet 421™ anti-human CD19 (Biolegend, 302233), Brilliant Violet 421™ anti-human CD56 (NCAM) (Biolegend, 362551), Brilliant Violet 421™ anti-human CD11b (Biolegend, 301323), Brilliant Violet 421™ anti-human CD11c (Biolegend, 301627), PerCP/Cyanine5.5 anti-human CD14 (Biolegend, 367110), PE anti-human HLA-DR, DP, DQ (MHC class II) (Biolegend, 361716) antibodies were used.

As shown in Figures 14a to 14c, it was confirmed that the uptake of ciMSC-derived exosomes labeled with PKH fluorescence was significantly increased, especially in dendritic cells, monocytes, and macrophages among PBMCs.

**Experimental Example 9. Confirmation of macrophage activation and differentiation by ciMSC-derived exosomes**

**Experimental Example 9.1. Confirmation of macrophage activation by exosomes**

In order to confirm whether ciMSC-derived exosomes activate macrophages, Raw264.7 cells, a mouse-derived macrophage cell line, were treated with 10 ng/ml of LPS or 1000 ptc/ml of IEP-01i' (2-chem-treated ciMSC-derived exosomes) for 24 hours. After treatment, the cells were washed with PBS and cultured with PE anti-mouse CD80 (Biolegend, 104708) antibody at 4 °C for 30 minutes. Next, they were washed with PBS containing 2% FBS and analyzed using a Cytoflex flow cytometer. All data were analyzed using Kaluza software.

As shown in Figures 15a and 15b, it was confirmed that IEP-01i' induced the activation of macrophages as well as LPS, a positive control group.

**Experimental Example 9.2. Confirmation of macrophage differentiation by exosomes**

In order to confirm macrophage differentiation by ciMSC-derived exosomes, Raw264.7 cells, a mouse-derived macrophage cell line, were pretreated with 20 ng/ml IL-4 for 24 hours to induce the differentiation into M2 macrophages. Thereafter, the cells were treated with 10 ng/ml of LPS or 1000 ptc/ml of IEP-01i’ (2-chem-treated ciMSC-derived exosomes) for 24 hours. After treatment, the cells were washed with PBS and cultured with PE anti-mouse CD80 (Biolegend, 104708) antibody for 30 minutes at 4 °C, and then they were washed with PBS containing 2% FBS and analyzed using a Cytoflex flow cytometer. All data were analyzed using Kaluza software.

As shown in Figures 15c and 15d, it was confirmed that when treated with IL-4, differentiation into M2 macrophages occurred, and M1 macrophage markers were decreased, whereas when treated with IEP-01i’, the expression of CD80, an M1 macrophage marker, was strongly induced, as when treated with LPS.

These results indicate that differentiation into M2 macrophages is inhibited by IEP-01i’ and differentiation into M1 macrophages is induced.

**Experimental Example 10. Confirmation of dendritic cell activation by ciMSC-derived exosomes**

In order to confirm dendritic cell activation by ciMSC-derived exosomes, fluorescence-labeled ciMSC-derived exosomes were prepared for each treated group as in Experimental Example 8 above.

Immature dendritic cells derived from human peripheral blood (Stem cell technologies, #70041) were treated with labeled naive or IEP-01i’ exosomes or unlabeled naive or IEP-01i’ exosomes at a concentration of 3000 particles/cell, cultured in a $CO_2$ incubator at 37 °C for 24 hours, and then washed twice with PBS. As a positive control group, immature dendritic cells were treated with 1 μg/mL of LPS for 24 hours to activate dendritic cells. In order to confirm dendritic cell activation, the cells were cultured with fluorescence-labeled antibodies at 4 °C for 30 minutes. Thereafter, they were washed with PBS containing 2% FBS and analyzed using a Cytoflex flow cytometer. All data were analyzed using Kaluza software.

APC anti-human CD11c (Biolegend, 301614), PE anti-human CD80 (Biolegend, 305208), PE anti-human CD86 (Biolegend, 374206), PE anti-human HLA-A,B,C (MHC class I) (Biolegend, 311406), PE anti-human HLA-DR, DP, DQ (MHC class II) (361716) antibodies were used for the analysis.

As shown in Figures 16a to 16d, PKH-positive dendritic cells were confirmed in the group treated with PKH-labeled exosomes.

As shown in Figures 16e and 16f, it was confirmed that CD80, a dendritic cell activation marker, was expressed more in the dendritic cell group treated with IEP-01i’ exosomes than in the untreated dendritic cells. In particular, it was confirmed that the expression of CD80 was significantly increased specifically in the dendritic cells labeled with PKH, i.e., the dendritic cells that had absorbed the exosomes.

In addition, as shown in Figures 16g and 16h, it was confirmed that CD86, a dendritic cell activation marker, was expressed more in the dendritic cell group treated with IEP-01i’ exosomes than in the untreated dendritic cells. In particular, it was confirmed that the expression of CD86 was significantly increased specifically in the dendritic cells labeled with PKH, i.e., the dendritic cells that had absorbed the exosomes. In addition, it was confirmed that CD86 was expressed more highly in the dendritic cells treated with IEP-01i’ compared to the dendritic cells treated with naive exosomes.

As shown in Figures 16i and 16j, it was confirmed that MHC class I, an antigen presenting molecule, was expressed more highly in the dendritic cell group treated with IEP-01 i’ exosomes compared to the untreated dendritic cells. In particular, it was confirmed that the expression of MHC class I was significantly increased specifically in the dendritic cells labeled with PKH, i.e., the dendritic cells that had absorbed the exosomes.

As shown in Figures 16k and 16l, it was confirmed that MHC class II, an antigen presenting molecule, was expressed more highly in the dendritic cell group treated with IEP-01 i’ exosomes compared to the untreated dendritic cells. In particular, it was confirmed that the expression of MHC class II was significantly increased specifically in the dendritic cells labeled with PKH, i.e., the dendritic cells that had absorbed the exosomes. In addition, it was confirmed that significantly more MHC class II was expressed in the dendritic cells treated with IEP-01i’ compared to the dendritic cells treated with naive exosomes.

**Experimental Example 11. Analysis of cytokines of ciMSC-derived exosomes**

In order to analyze cytokines contained in ciMSC-derived exosomes, the ciMSC-derived exosome sample of Experimental Example 7.1 above was prepared at a concentration of $1 \times 10^{10}$ ptc/ml. Thereafter, 1% Triton X-100 was added and treated at room temperature for 10 minutes to lyse the exosomes. Next, the cytokines inside the exosomes were identified and the amount of cytokines was quantified using the Legendplex human essential immune response panel (Biolegend, 740930). The analysis was performed according to the manufacturer’s manual, and the analysis of all samples was performed in triplicate.

As shown in Figures 17a to 17e, it was confirmed that IEP-01i' contained significantly more IP-10, IL-6, MCP-1, and IL-8 than naive exosomes. On the other hand, the amount of TGF-β1 was much smaller than that of naive exosomes.
These results indicate that IEP-01i' contains cytokine molecules that can activate the chemoattraction of dendritic cells or macrophages. In addition, these results indicate that it contains less TGF-β1, which promotes the growth of cancer cells.
Therefore, it is predicted that the growth of cancer cells can be suppressed through the infiltration and activation of innate immune cells by cytokines of ciMSC-derived exosomes.

**Experimental Example 12. Animal experiment for colorectal cancer cancer using ciMSC-derived exosomes**

**Experimental Example 12.1. Single administration of ciMSC-derived exosomes**

In order to confirm the anticancer effect of single administration of ciMSC-derived exosomes, the exosomes were administered alone at various doses to the colorectal cancer cancer mice. The colorectal cancer cancer cells were constructed as in Example 6.1. above, and the colorectal cancer cancer mice were prepared as in Example 6.2. above.
During the acclimatization period, healthy animals were selected and cell line inoculation was performed. Thereafter, when the tumor size of the site transplanted with the cell line reached about 100 $mm^3$, the mice were grouped according to tumor size so that the tumor size of each group was distributed as evenly as possible. Administration was performed as shown in Figure 18a. Specifically, the test substance was administered intravenously at a low dose ($5 \times 10^8$ ptc/200 μl), a mid dose ($1.5 \times 10^9$ ptc/200 μl), and a high dose ($5 \times 10^9$ ptc/200 μl) once daily or once every two days, for a total of six times. The occurrence of tumors was checked three times a week from the date of group separation or from the start date of the test substance administration (Day 0), and the tumor size was measured. The tumor size was measured as in Example 6.4. above.
As shown in Figure 18b, it was confirmed that IEP-01i' exhibited an anticancer effect against CT26 in a dose-dependent manner regardless of the treatment interval. In addition, it was confirmed that IEP-01i' exhibited significant anticancer effects in the CT26 tumor model as a single administration.
In addition, as shown in Figures 18c to 18i, it was confirmed that IEP-01i' exhibited an anticancer effect against CT26 in a dose-dependent manner regardless of the treatment interval. In particular, it was confirmed that some subjects in the high-dose treated group showed complete response.

**Experimental Example 12.2. Combined administration of ciMSC-derived exosomes**

In order to confirm the anticancer effect when exosomes were administered alone or in combination with an immune checkpoint inhibitor, the colorectal cancer cancer mice were treated with an anti-PD-1 antibody, IEP-01, and IEP-01i alone or in combination. The experiment was performed in the same manner as in Experimental Example 12.1 above.
Administration was performed as shown in Figure 19a. Each test animal was treated three times a week, for a total of six times by administering intraperitoneally the anti-PD-1 antibody at 5 mg/kg and administering intravenously IEP-01 (WJ-MSC-derived exosome) or IEP-01i (ciMSC-derived exosome) at $5 \times 10^8$ ptc/200 μl. The occurrence of tumors was checked three times a week from the date of group separation or from the start date of the test substance administration (Day 0), and the tumor size was measured.
As shown in Figure 19b, some anticancer effects were observed in the group treated with IEP-01 or IEP-01i alone. In addition, it was confirmed that the anticancer effects were significant in the group treated with IEP-01 or IEP-01i in combination with an anti-PD-1 antibody. In some mouse subjects, complete response was observed by each treatment, and in particular, the most complete response (CR) was observed in the group treated with IEP-01 or IEP-01i in combination with an anti-PD-1 antibody. In the mouse models where complete response was observed, when CT26 cancer cells were re-injected, the cancer cells were observed to not grow, which means that immunological memory was formed in the subjects treated with each treatment.
In addition, as shown in Figures 19c to 19i, as a result of monitoring the tumor growth of each subject in a CT26 colorectal cancer cancer animal model, it was confirmed that there was a clear tumor growth inhibition, although there were differences among subjects.

**Experimental Example 13. Animal experiment for melanoma using ciMSC-derived exosomes**

In order to confirm the anticancer effect when ciMSC-derived exosomes were administered alone or in combination, the melanoma mice were treated with an anti-PD-1 antibody and IEP-01i alone or in combination.
Melanoma mice were constructed in the same manner as in Example 6.1. above except that the frozen mouse tumor cell line (B16F10 cell line) was used. In addition, experiments were prepared in the same manner as in Example 6.2.

above.

During the acclimatization period, healthy animals were selected and cell line inoculation was performed. Thereafter, when the tumor size of the site transplanted with the cell line reached about 100 $mm^3$, the mice were grouped according to tumor size so that the tumor size of each group was distributed as evenly as possible. Administration was performed as shown in Figure 20a. Each test animal was treated three times a week for 2 weeks, for a total of six times by administering intraperitoneally the anti-PD-1 antibody at 5 mg/kg and administering intravenously IEP-01i at $5 \times 10^8$ ptc/200 $\mu$l. The occurrence of tumors was checked three times a week from the date of group separation or from the start date of the test substance administration (Day 0), and the tumor size was measured.

As shown in Figure 20b, it was confirmed that when treated with IEP-01i and an anti-PD-1 antibody, respectively, the tumor size of B16F10 was reduced, and when treated in combination, tumor inhibition was more significant than in the group treated alone.

In addition, as shown in Figures 20c to 20i, in the groups treated with IEP-01i and an anti-PD-1 antibody alone or in combination, a number of subjects were observed to have slowed tumor growth rates. In addition, it was confirmed that a large number of mice (3/7) died in the group treated with PBS, whereas the number of dead subjects was lower in the groups treated with IEP-01 and an anti-PD-1 antibody alone or in combination (IEP-01i alone; 2/7, anti-PD-1 antibody alone; 1/7, IEP-01i in combination with anti-PD-1 antibody; 1/7).

**Experimental Example 14. Animal experiment for pancreatic cancer using ciMSC-derived exosomes**

In order to confirm the anticancer effect when ciMSC-derived exosomes were administered alone or in combination, the pancreatic cancer mice were treated with an anti-PD-1 antibody and IEP-01i alone or in combination.

Pancreatic cancer mice were prepared in the same manner except that the frozen mouse tumor cell line (Pan-02 cell line) produced as follows was used.

For cell line preparation, 1 vial of the frozen mouse tumor cell line (Pan-02 cell line) was placed in a cell culture flask containing RPMI1640 medium containing 10% heat-inactivated FBS (fetal bovine serum, Gibco, 10082-147) and cultured in a 5% $CO_2$ incubator at 37 °C. The cells were washed with PBS, and 2.5% Trypsin-EDTA (Gibco, 15090) was diluted 10 times. Thereafter, the cell line was separated by adding it and then subjected to centrifugation (1,000 rpm, 5 minutes), and the supernatant was discarded, and then the cell suspension was obtained with fresh medium. The viability was confirmed using a microscope, and then the cell line was prepared by diluting it in a 1:1 mixture of medium and Matrigel at a concentration of $5.0 \times 10^6$ cells/mL.

During the acclimatization period, healthy animals were selected and cell line inoculation was performed. Thereafter, when the tumor size of the site transplanted with the cell line reached about 100 $mm^3$, the mice were grouped according to tumor size so that the tumor size of each group was distributed as evenly as possible. Administration was performed as shown in Figure 20a. Each test animal was treated three times a week for 2 weeks, for a total of six times by administering intraperitoneally the anti-PD-1 antibody at 15 mg/kg and administering intravenously IEP-01i' at $1 \times 10^{10}$ ptc/200 $\mu$l. The occurrence of tumors was checked three times a week from the date of group separation or from the start date of the test substance administration (Day 0), and the tumor size was measured.

As shown in Figures 21b to 21f, it was confirmed that there was little difference in tumor growth rate in the group treated with an anti-PD-1 antibody compared to the negative control group treated with vehicle (PBS), whereas the tumor growth rate of pancreatic cancer was significantly slowed in the group treated with IEP-01 i' alone. In addition, it was confirmed that pancreatic cancer was inhibited the most in the group treated with IEP-01 i' in combination with an anti-PD-1 antibody.

These results indicate that IEP-01 i' can effectively inhibit tumor growth even in solid tumors such as pancreatic cancer that do not respond well to immune checkpoint inhibitors such as anti-PD-1 antibodies.

**Experimental Example 15. Evaluation of poly IC inclusion in IEP-01**

After purification of exosomes (IEP-01), samples before and after the purification process were analyzed using HPLC to determine whether poly IC peaks were detected.

As shown in Figures 22a to 22c, in the case of poly IC simply mixed in the medium, it was confirmed that all of poly IC was removed during the purification process, but in the case of IEP-01, poly IC was still detected even after the purification process.

These results indicate that poly IC is contained within IEP-01, and the comparison results with naive exosomes also indicate that poly IC exists only in IEP-01.

**Experimental Example 16. Analysis of miRNAs present in ciMSC-derived exosomes treated with chemical substance cocktail**

miRNAs present in exosomes were analyzed using Macrogen's Affymetrix miRNA 4.0 service.

Specifically, miRNAs contained in exosome samples were extracted, the library was constructed, and then sequencing was performed. By comparing the resulting sequence information with the existing miRbase database, the miRNAs that are relatively more expressed in each exosome sample were analyzed, and the results are shown in Figure 23. Ctrl_Exo is an exosome obtained from untreated ciMSCs, 3 chem Exo is an exosome obtained from ciMSCs that were treated with 3 chem, and 2 chem Exo is an exosome obtained from ciMSCs that were treated with 2 chem.

The results of measuring the types of miRNAs present in each exosome and their expression intensities are shown in Table 4 below.

[Table 4]

| Transcript ID (Array Design) | Ctrl Exo | 3 chem Exo | 2 chem Exo |
|---|---|---|---|
| hsa-miR-574-5p | 2.431340 | 10.242110 | 11.253710 |
| hsa-miR-6086 | 4.905830 | 10.407030 | 1.620650 |
| hsa-miR-4701-3p | 3.464640 | 8.666940 | 9.709270 |
| hsa-miR-3149 | 0.854740 | 6.039780 | 8.705470 |
| hsa-miR-4743-5p | 6.657570 | 11.590950 | 11.714050 |
| hsa-miR-8075 | 5.973260 | 10.838260 | 11.220240 |
| hsa-miR-6880-5p | 5.401840 | 10.000900 | 5.018530 |
| hsa-miR-3148 | 1.299280 | 5.893540 | 7.617760 |
| hsa-miR-6780b-5p | 6.760150 | 11.072200 | 9.817130 |
| hsa-miR-6846-5p | 6.528900 | 10.589760 | 10.657420 |
| hsa-mir-320e | 6.245890 | 10.228630 | 10.101240 |
| hsa-miR-32-3p | 1.483410 | 5.424420 | 7.875170 |
| hsa-miR-642a-3p | 7.378470 | 10.459880 | 10.819200 |
| hsa-miR-4505 | 10.281990 | 13.026020 | 12.986160 |
| hsa-miR-595 | 2.434340 | 5.102860 | 8.189200 |
| hsa-miR-4487 | 4.401570 | 6.858980 | 5.015790 |
| hsa-miR-4721 | 6.131810 | 8.575690 | 7.869180 |
| hsa-miR-7151-3p | 3.061450 | 5.424420 | 5.955770 |
| hsa-miR-4484 | 10.642280 | 12.915840 | 11.641950 |
| hsa-miR-642b-3p | 6.809470 | 9.076000 | 10.405250 |
| ENSG00000239154 | 3.881070 | 6.112830 | 5.860120 |
| hsa-miR-4706 | 7.523010 | 9.646450 | 7.645370 |
| hsa-miR-6126 | 10.413740 | 12.388690 | 12.388690 |
| hsa-miR-378h | 3.627520 | 5.496800 | 4.926410 |
| hsa-miR-6849-5p | 3.703310 | 5.424420 | 7.195840 |
| hsa-miR-7844-5p | 1.371450 | 3.089640 | 6.132980 |
| hsa-miR-4535 | 4.702010 | 6.373170 | 6.373170 |
| hsa-mir-9-1 | 2.941450 | 4.552650 | 5.270960 |
| hsa-miR-4459 | 8.104580 | 9.663110 | 8.935170 |
| hsa-miR-4443 | 5.790840 | 7.230830 | 12.209800 |
| hsa-miR-6732-5p | 9.461700 | 10.722030 | 11.155110 |

(continued)

| Transcript ID (Array Design) | Ctrl Exo | 3 chem Exo | 2 chem Exo |
|---|---|---|---|
| hsa-miR-3064-5p | 2.513040 | 3.733250 | 4.466610 |
| hsa-miR-3178 | 10.666070 | 11.761590 | 12.505970 |
| hsa-miR-3135b | 10.625810 | 11.636710 | 11.807040 |
| hsa-miR-4717-3p | 3.551030 | 4.180980 | 7.376090 |
| hsa-miR-206 | 1.761700 | 2.084930 | 4.191640 |
| hsa-miR-4529-3p | 2.432770 | 2.432770 | 6.025090 |
| hsa-miR-3613-5p | 2.030240 | 1.363560 | 4.762030 |

## Claims

**1.** An extracellular vesicle isolated from a human-derived cell, wherein the extracellular vesicle

i) comprises at least one miRNA selected from the group consisting of NovelmiRNA-67, hsa-miR-199a-3p, NovelmiRNA-298, hsa-miR-145-5p, NovelmiRNA-281, NovelmiRNA-109, hsa-miR-432-5p, NovelmiRNA-230, NovelmiRNA-88, hsa-miR-200b-3p, NovelmiRNA-169, hsa-miR-200a-3p, hsa-miR-487b-3p, hsa-miR-214-3p, NovelmiRNA-208, NovelmiRNA-164, NovelmiRNA-225, NovelmiRNA-174, hsa-miR-708-5p, hsa-miR-124-3p, NovelmiRNA-150, hsa-miR-135a-5p, NovelmiRNA-64, NovelmiRNA-92, NovelmiRNA-168, NovelmiRNA-22, NovelmiRNA-35, NovelmiRNA-161, hsa-miR-3940-3p, hsa-miR-219a-3p, hsa-miR-212-3p, NovelmiRNA-76, hsa-miR-323a-3p, hsa-miR-654-3p, hsa-miR-204-5p, hsa-miR-136-3p, NovelmiRNA-39, NovelmiRNA-175, hsa-miR-134-5p, hsa-miR-454-3p, NovelmiRNA-286, hsa-miR-542-3p, hsa-miR-433-3p, hsa-miR-574-5p, hsa-miR-6086, hsa-miR-4701-3p, hsa-miR-3149, hsa-miR-4743-5p, hsa-miR-8075, hsa-miR-6880-5p, hsa-miR-3148, hsa-miR-6780b-5p, hsa-miR-6846-5p, hsa-mir-320e, hsa-miR-32-3p, hsa-miR-642a-3p, hsa-miR-4505, hsa-miR-595, hsa-miR-4487, hsa-miR-4721, hsa-miR-7151-3p, hsa-miR-4484, hsa-miR-642b-3p, ENSG00000239154, hsa-miR-4706, hsa-miR-6126, hsa-miR-378h, hsa-miR-6849-5p, hsa-miR-7844-5p, hsa-miR-4535, hsa-mir-9-1, hsa-miR-4459, hsa-miR-4443, hsa-miR-6732-5p, hsa-miR-3064-5p, hsa-miR-3178, hsa-miR-3135b, hsa-miR-4717-3p, hsa-miR-206, hsa-miR-4529-3p, and hsa-miR-3613-5p, and

ii) comprises at least one mRNA selected from the group consisting of MED13L, lnc-TOMM20L-1, lnc-CDK5R1-1, ETV3, SLC35G1, SMARCA4, ACAT2, ADAM12, ARFGEF1, CELF3, DNAJB5, lnc-WRNIP1-2, ACTL8, lnc-TM2D3-2, GCN1L1, LOC101929337, FAM217A, XLOC_l2_013467, SNHG4, NUDT13, MCTS1, ATG4D, NPEPL1, YEATS2, SPEN, GGT6, lnc-RP11-770J1.4.1-1, MIAT, lnc-HMCN1-2, RETN, lnc-EXT1-2, LIN54, C17orf74, SLC12A2, TTC28, ADRA2A, NLGN2, lnc-FBXO25-4, LOC100506603, LOC399886, RCC2, KIAA0040, HOXB9, JMJD8, LINC01146, lnc-GIT1-1, VRTN, NHP2, KIF26A, HAUS5, lnc-SOD2-2, APOL3, lnc-POLR2L-1, lnc-SUPT3H-1, lnc-XRN2-3, RTP2, TERT, T'NFRSF12A, LOC645427, lnc-CTR9-5, LOC100507420, OPA3, XLOC_l2_014245, lnc-NR5A1-1, DMTN, SMPD4, TXLNB, SPRR1A, DISP1, LOC100130285, lnc-SLC17A9-1, lnc-SELV.1-1, XLOC_l2_000018, lnc-ROM1-2, lnc-RABEPK-1, lnc-RRP8-1, lnc-RP11-234B24.6.1-1, lnc-TMEM189-UBE2V1-2, LAMTOR2, BTK, lnc-BSND-1, C2orf71, lnc-C17orf63-2, lnc-CHGA-1, ABCD1, NCAN, C9orf106, AGAP2, EMC6, CD86, LOC100129175, lnc-PHYHD1-1, GIGYF2, RNF151, BAGE, ADRA2C, lnc-SLC43A1-1, ENTPD8, NYAP1, lnc-GLIPR1L1-1, CCNT2-AS1, NAVI, TBX21, GNAZ, DNM3OS, PRKAG2-AS1, lnc-MFSD9-4, lnc-RPGRIP1-2, ABCC6, LRFN3, lnc-COL1A1-2, lnc-PPAPDC3-2, CTU1, lnc-PABPC4-1, LOC100507165, LOC102724861, SPTSSB, lnc-C20orf96-4, DCDC5, lnc-CRYL1-1, LOC100506114, SHARPIN, ASMTL-AS1, HMOX1, SHARPIN, LOC101928207, TRAIP, lnc-ANTXRL-2, LINC01135, BZRAP1, LOC101926983, WDR6, C19orf81, NAMA, LIM2, FAM155A-IT1, lnc-RPP30-2, ZNF264, MALAT1, HTT-AS, ZDHHC22, SEPHS2, ABTB1, lnc-RP11-791J7.2.1-1, CCDC28B, LOC284263, lnc-PPIC-2, CDC42BPB, C9orf173-AS1, C9orf139, FGD5, SP6, RCOR2, lnc-AC016251.1-2, lnc-PRKAA2-1, RANBP3, lnc-SH2B3-1, XLOC _l2_011901, CACNA1H, LINC01314, lnc-VAPB-1, ARHGAP23, ATG9A, GM2A, XLOC_l2_013547, lnc-SLC25A26-1, TMEM240, CLSTN3, ATG16L2, MMP11, TSSK3, lnc-SOX6-1, EDEM3, LOC101927210, C14orf169, EBLN2, lnc-XRN1-1, lnc-FTSJD2-1, MDFI, LOC102546226, SLC5A10, SENP7, lnc-C17orf97-4, ARNTL, lnc-RTL1-1, ZNF713, XLOC_l2_005871, QSOX1, SP2-AS1, ARSA, PHF7, GS1-24F4.2, LRIT1, PLLP, SLC4A11, C11orf95, FAM110B, IER5, LINC01508, lnc-GTPBP8-1, LOC400958, WFDC21P, lnc-CPXM2-2, P2RX6, SLC22A7, lnc-PPP2R2C-1, lnc-GATA5-2, MARVELD1, lnc-TRIM41-3, PRSS8, SEMA6C, SLC48A1, RPL28, BGN, BZW1, lnc-C6orf221-2, SLC20A2, HYALP1, SNAR-G1,

SEC22A, TIMM8B, ATAD1, FNDC7, B3GNT8, STARD7-AS1, TMEM100, LARP6, HELZ2, TMX2, lnc-EPHB6-1, PSMC2, lnc-LINC00273-1, TNFRSF25, BCL3, LOC101929450, LOC102724416, FLII, WAC, PPP6R1, LOC100507377, LRRFIP2, CCNT2-AS1, lnc-SCAMP5-1, BRD1, lnc-ELAVL4-3, CHRDL1, lnc-FUT8-1, lnc-DNAJC7-1, LINC01264, LMO2, LINC01197, LOC101927533, REP15, C1orf226, lnc-STEAP1B-1, SNORD3B-1, lnc-TMEM135-2, lnc-FAM160A1-1, GOLGA2P5, TMEM132C, GPX3, lnc-DCAF4L2-2, XLOC_l2_014217, PPT1, RPA4, KCTD5, lnc-UQCRFS1-9, MTUS2-AS1, CHKA, TBC1D31, SNORD38A, lnc-RP11-322L20.1.1-7, RAD51, MEIOB, lnc-ADAMTS14-1, PCDHA13, and NOS2, and
iii) comprises at least one cytokine selected from the group consisting of IL-6, IL-8, MCP-3, MCP-1, IP-10, IL-1$\beta$, and RANTES.

**2.** The extracellular vesicle according to claim 1, wherein the extracellular vesicle comprises at least one selected from the group consisting of zymosan, polyinosinic-polycytidylic acid (Poly I:C), and monophosphoryl lipid A (MPLA).

**3.** The extracellular vesicle according to claim 1, wherein the human-derived cell includes a cell, a cell line, or a stem cell derived from human tissue.

**4.** The extracellular vesicle according to claim 3, wherein the stem cell is a mesenchymal stem cell, a hematopoietic stem cell, a neural stem cell, an embryonic stem cell, or an induced pluripotent stem cell.

**5.** The extracellular vesicle according to claim 4, wherein the mesenchymal stem cell is derived from umbilical cord, umbilical cord blood, Wharton's jelly, bone marrow, fat, muscle, nerve, skin, amniotic membrane, tooth, hair root cell, or placenta, or is differentiated from an induced pluripotent stem cell.

**6.** The extracellular vesicle according to claim 1, wherein the extracellular vesicle

i) is positive for at least one marker selected from the group consisting of CD9, CD63, CD81, and TSG101, and
ii) is negative for at least one marker selected from the group consisting of GM130 and Calnexin.

**7.** An extracellular vesicle isolated from a human-derived cell treated with at least one selected from the group consisting of zymosan, Poly I:C, and monophosphoryl lipid A (MPLA).

**8.** The extracellular vesicle according to claim 7, wherein the human-derived cell includes a cell, a cell line, or a stem cell derived from human tissue.

**9.** The extracellular vesicle according to claim 8, wherein the stem cell is a mesenchymal stem cell, a hematopoietic stem cell, a neural stem cell, an embryonic stem cell, or an induced pluripotent stem cell.

**10.** The extracellular vesicle according to claim 9, wherein the mesenchymal stem cell is derived from umbilical cord, umbilical cord blood, Wharton's jelly, bone marrow, fat, muscle, nerve, skin, amniotic membrane, tooth, hair root cell, or placenta, or is differentiated from an induced pluripotent stem cell.

**11.** A pharmaceutical composition for preventing or treating cancer, comprising the extracellular vesicle according to any one of claims 1 to 10 as an active ingredient.

**12.** The pharmaceutical composition for preventing or treating cancer according to claim 11, wherein the cancer is any one selected from the group consisting of gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, and lymphoma.

**13.** A pharmaceutical composition for combination administration for preventing or treating cancer, comprising the extracellular vesicle according to any one of claims 1 to 12 and an immune anticancer agent as active ingredients.

**14.** The pharmaceutical composition for combination administration for preventing or treating cancer according to claim 13, wherein the immune anticancer agent is any one selected from the group consisting of an immune checkpoint inhibitor, a chimeric antigen receptor-T cell (CAR-T), a T cell receptor modified T cell (TCR-T), a tumor infiltrating lymphocyte (TIL), a bispecific T-cell engager (BiTE), a tumor vaccine, and an oncolytic virus.

**15.** The pharmaceutical composition for combination administration for preventing or treating cancer according to claim

14, wherein the immune checkpoint inhibitor is any one selected from the group consisting of an anti-PD-1 antibody, an anti-PD-L1 antibody, an anti-PD-L2 antibody, an anti-CTLA-4 antibody, an anti-B7-H4 antibody, an anti-HVEM antibody, an anti-TIM3 antibody, an anti-GAL9 antibody, an anti-LAG3 antibody, an anti-VISTA antibody, an anti-KIR antibody, an anti-BTLA antibody, and an anti-TIGIT antibody.

**16.** A method for preparing extracellular vesicles, comprising:

a) culturing human-derived cells in a serum-free cell culture medium supplemented with Poly I:C and monophosphoryl lipid A (MPLA);
b) obtaining a culture solution during the culturing process; and
c) filtering the obtained culture solution to remove particles larger than 200 nm and then isolating the extracellular vesicles.

**17.** The method for preparing extracellular vesicles according to claim 15, wherein the serum-free cell culture medium further comprises zymosan.

**18.** A method for preventing or treating cancer, comprising administering the extracellular vesicle according to claim 1 or 7 to a subject.

**19.** Use of the extracellular vesicle according to claim 1 or 7 for the prevention or treatment of cancer.

[Figure 1]

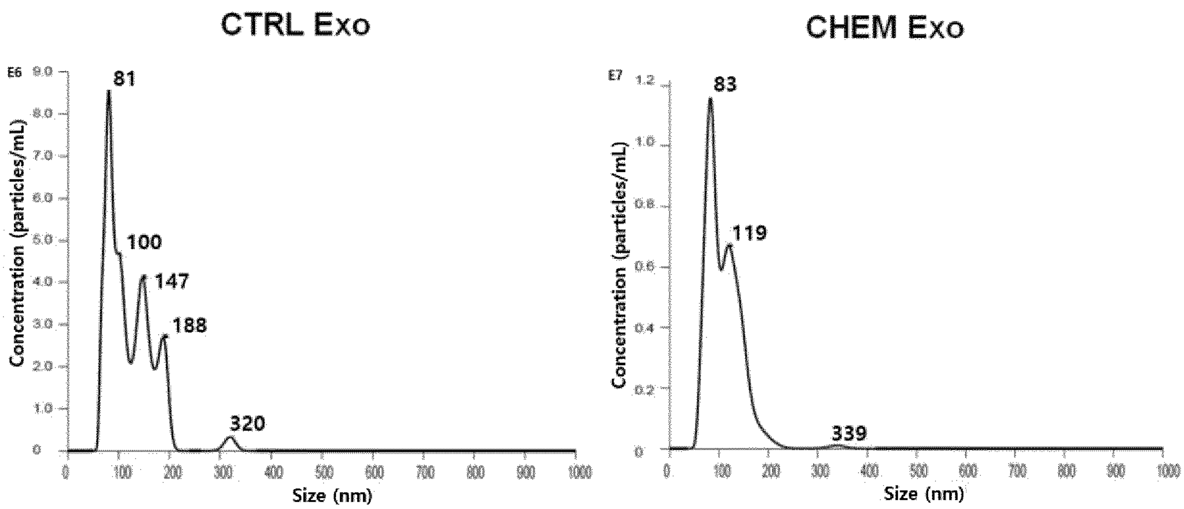


[Figure 2]

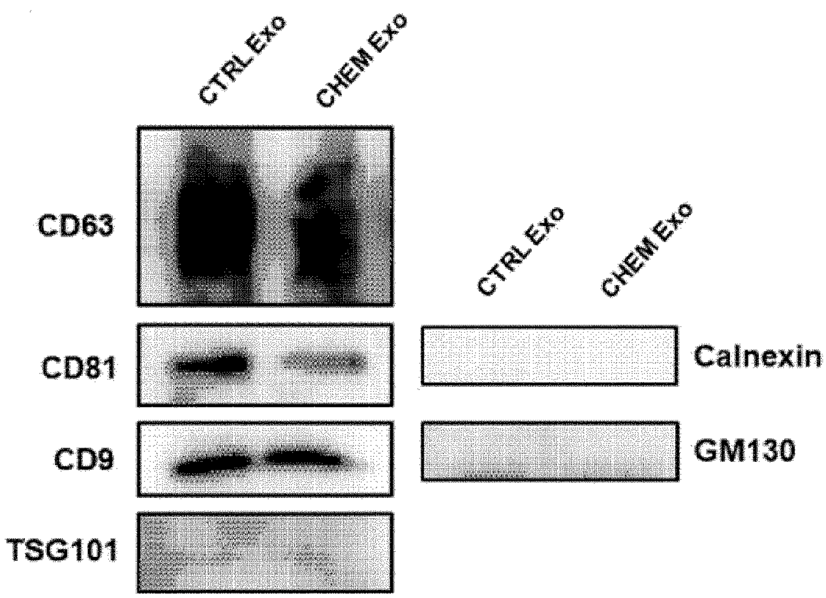

[Figure 3]

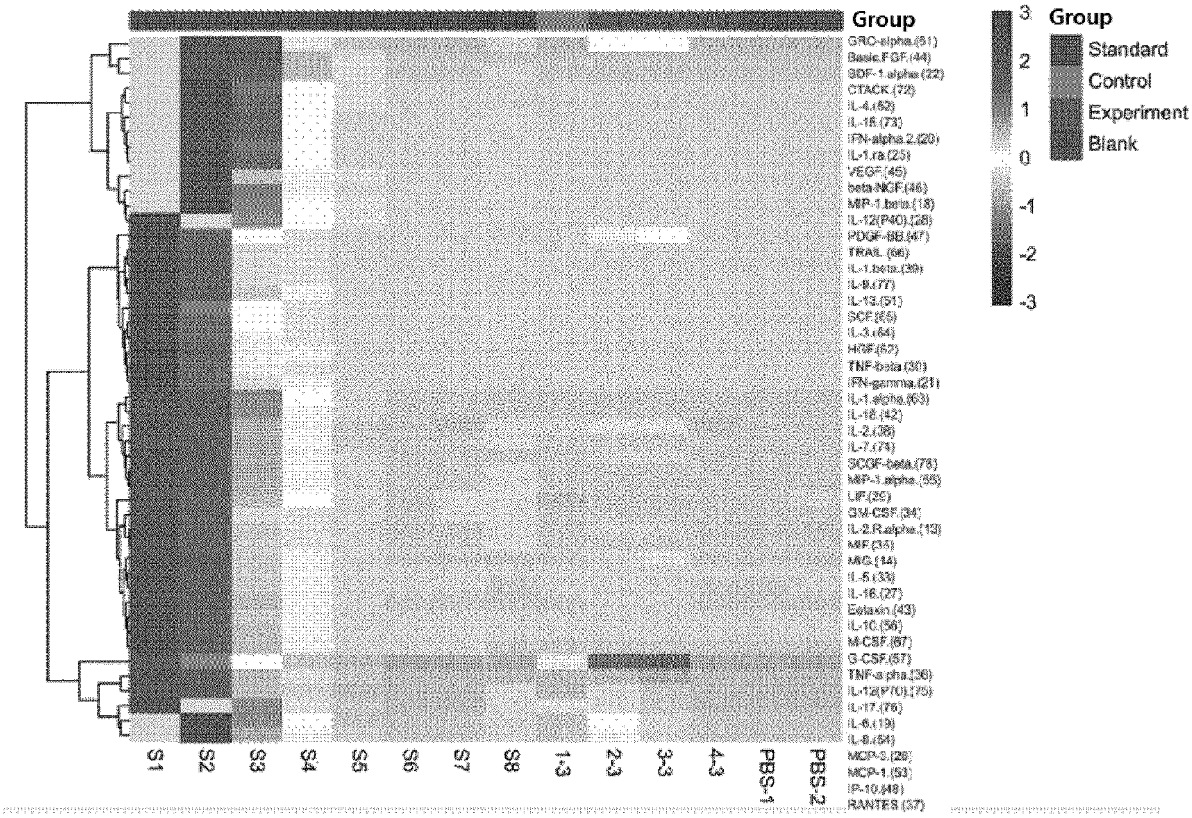
Group
GRO-alpha.(51)
Basic.FGF.(44)
SDF-1.alpha.(22)
CTACK.(72)
IL-4.(52)
IL-15.(73)
IFN-alpha.2.(20)
IL-1.ra.(25)
VEGF.(45)
beta-NGF.(46)
MIP-1.beta.(18)
IL-12(P40).(28)
PDGF-BB.(47)
TRAIL.(66)
IL-1.beta.(39)
IL-9.(77)
IL-13.(51)
SCF.(65)
IL-3.(64)
HGF.(62)
TNF-beta.(30)
IFN-gamma.(21)
IL-1.alpha.(63)
IL-18.(42)
IL-2.(38)
IL-7.(74)
SCGF-beta.(78)
MIP-1.alpha.(55)
LIF.(29)
GM-CSF.(34)
IL-2.R.alpha.(13)
MIF.(35)
MIG.(14)
IL-5.(33)
IL-16.(27)
Eotaxin.(43)
IL-10.(56)
M-CSF.(67)
G-CSF.(57)
TNF-alpha.(36)
IL-12(P70).(75)
IL-17.(76)
IL-6.(19)
IL-8.(54)
MCP-3.(26)
MCP-1.(53)
IP-10.(48)
RANTES.(37)
S1
S2
S3
S4
S5
S6
S7
S8
1-3
2-3
3-3
4-3
PBS-1
PBS-2
3
2
1
0
-1
-2
-3
Group
Standard
Control
Experiment
Blank

[Figure 4]

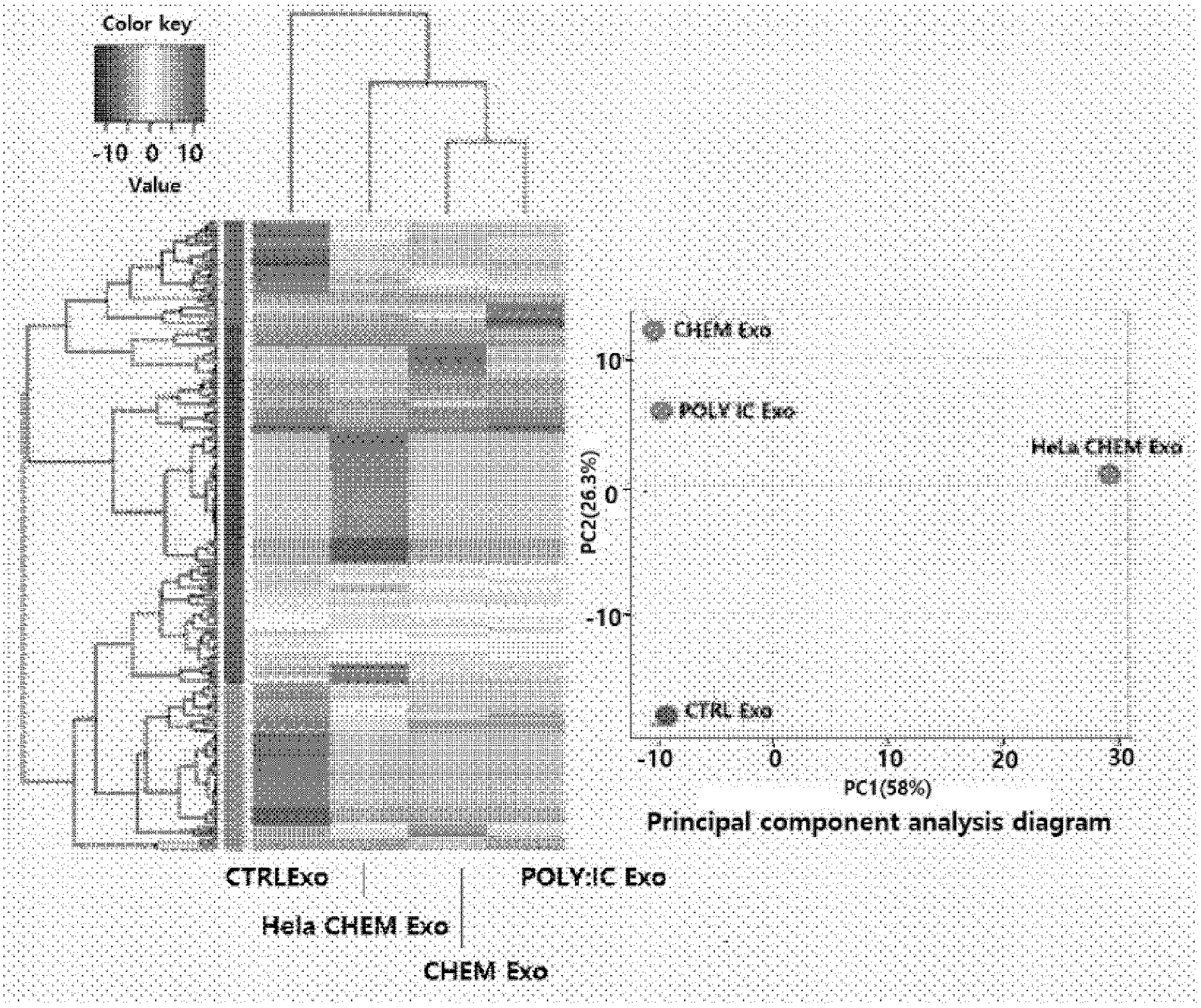


[Figure 5]

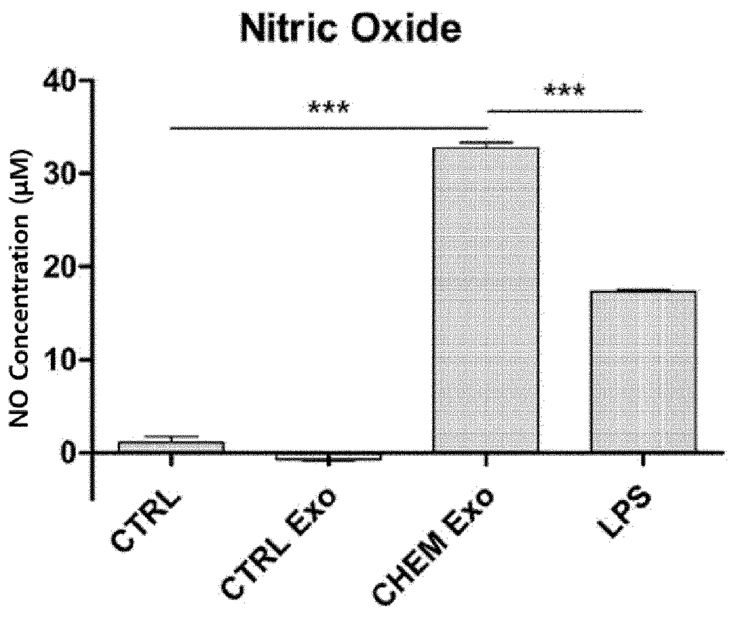

[Figure 6]

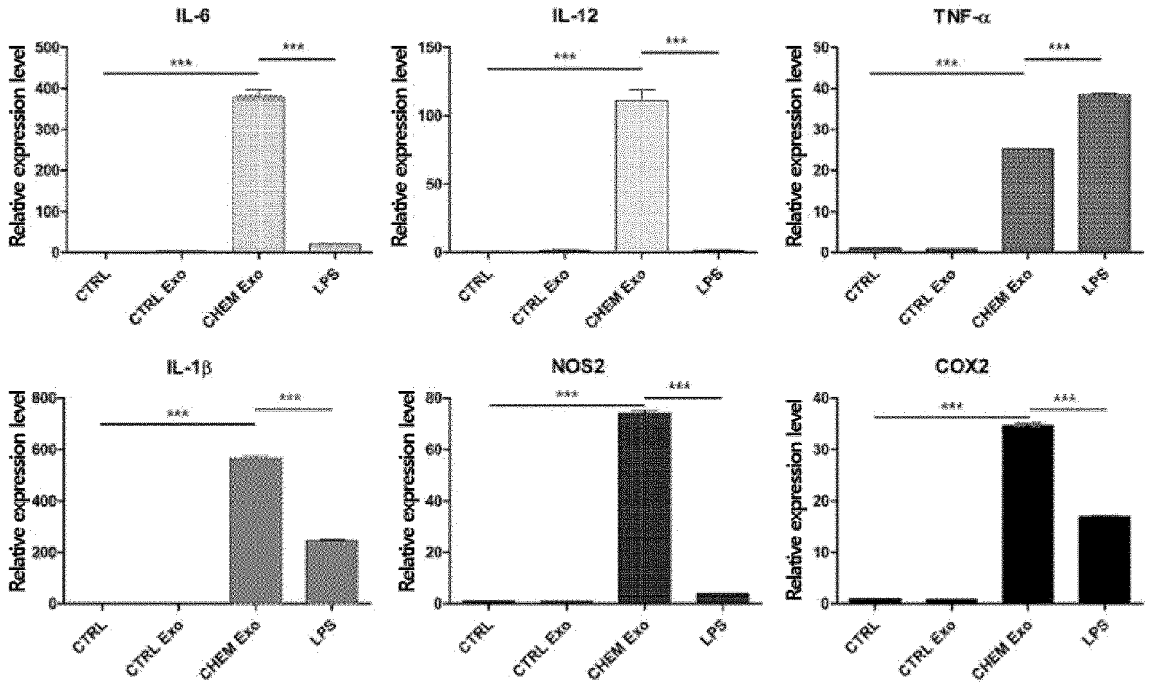


[Figure 7]

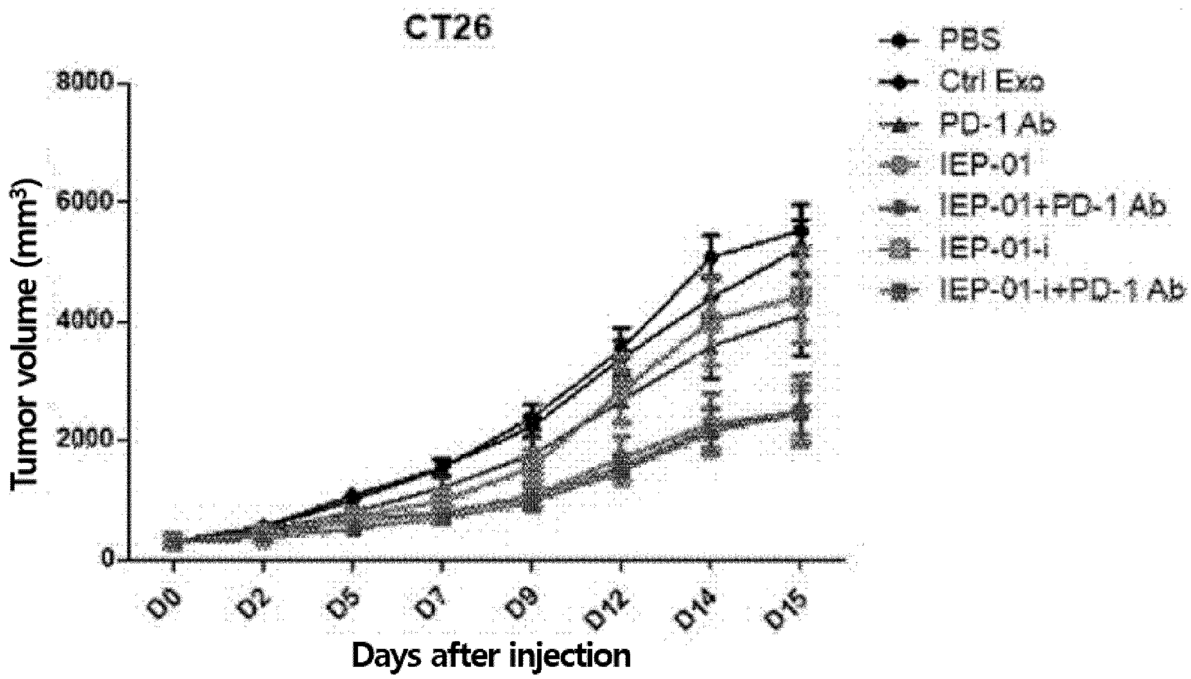

[Figure 8]

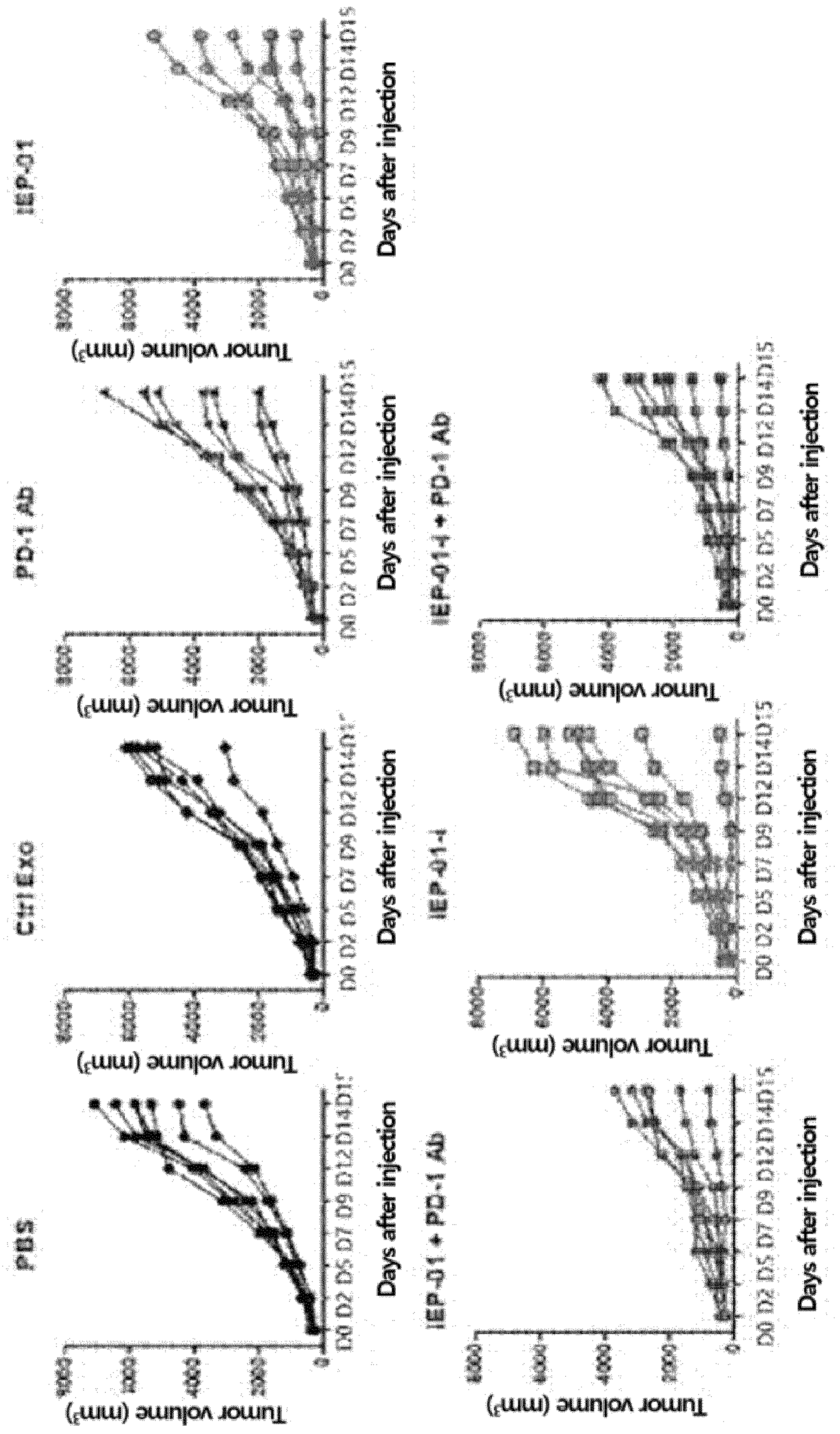
PBS
PD-1 Ab
IEP-01
IEP-01 + PD-1 Ab
IEP-01-4
IEP-01-4 + PD-1 Ab
Tumor volume (mm3)
Days after injection
D0 D2 D5 D7 D9 D12 D14 D15

[Figure 9]

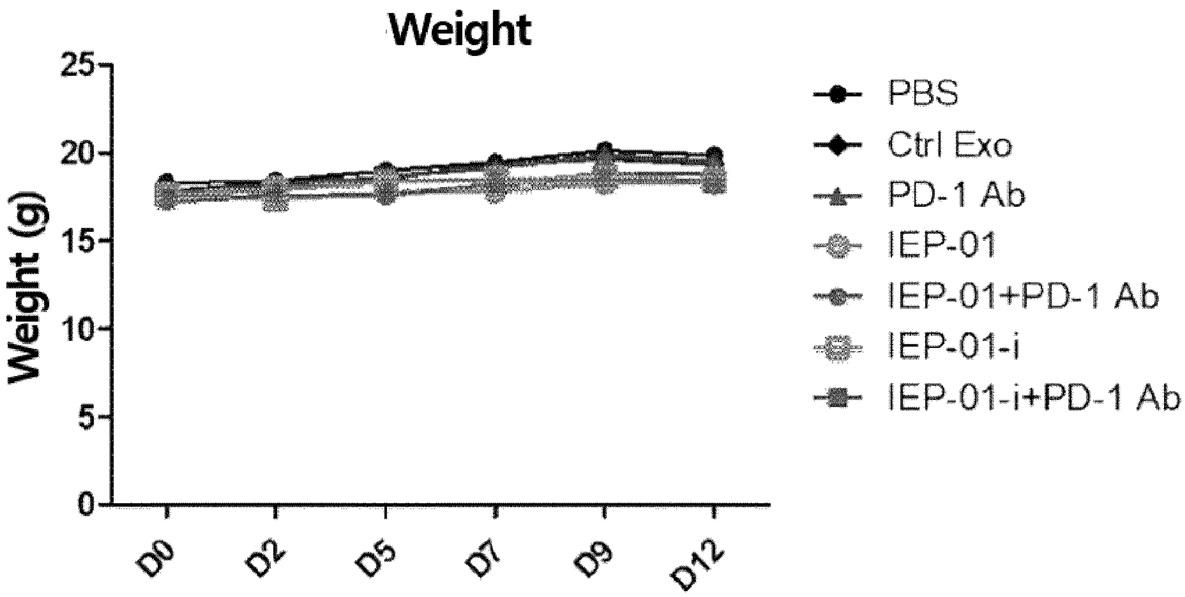


[Figure 10]

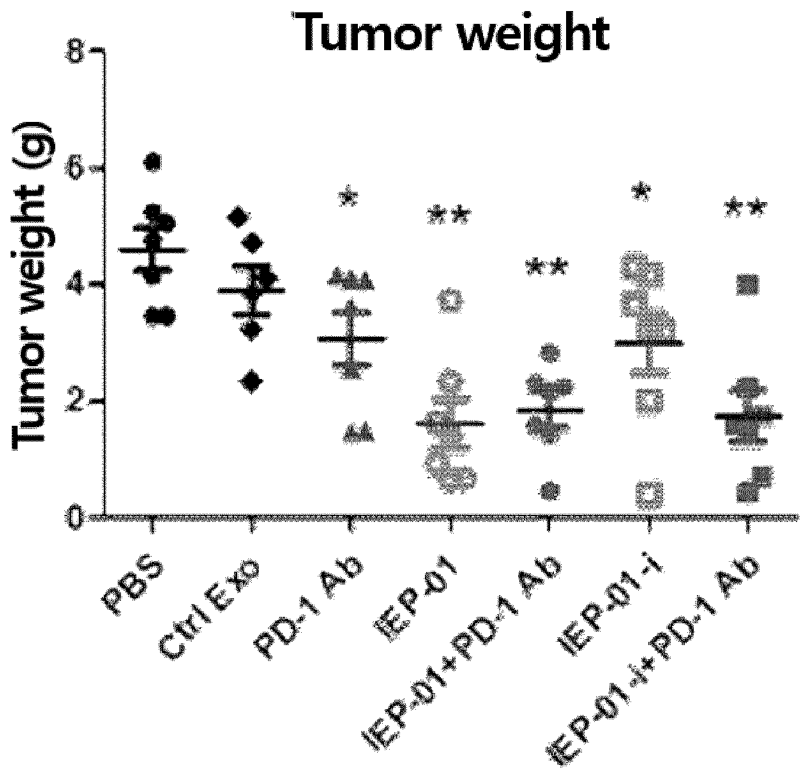

[Figure 11]

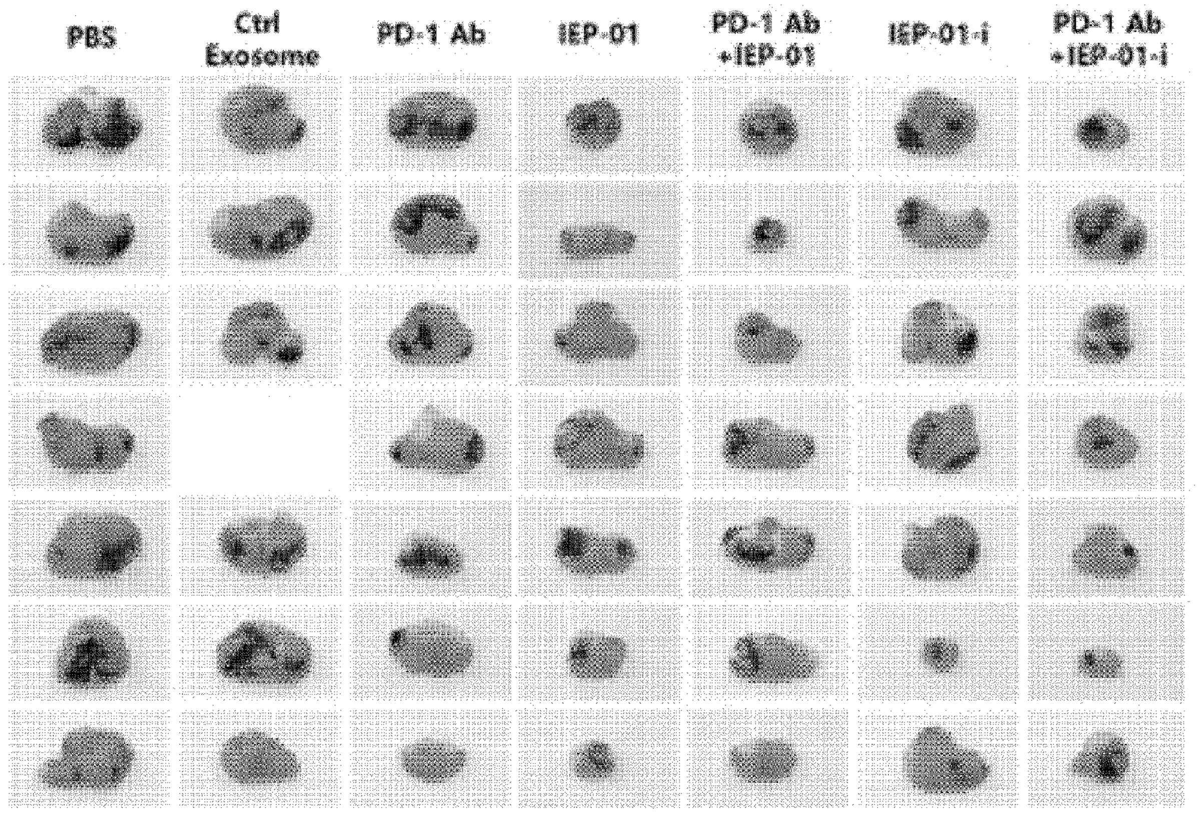


[Figure 12]

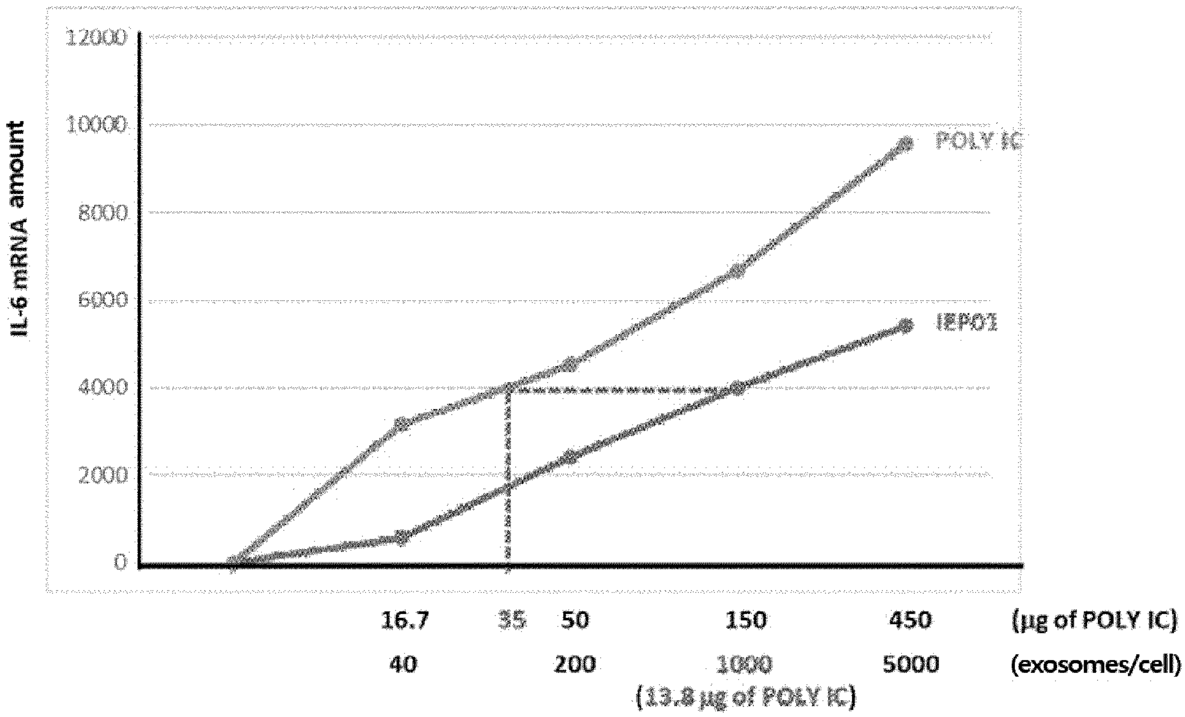

[Figure 13a]

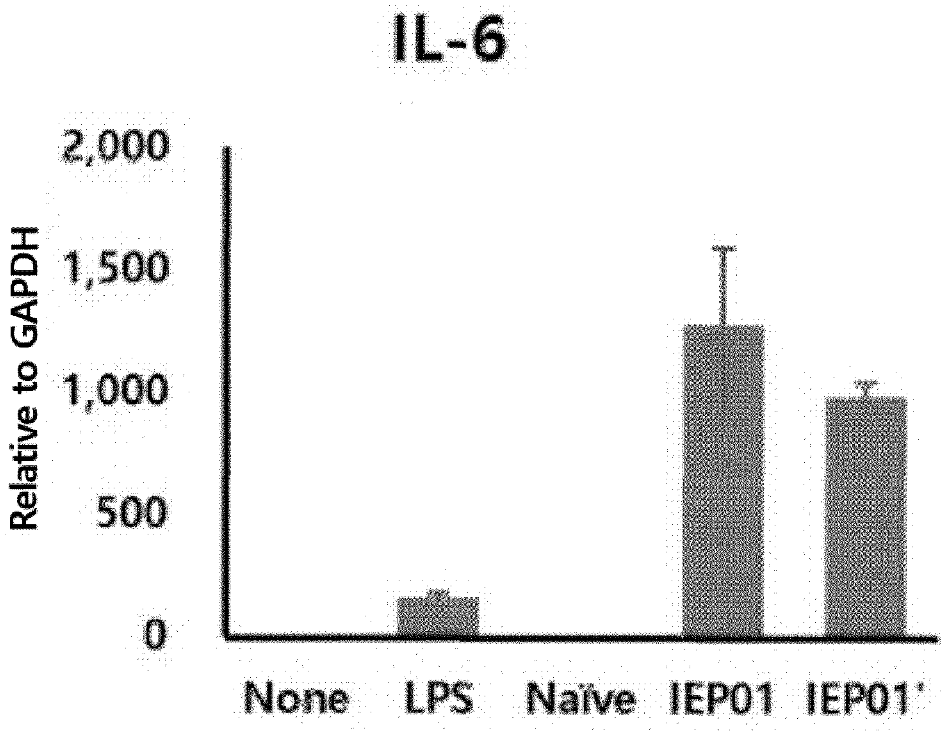


[Figure 13b]

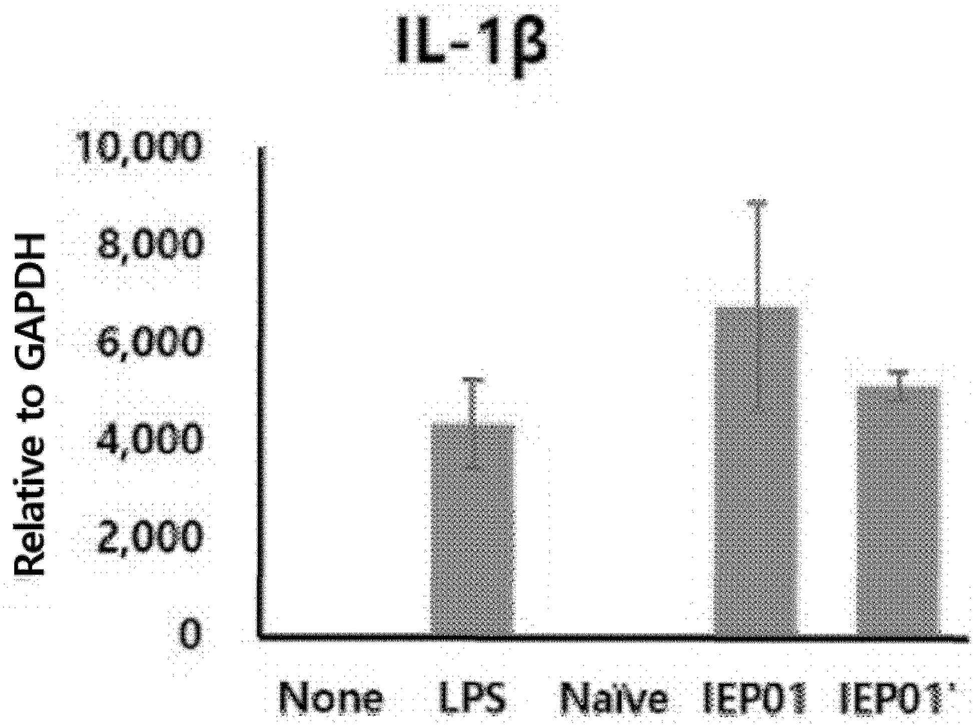


[Figure 13c]

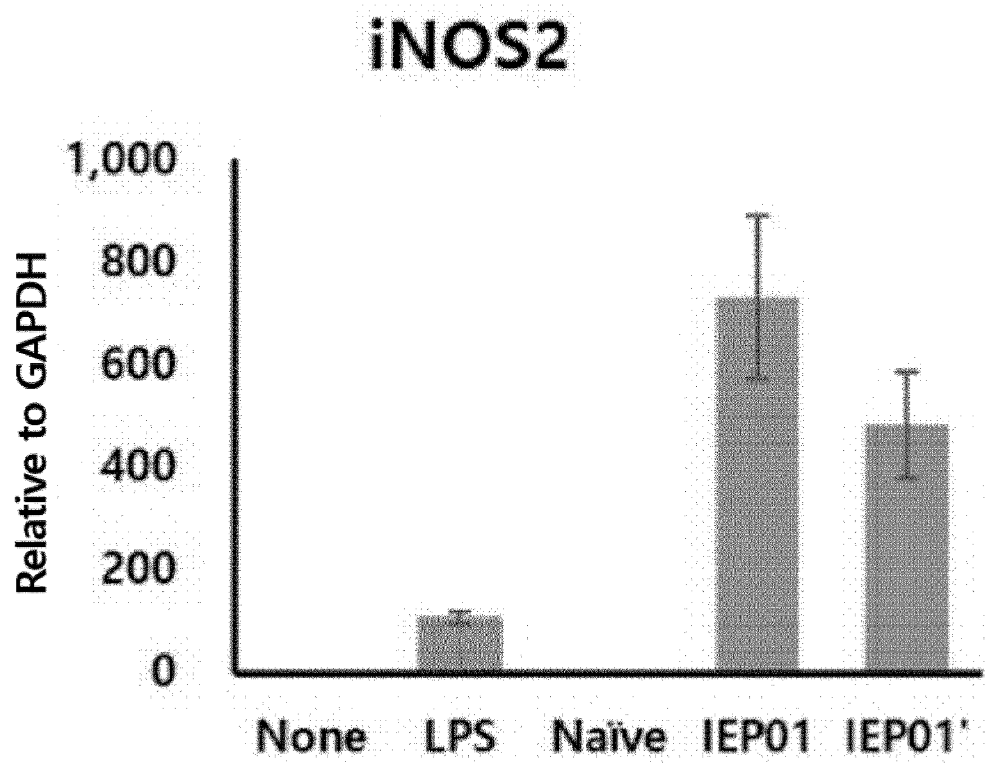
iNOS2
Relative to GAPDH
1,000
800
600
400
200
0
None
LPS
Naïve
IEP01
IEP01'

[Figure 13d]

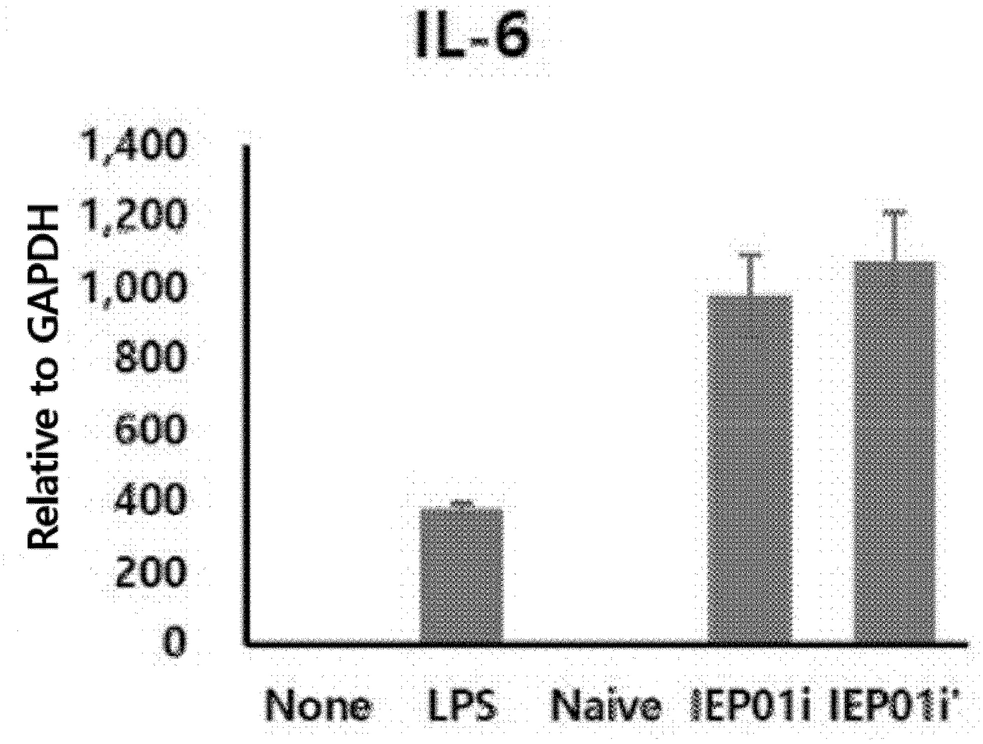
IL-6
Relative to GAPDH
1,400
1,200
1,000
800
600
400
200
0
None
LPS
Naïve
IEP01i
IEP01i'

[Figure 13e]

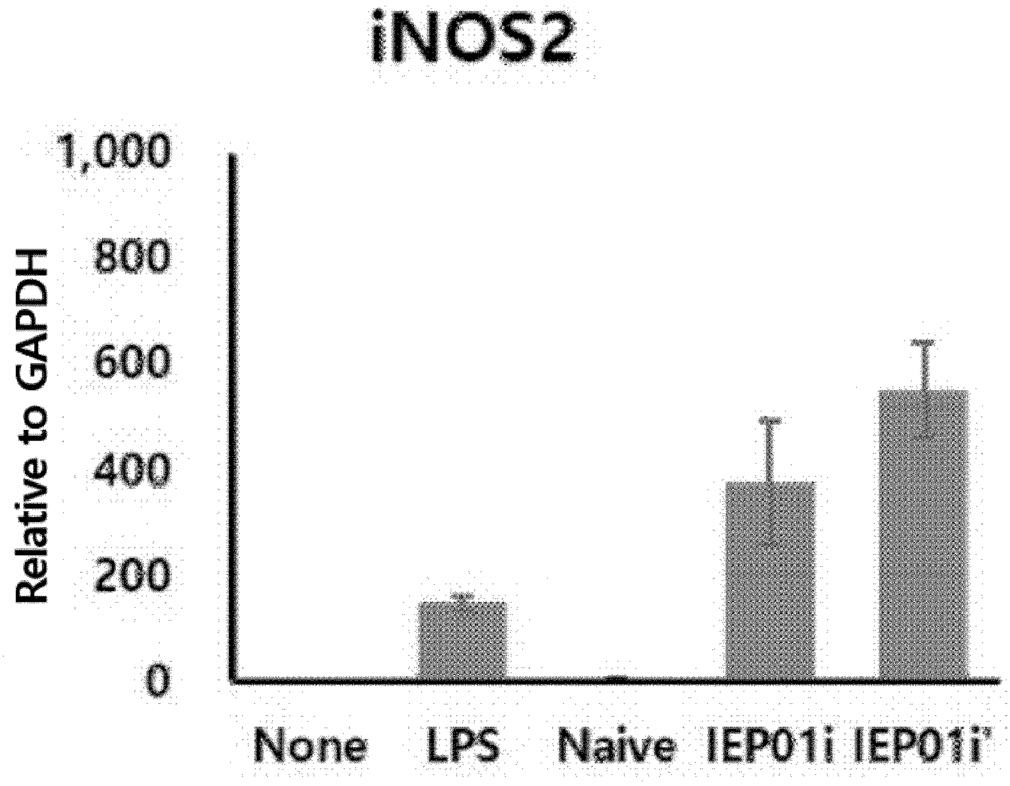


[Figure 13f]

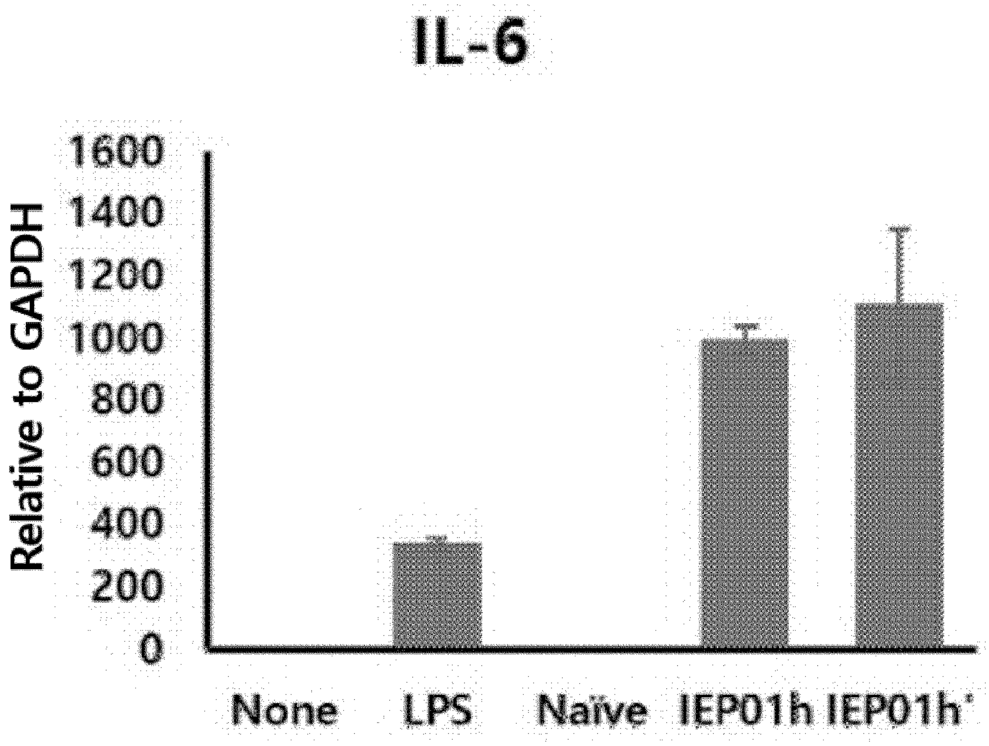

[Figure 13g]

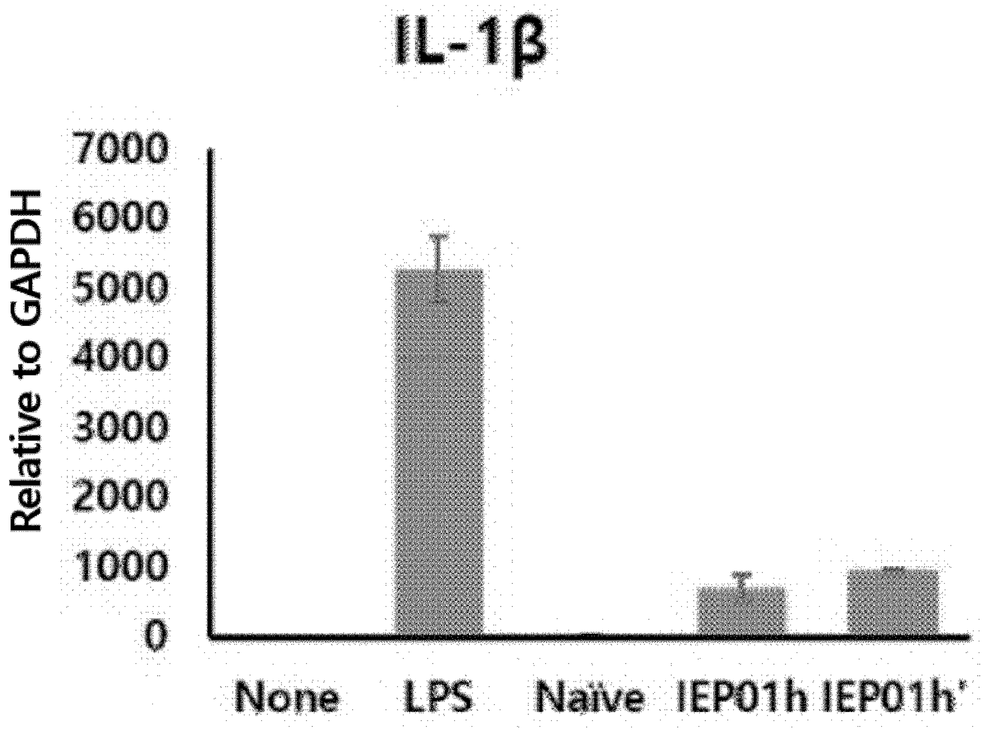
IL-1β
Relative to GAPDH
7000
6000
5000
4000
3000
2000
1000
0
None
LPS
Naïve
IEP01h
IEP01h'

[Figure 14a]

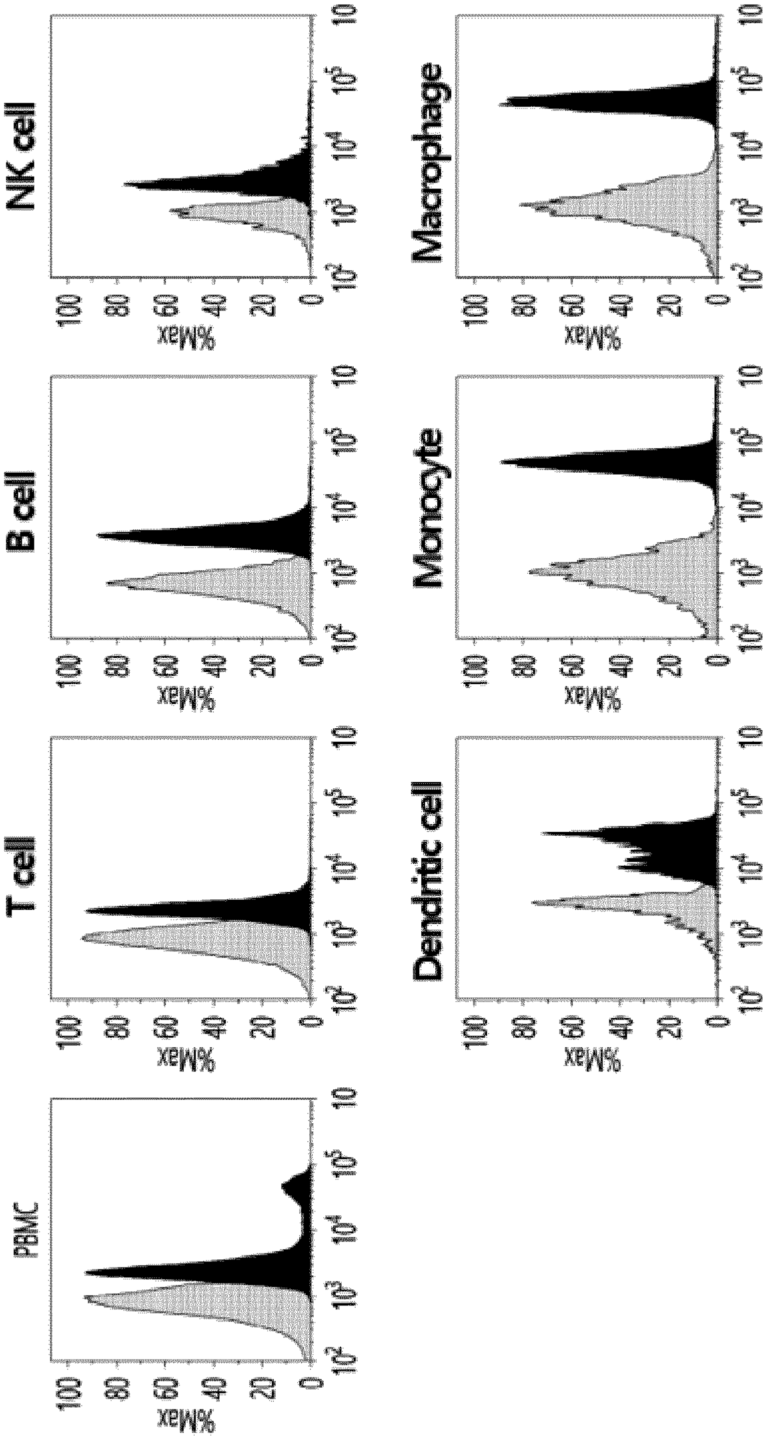
NK cell
B cell
T cell
PBMC
Macrophage
Monocyte
Dendritic cell
%Max

[Figure 14b]

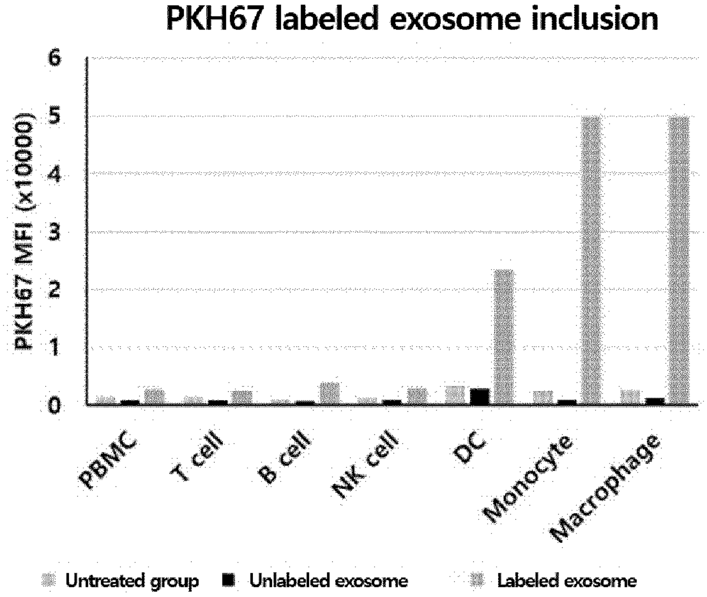


[Figure 14c]

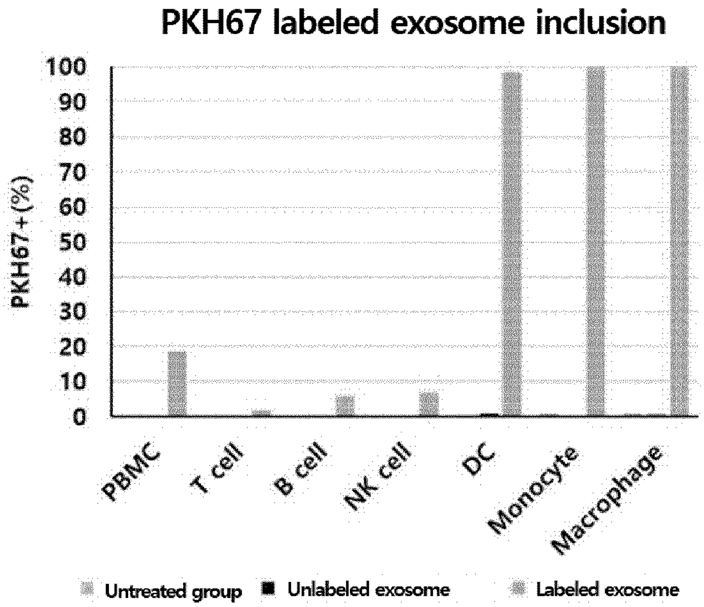


[Figure 15a]

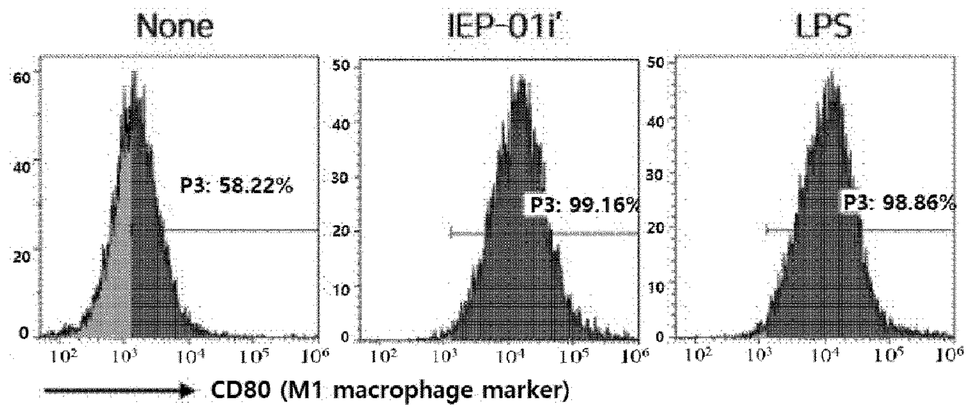

[Figure 15b]

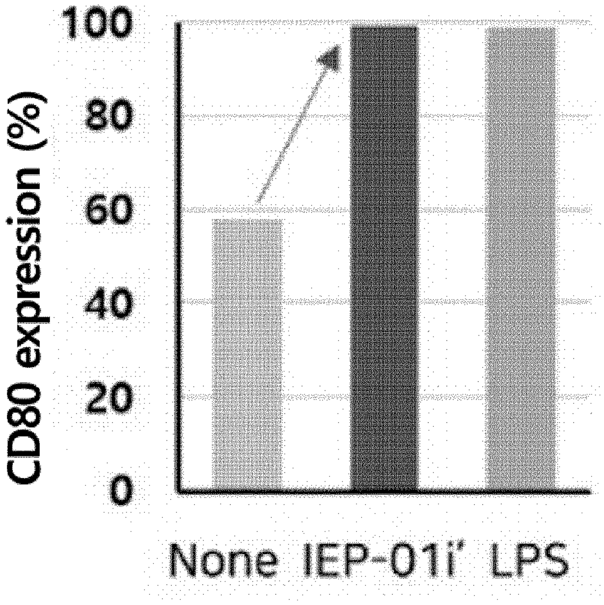

[Figure 15c]

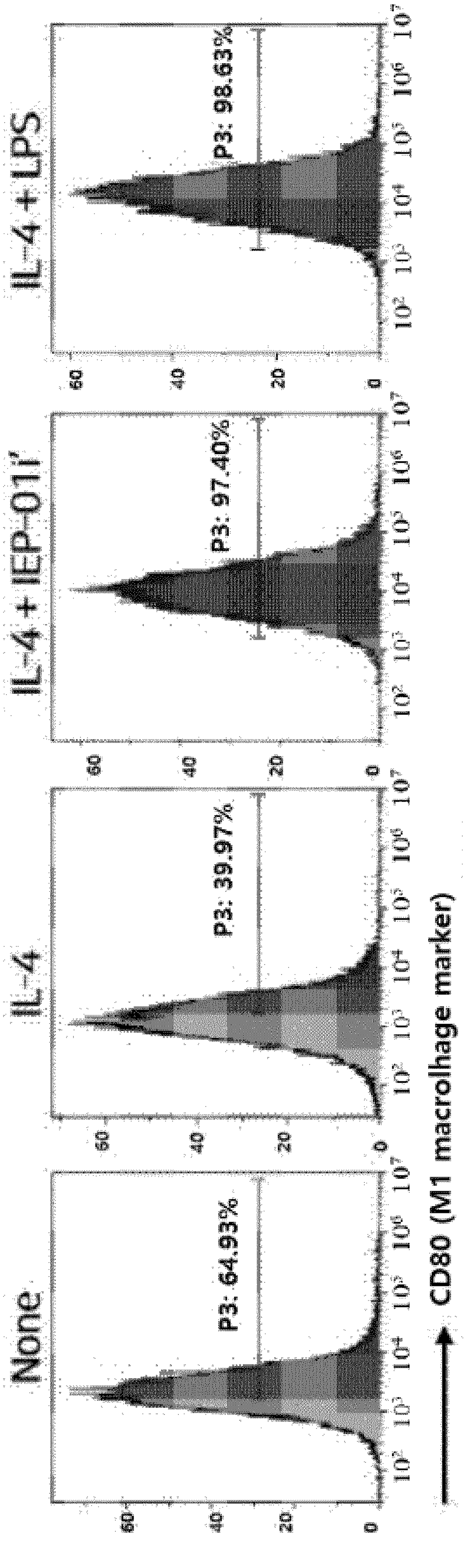
None
P3: 64.93%
IL-4
P3: 39.97%
IL-4 + IEP-01i'
P3: 97.40%
IL-4 + LPS
P3: 98.63%
CD80 (M1 macrolhage marker)

[Figure 15d]

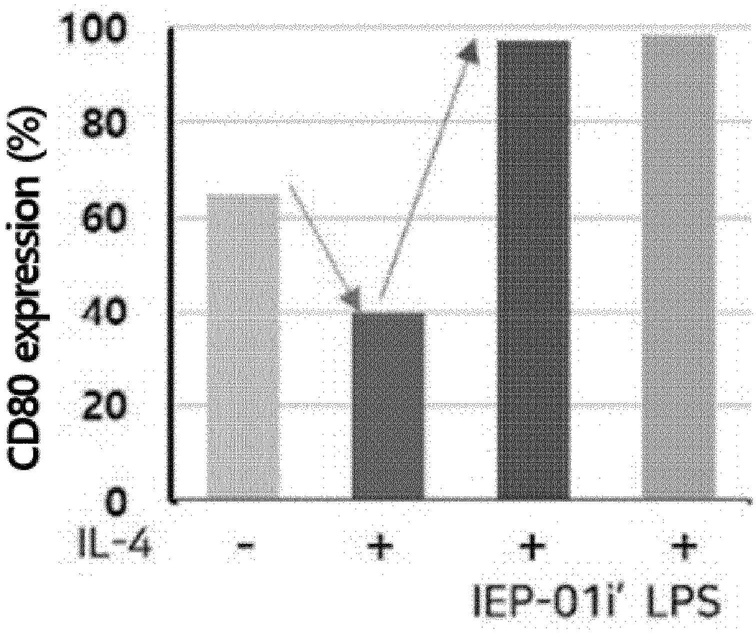

[Figure 16a]

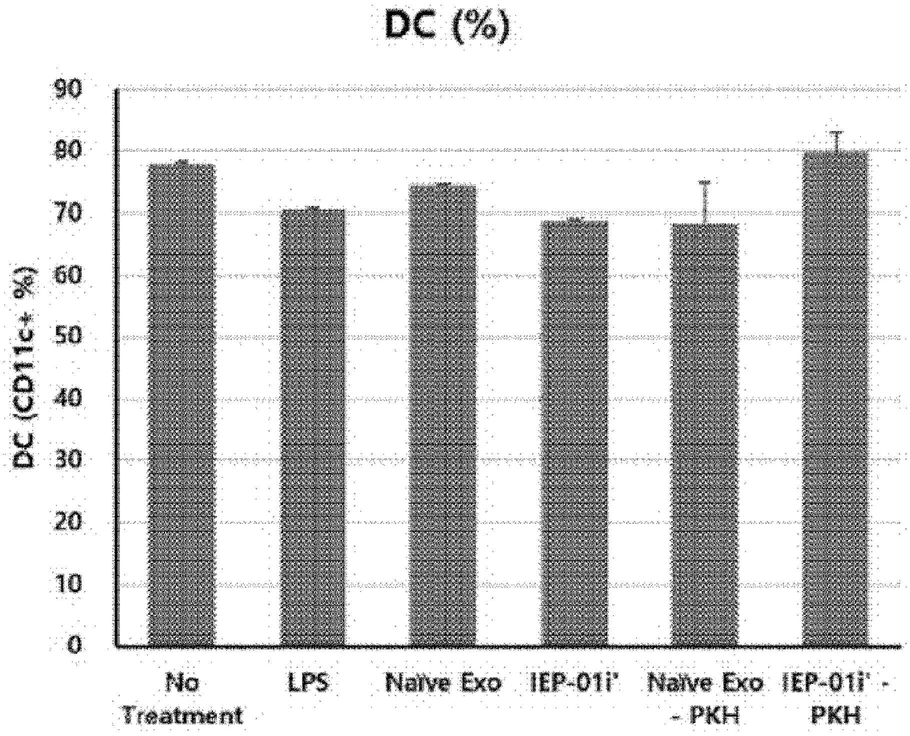


[Figure 16b]

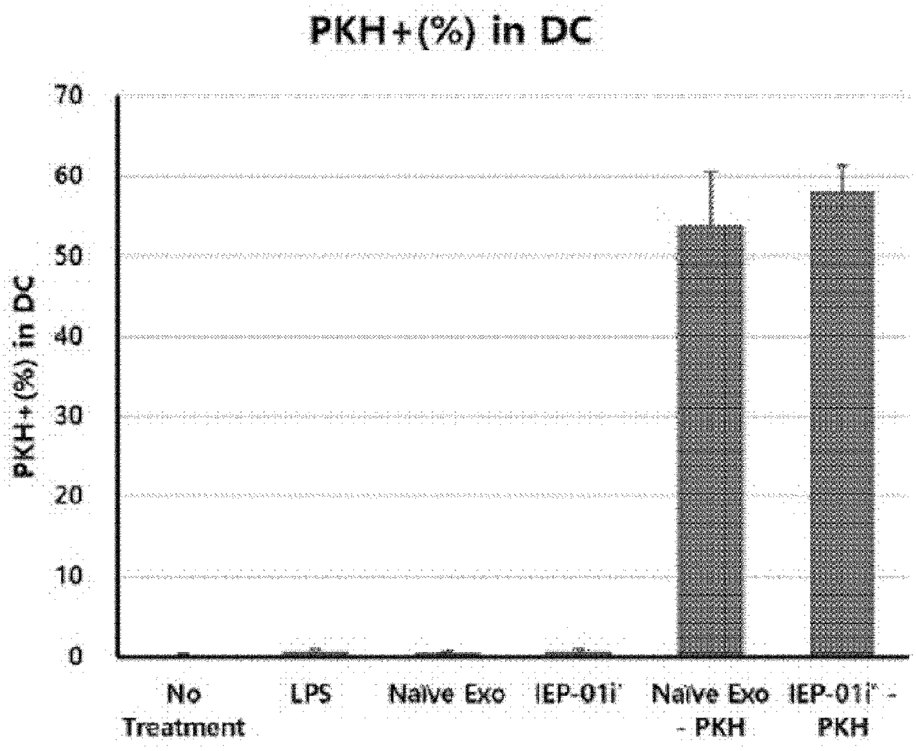

[Figure 16c]

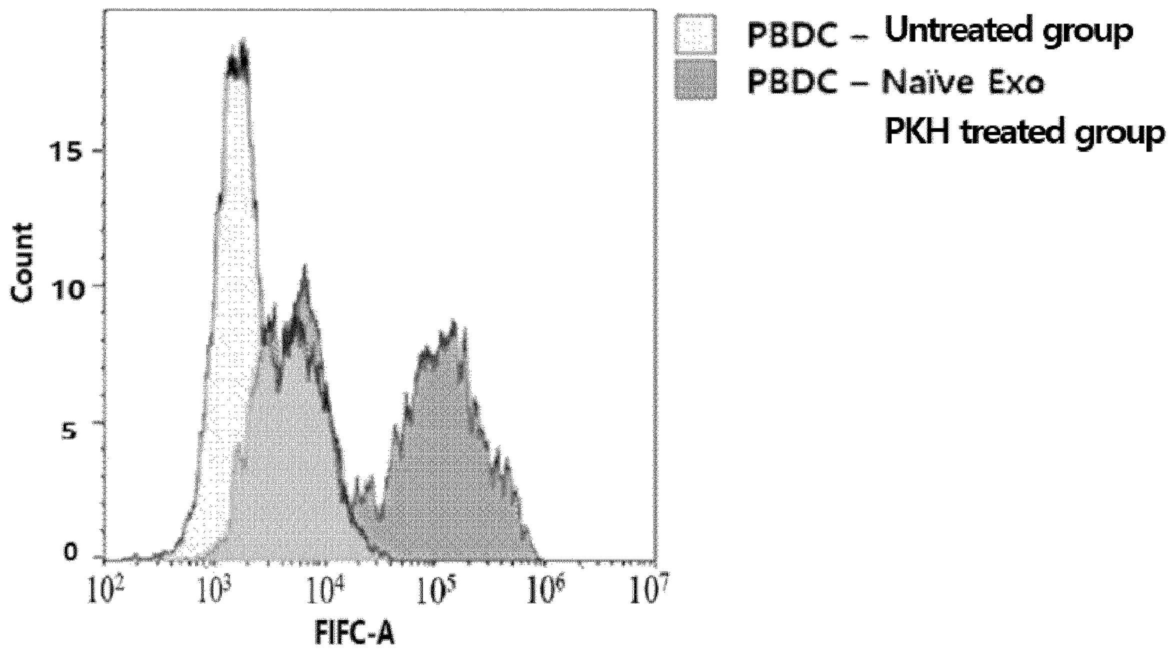


[Figure 16d]

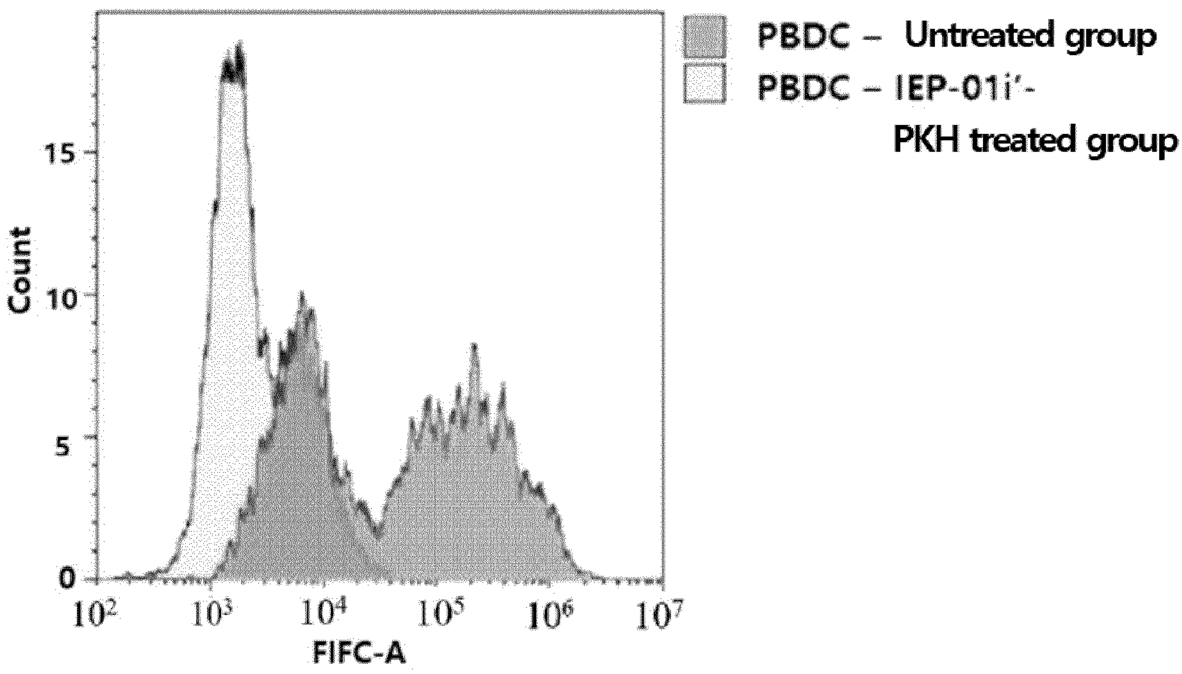

[Figure 16e]

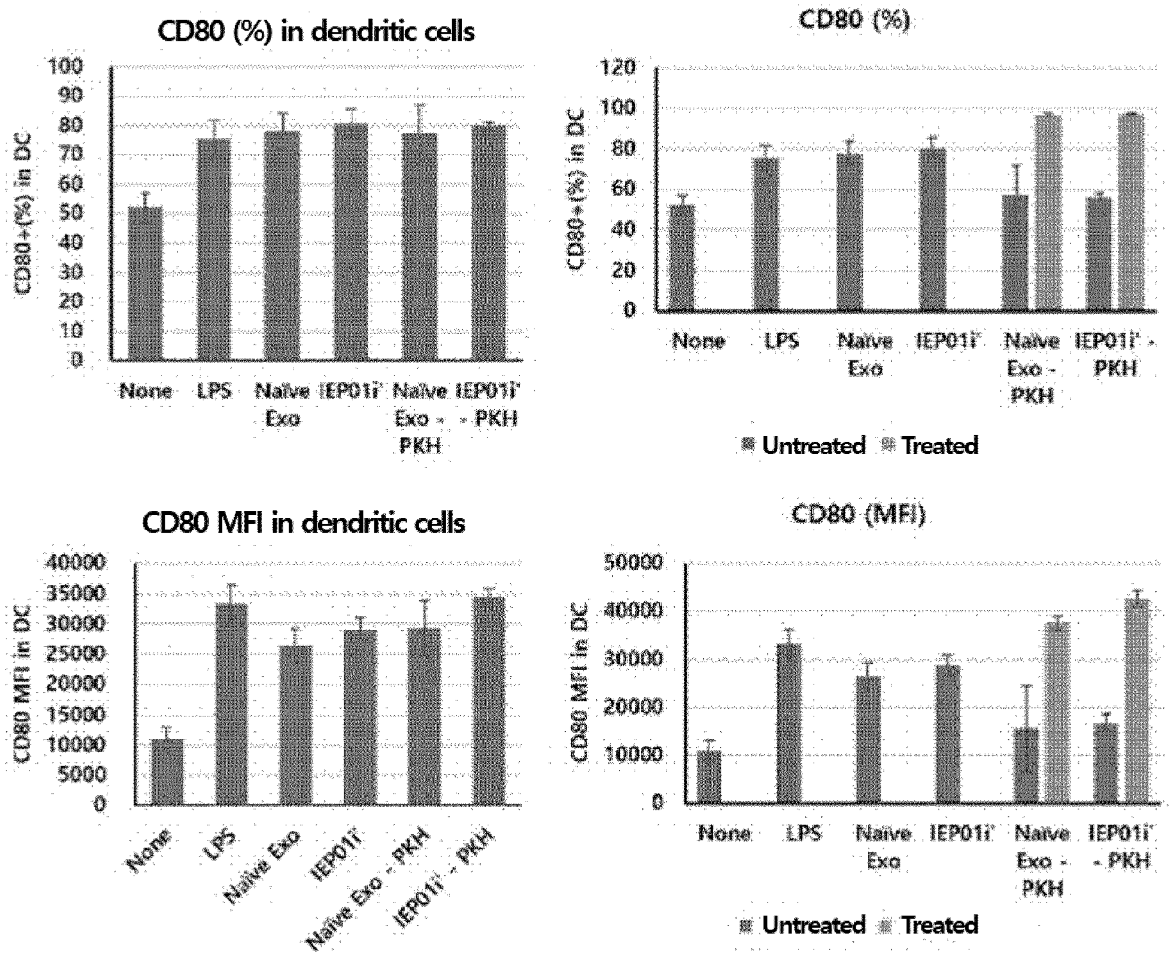
CD80 (%) in dendritic cells
CD80+(%) in DC
100
90
80
70
60
50
40
30
20
10
0
None
LPS
Naïve Exo
IEP01i'
Naïve Exo - PKH
IEP01i' - PKH
CD80 (%)
CD80+(%) in DC
120
100
80
60
40
20
0
None
LPS
Naïve Exo
IEP01i'
Naïve Exo - PKH
IEP01i' - PKH
Untreated
Treated
CD80 MFI in dendritic cells
CD80 MFI in DC
40000
35000
30000
25000
20000
15000
10000
5000
0
None
LPS
Naïve Exo
IEP01i'
Naïve Exo - PKH
IEP01i' - PKH
CD80 (MFI)
CD80 MFI in DC
50000
40000
30000
20000
10000
0
None
LPS
Naïve Exo
IEP01i'
Naïve Exo - PKH
IEP01i' - PKH
Untreated
Treated

**[Figure 16f]**

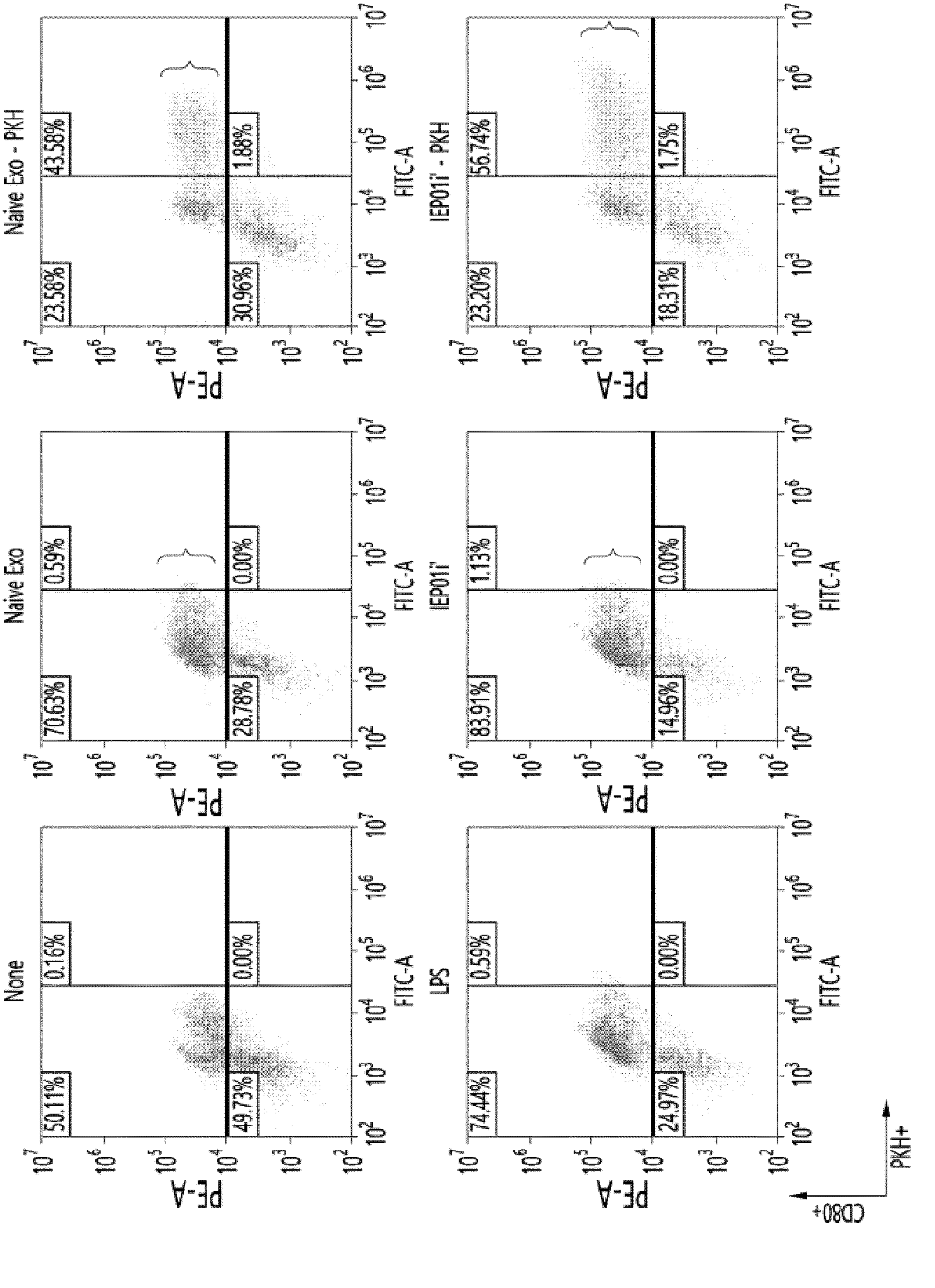
None
50.11%
0.16%
49.73%
0.00%
Naive Exo
70.63%
0.59%
28.78%
0.00%
Naive Exo - PKH
23.58%
43.58%
30.96%
1.88%
LPS
74.44%
0.59%
24.97%
0.00%
IEP01i'
83.91%
1.13%
14.96%
0.00%
IEP01i' - PKH
23.20%
56.74%
18.31%
1.75%
PE-A
FITC-A
CD80+
PKH+

**[Figure 16g]**

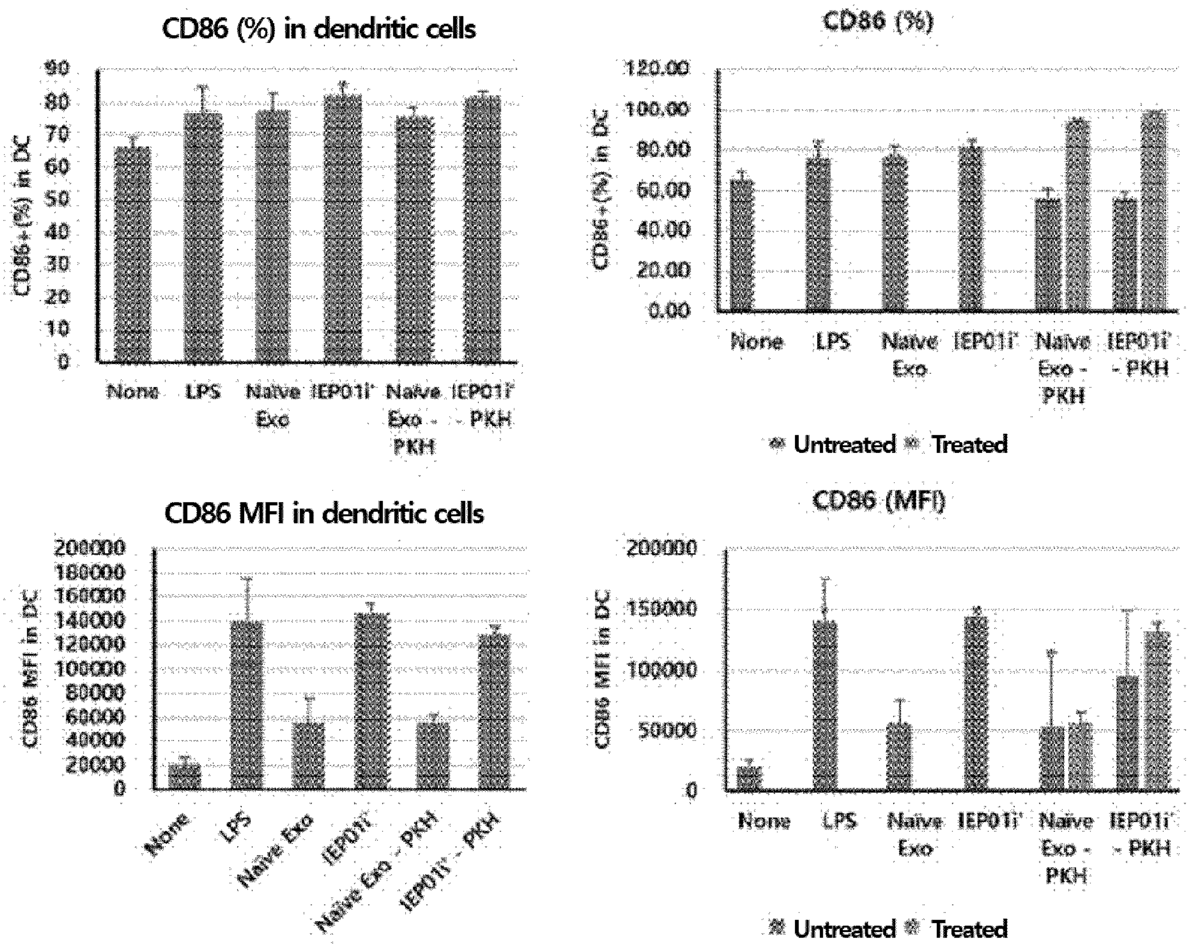

CD86 (%) in dendritic cells
CD86+(%) in DC
None
LPS
Naive Exo
IEP01i'
Naive Exo - PKH
IEP01i' - PKH
CD86 (%)
CD86+(%) in DC
Untreated
Treated
CD86 MFI in dendritic cells
CD86 MFI in DC
CD86 (MFI)
CD86 MFI in DC
Untreated
Treated

**[Figure 16h]**

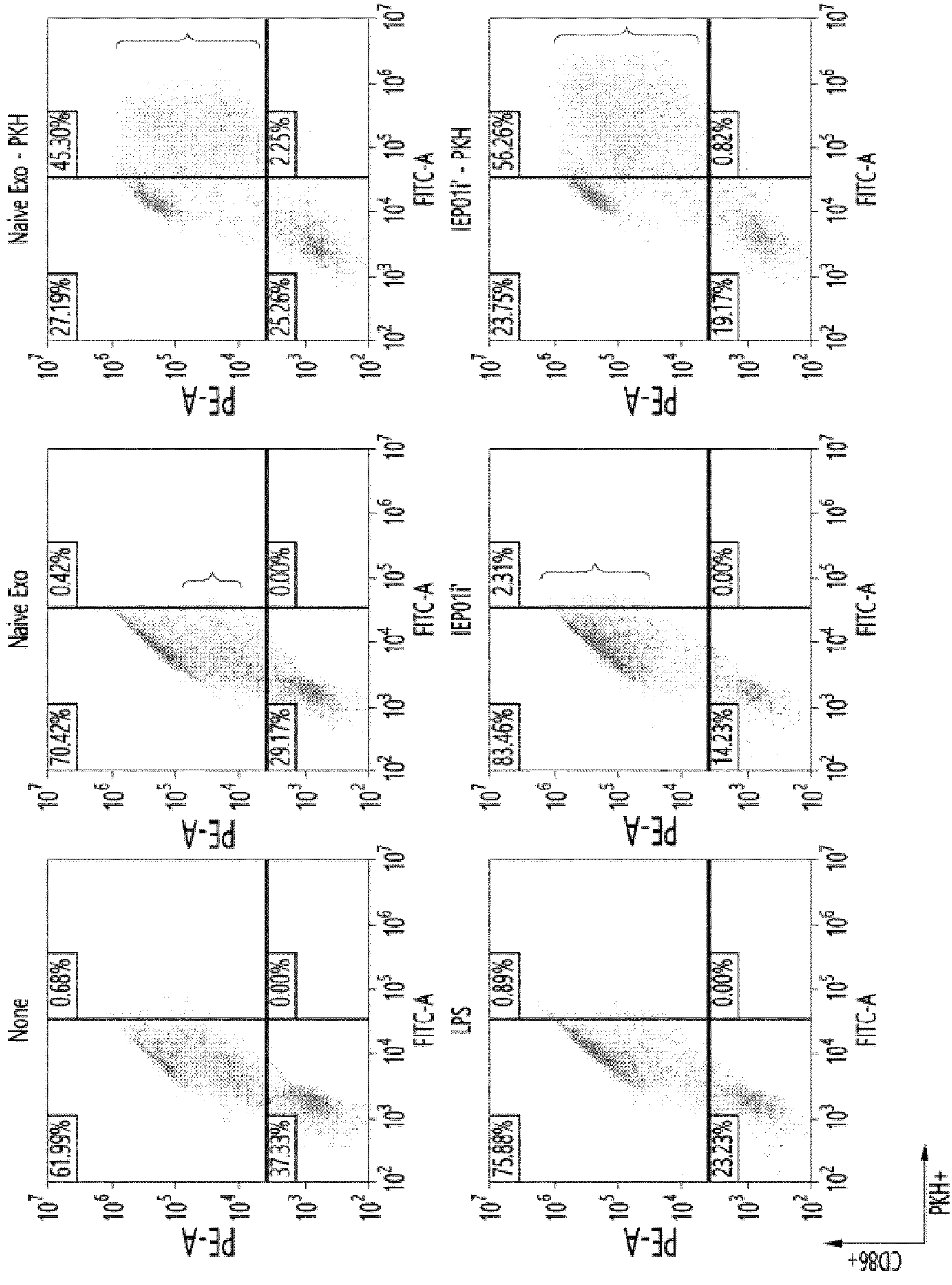
None
61.99%
0.68%
37.33%
0.00%
Naive Exo
70.42%
0.42%
29.17%
0.00%
Naive Exo - PKH
27.19%
45.30%
25.26%
2.25%
LPS
75.88%
0.89%
23.23%
0.00%
IEP01i'
83.46%
2.31%
14.23%
0.00%
IEP01i' - PKH
23.75%
56.26%
19.17%
0.82%
PE-A
FITC-A
CD86+
PKH+

[Figure 16i]

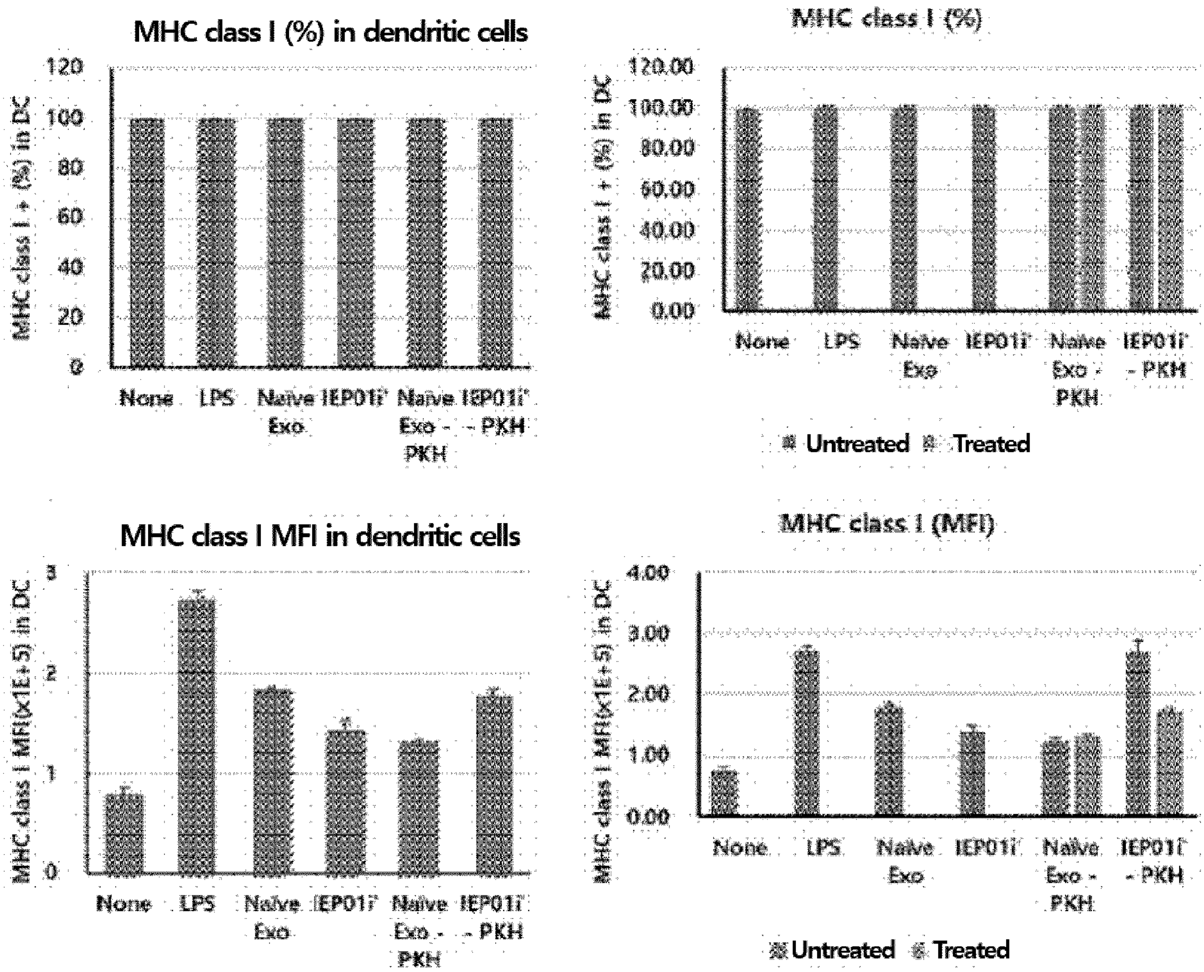
MHC class I (%) in dendritic cells
MHC class I + (%) in DC
120
100
80
60
40
20
0
None
LPS
Naive Exo
IEP01i'
Naive Exo - PKH
IEP01i' - PKH
MHC class I (%)
MHC class I + (%) in DC
120.00
100.00
80.00
60.00
40.00
20.00
0.00
Untreated
Treated
MHC class I MFI in dendritic cells
MHC class I MFI(x1E+5) in DC
3
2
1
0
MHC class I (MFI)
4.00
3.00
2.00
1.00
0.00

[Figure 16j]

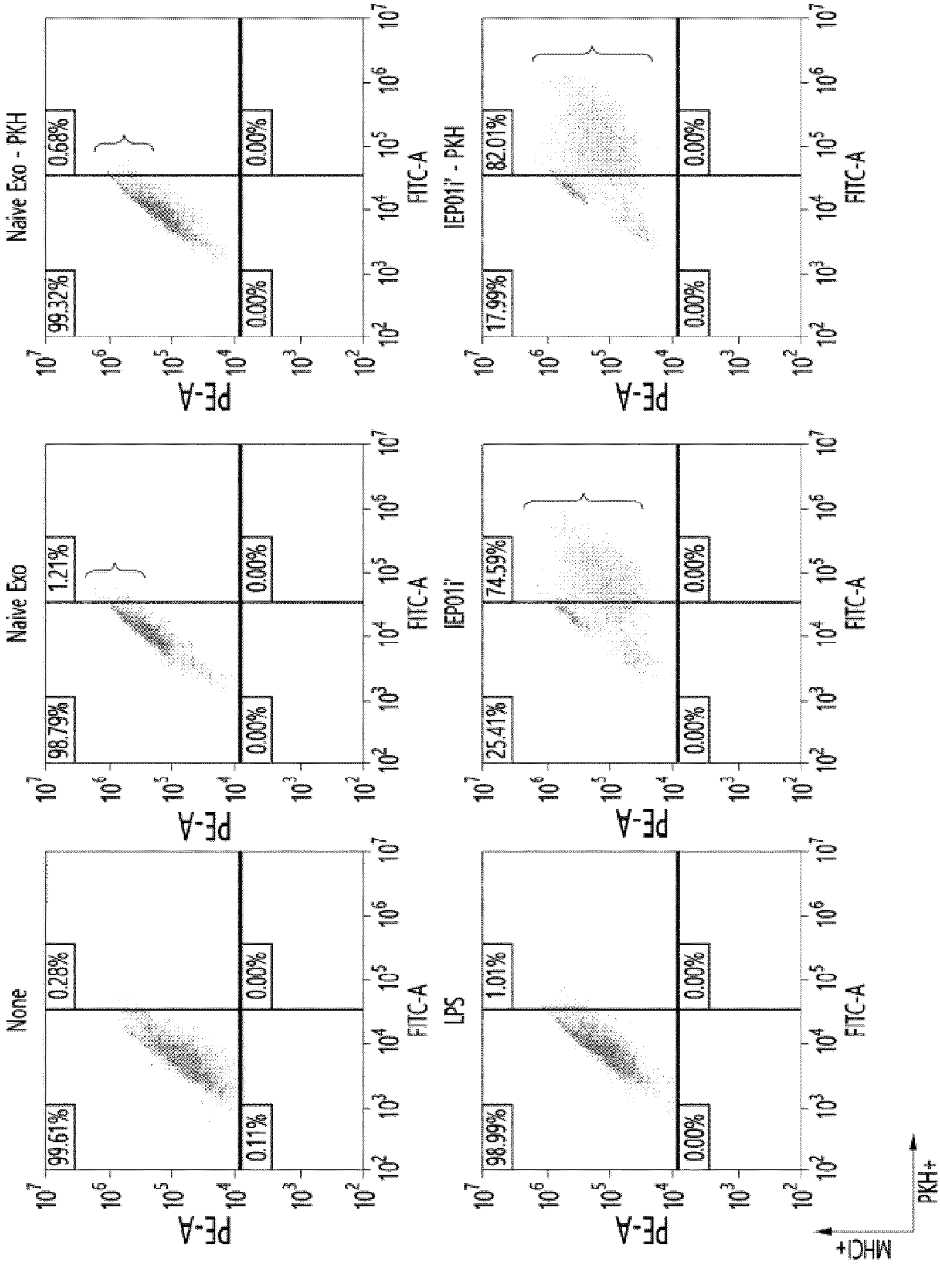
None
99.61%
0.28%
0.11%
0.00%
Naive Exo
98.79%
1.21%
0.00%
0.00%
Naive Exo - PKH
99.32%
0.68%
0.00%
0.00%
LPS
98.99%
1.01%
0.00%
0.00%
IEP01i'
25.41%
74.59%
0.00%
0.00%
IEP01i' - PKH
17.99%
82.01%
0.00%
0.00%
PE-A
FITC-A
MHCI+
PKH+

[Figure 16k]

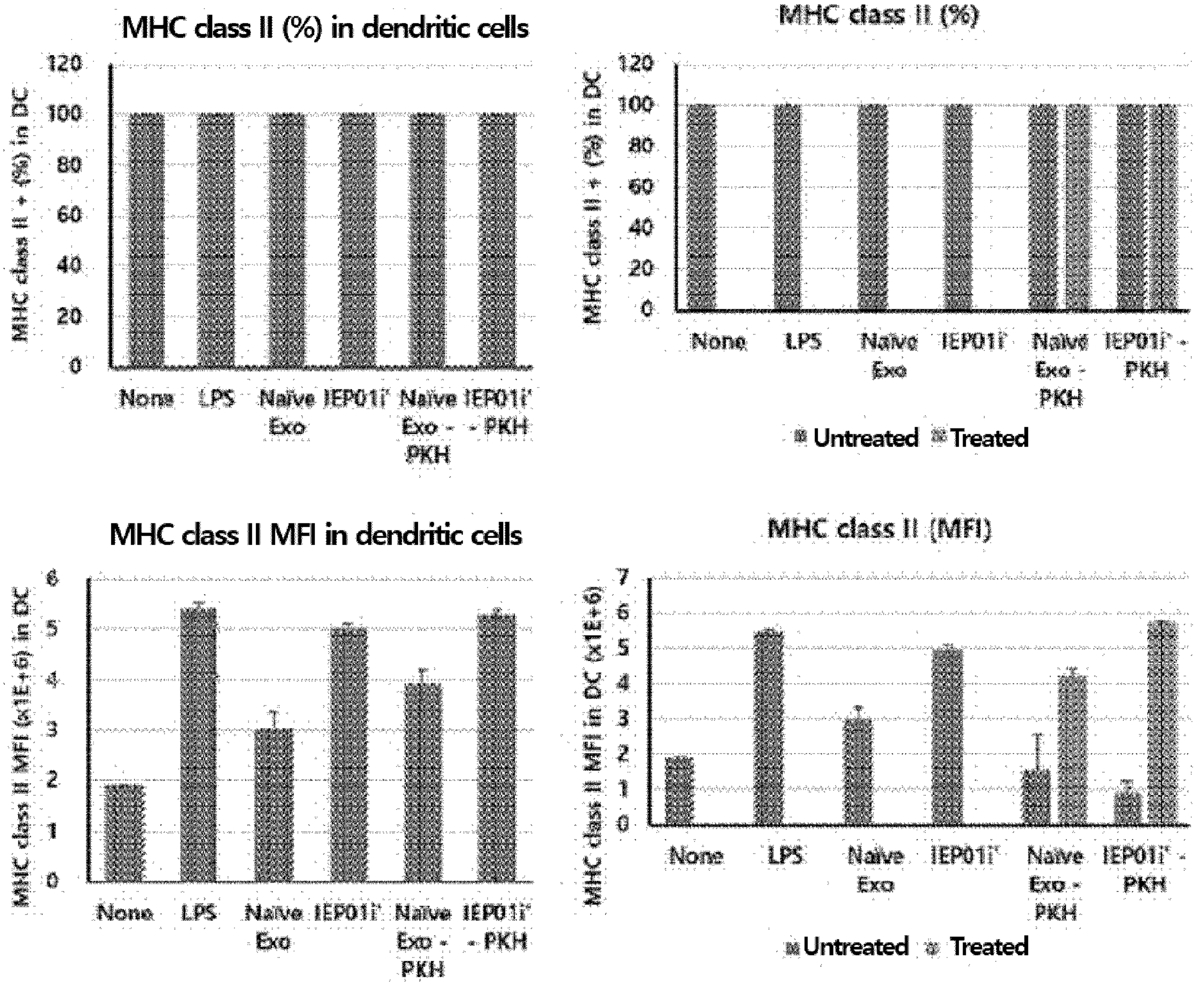
MHC class II (%) in dendritic cells
MHC class II + (%) in DC
120
100
80
60
40
20
0
None
LPS
Naïve Exo
IEP01i'
Naïve Exo - PKH
IEP01i' - PKH
MHC class II (%)
MHC class II + (%) in DC
Untreated
Treated
MHC class II MFI in dendritic cells
MHC class II MFI (x1E+6) in DC
6
5
4
3
2
1
MHC class II (MFI)
MHC class II MFI in DC (x1E+6)
7

**[Figure 16l]**

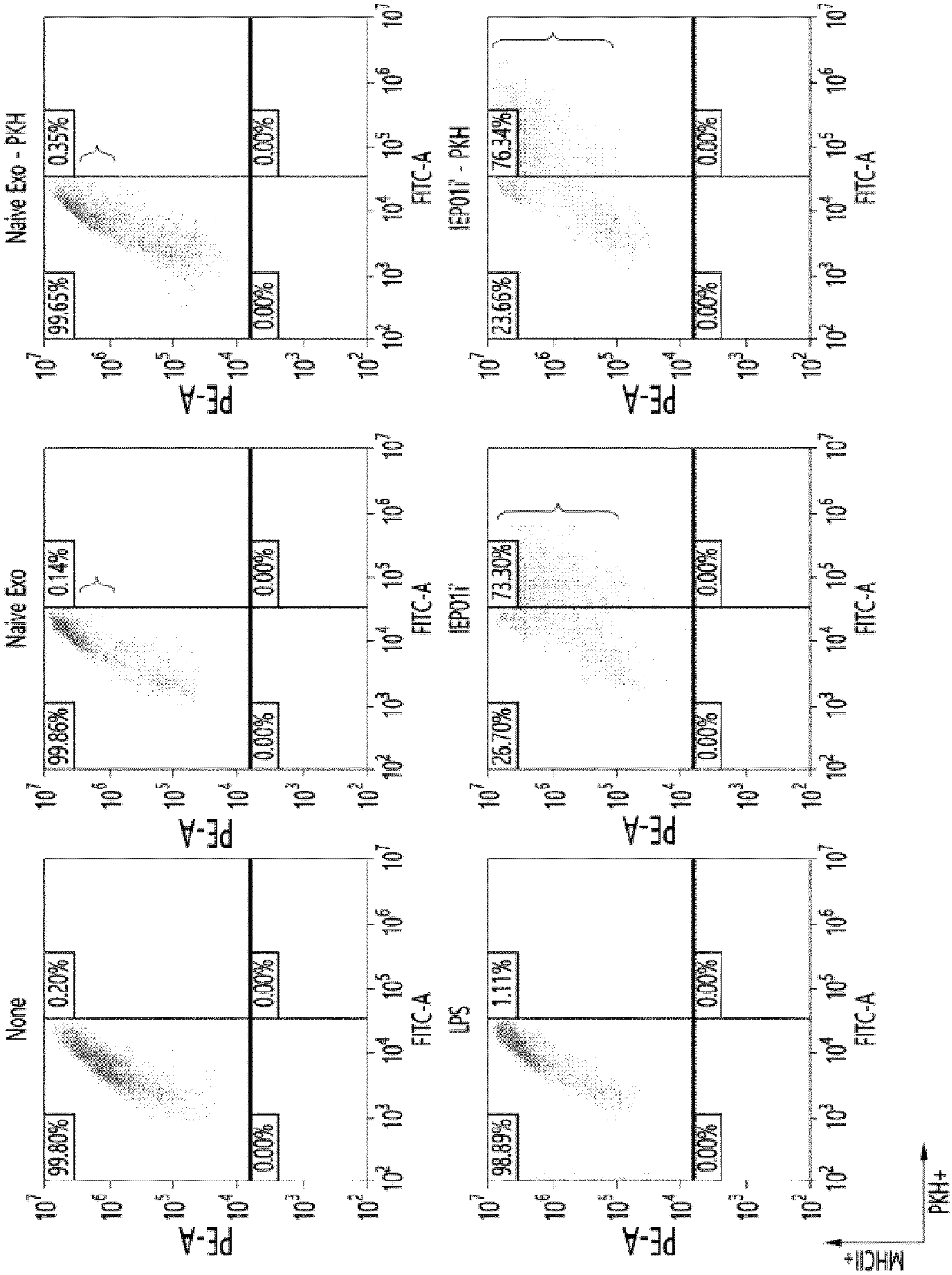
None
LPS
Naive Exo
IEP01i'
Naive Exo - PKH
IEP01i' - PKH
PE-A
FITC-A
99.80%
0.20%
0.00%
0.00%
98.89%
1.11%
99.86%
0.14%
26.70%
73.30%
99.65%
0.35%
23.66%
76.34%
MHCII+
PKH+

[Figure 17a]

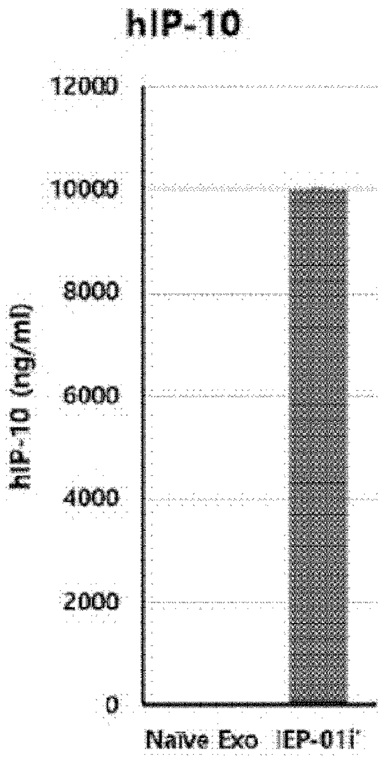


[Figure 17b]

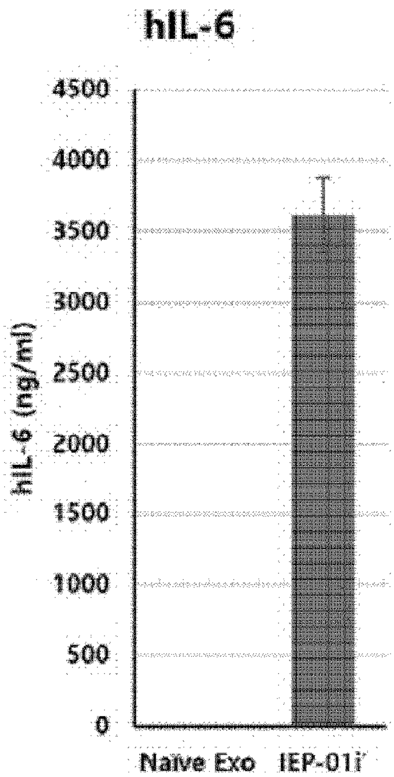

[Figure 17c]

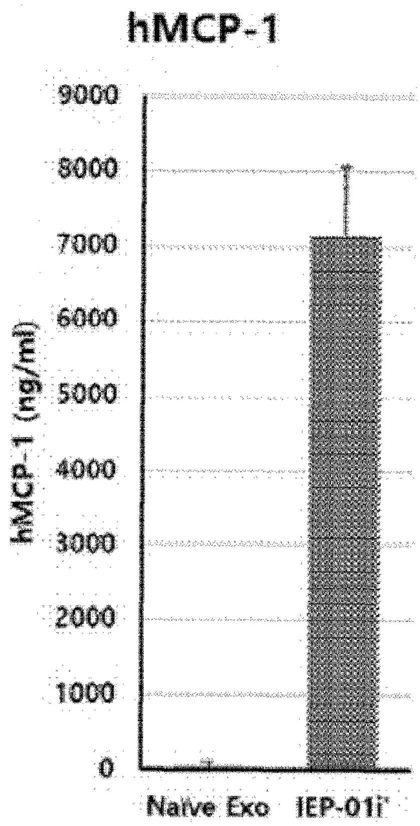


[Figure 17d]

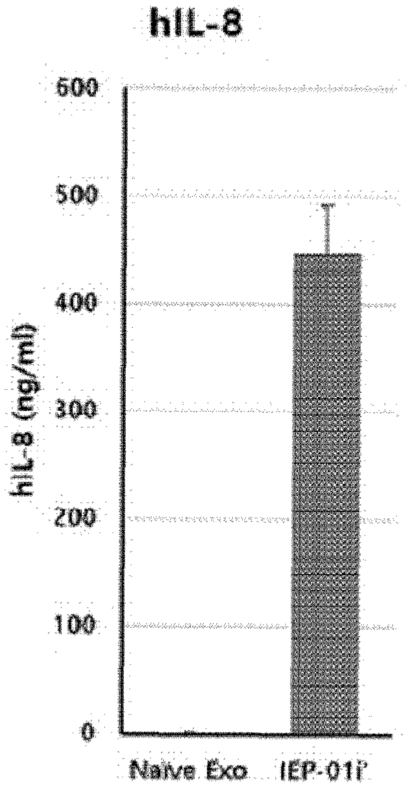

[Figure 17e]

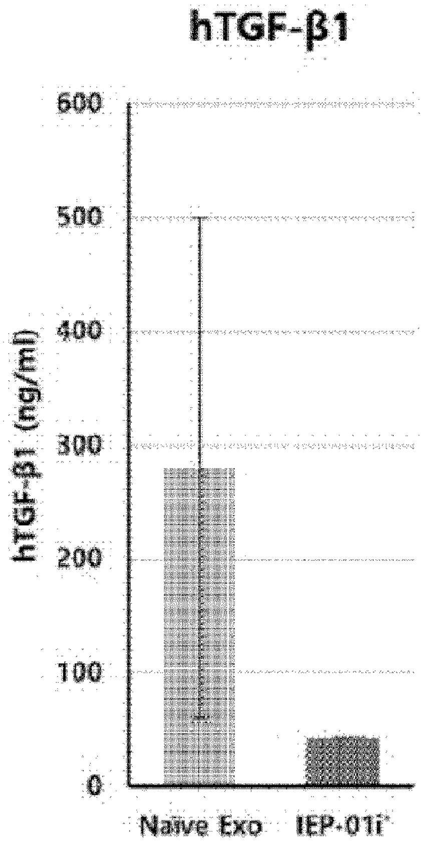


[Figure 18a]

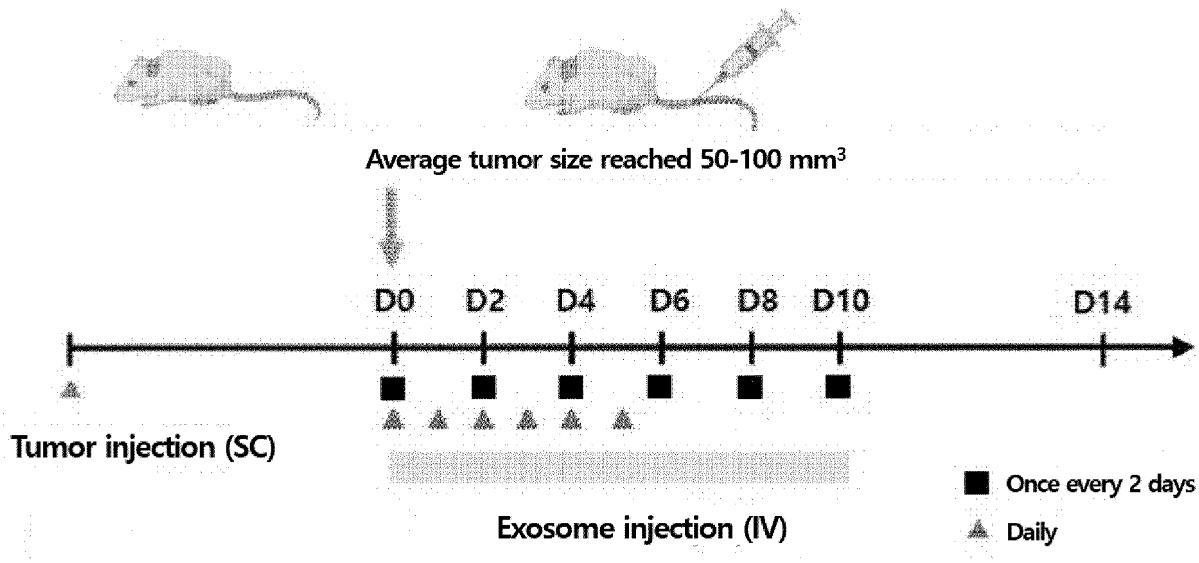

[Figure 18b]

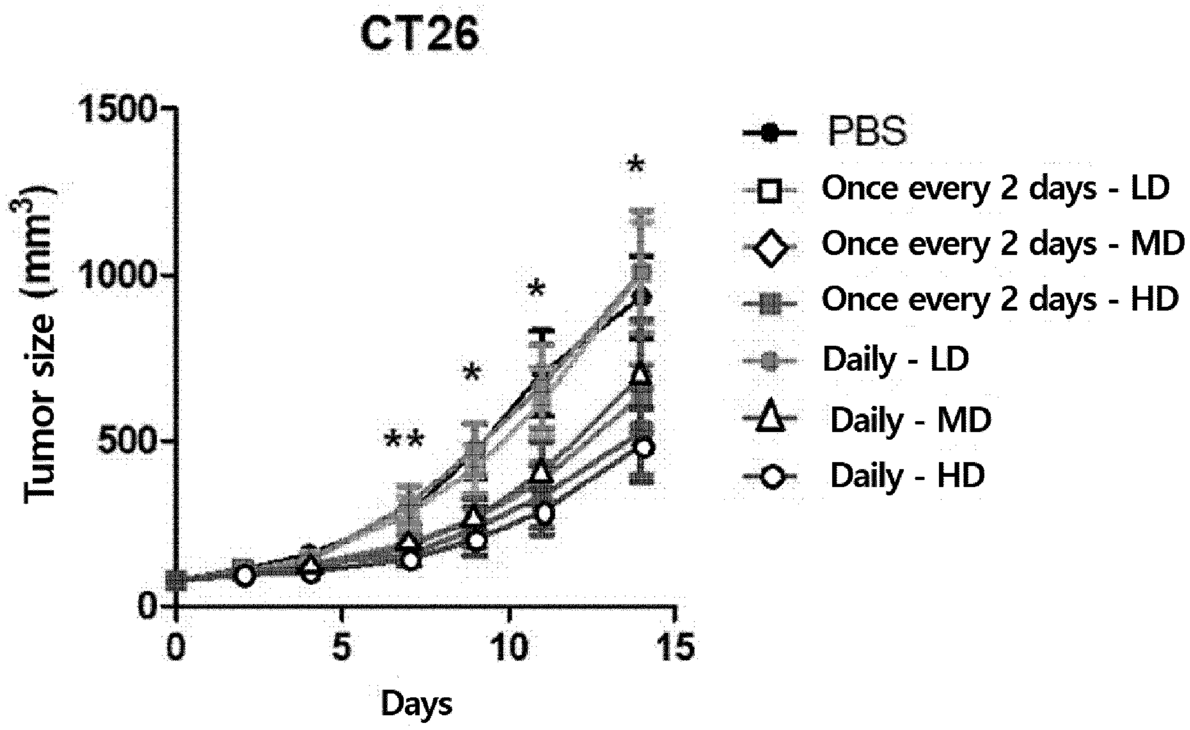


[Figure 18c]

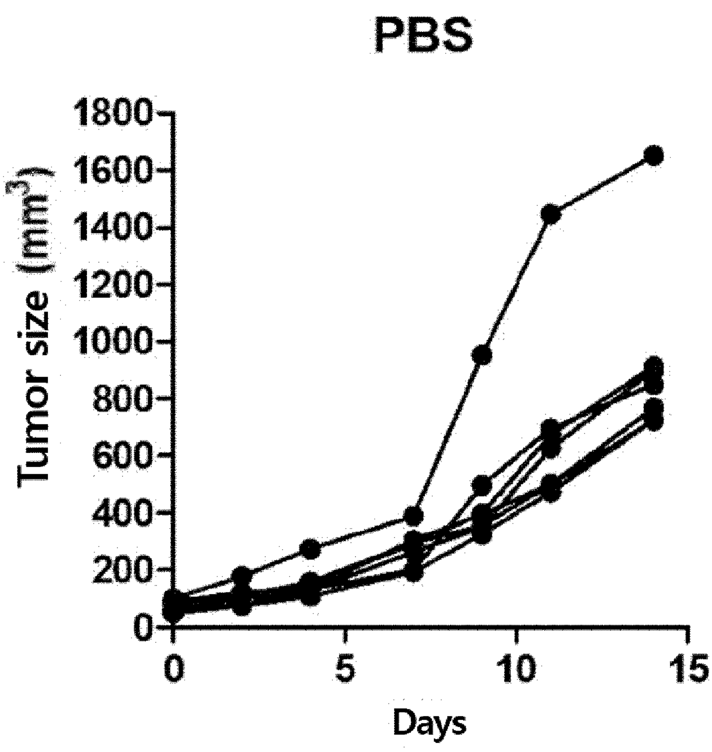

[Figure 18d]

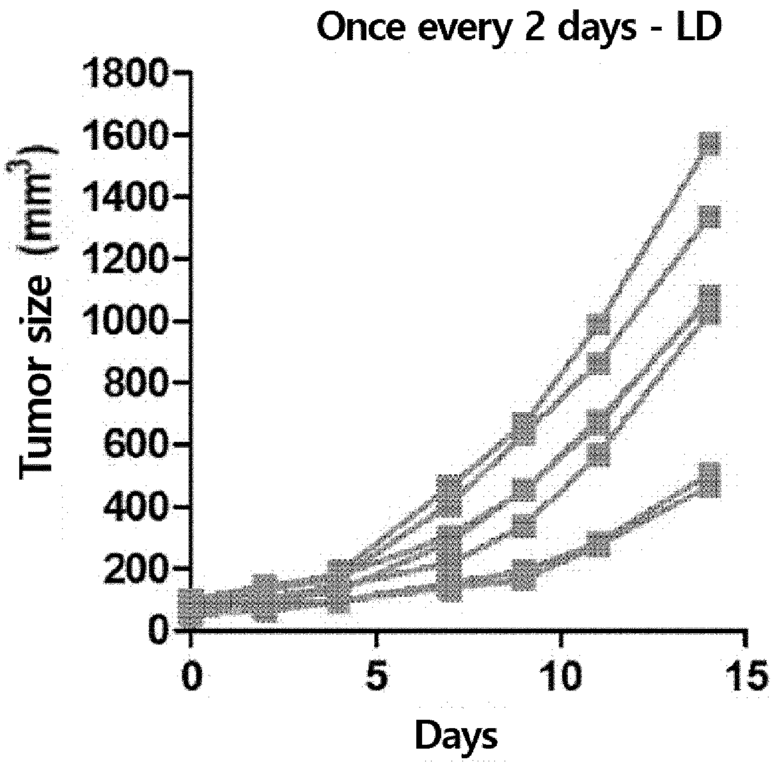


[Figure 18e]

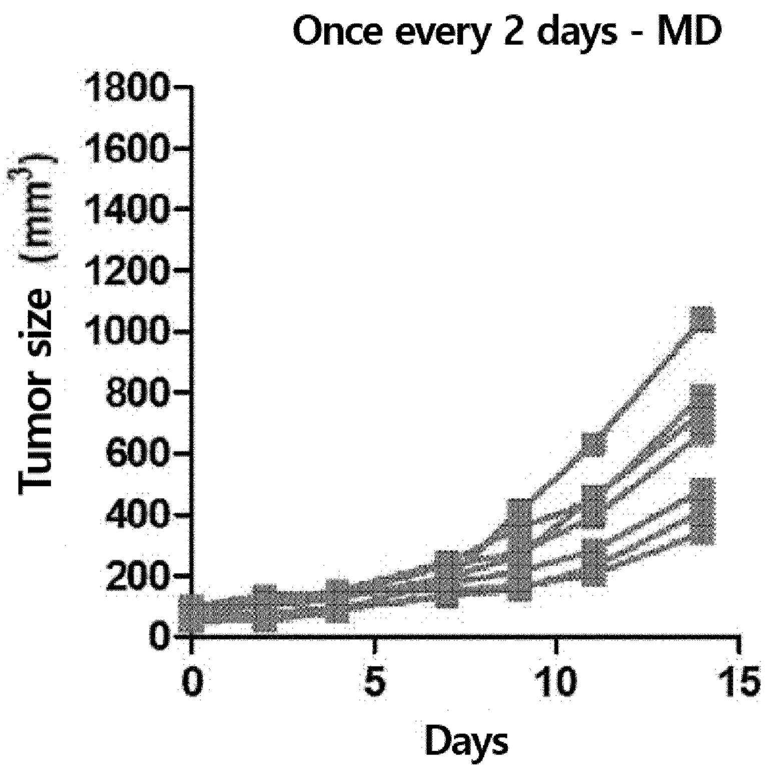

[Figure 18f]

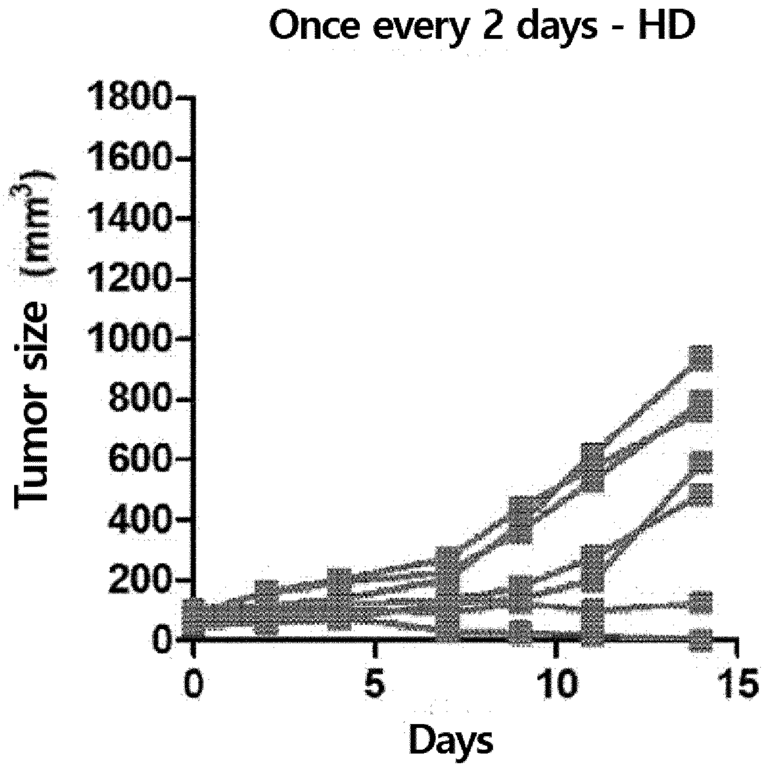


[Figure 18g]

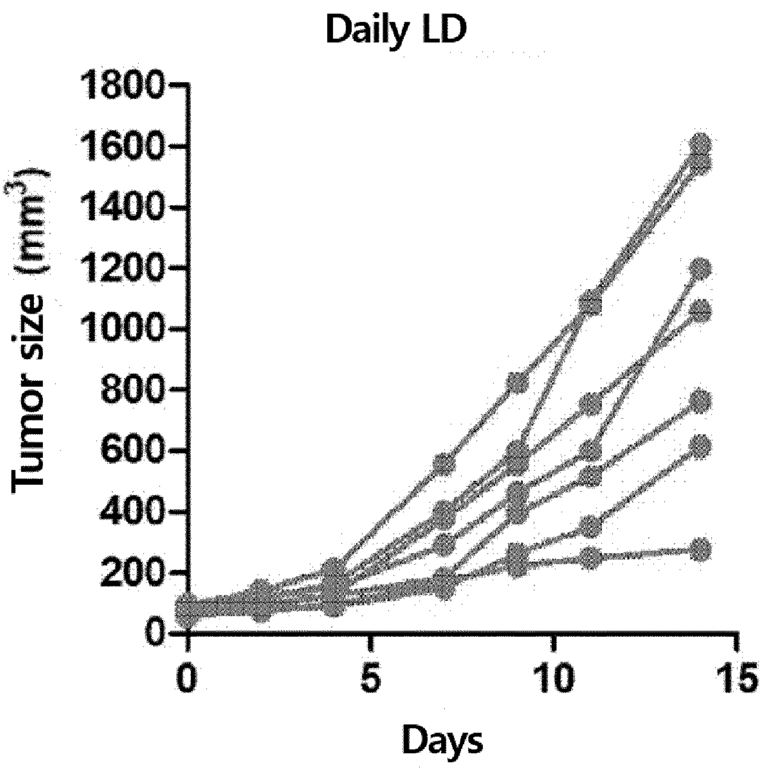

[Figure 18h]

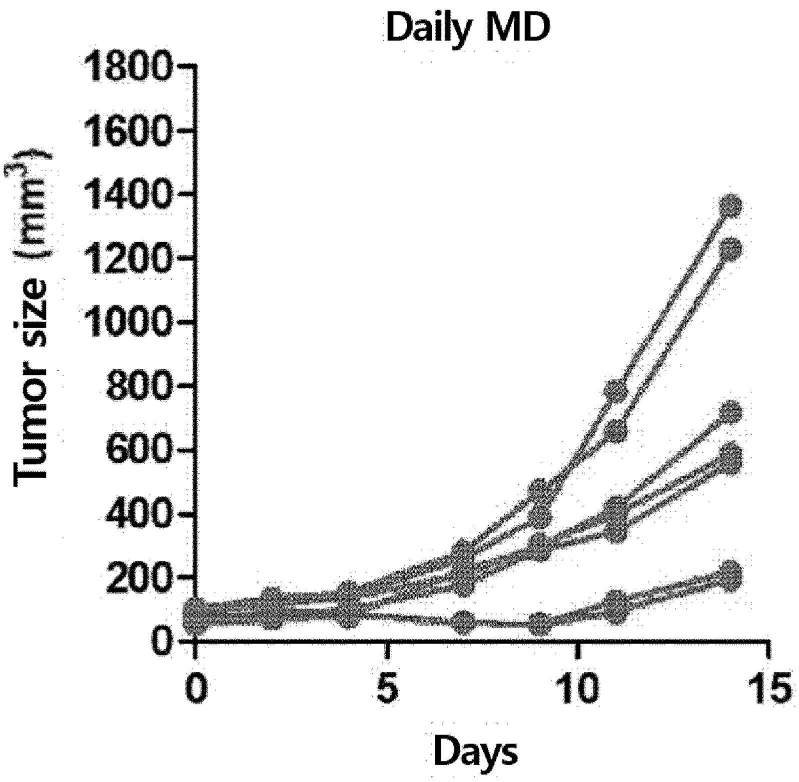


[Figure 18i]

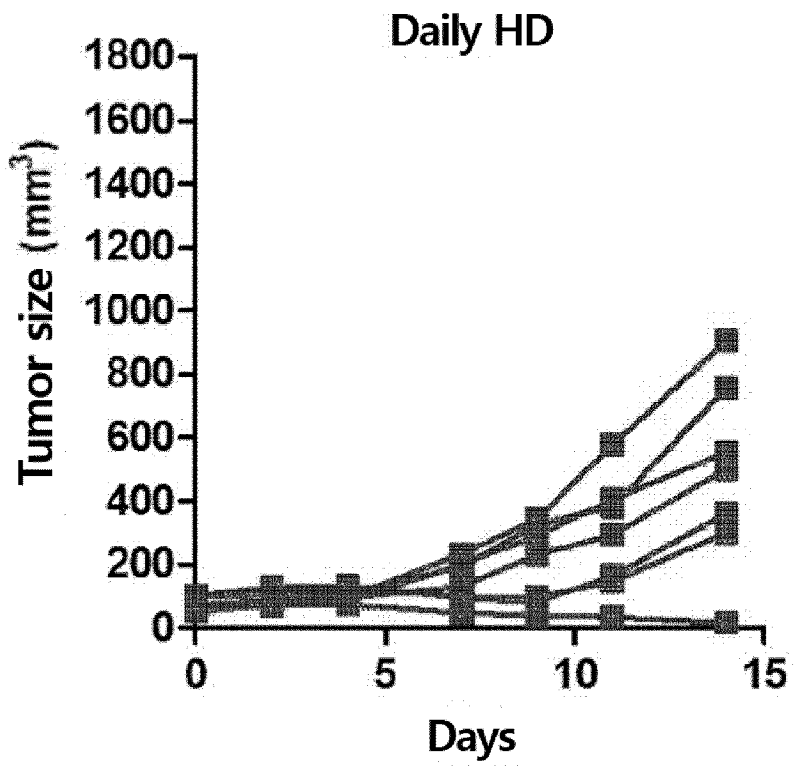

[Figure 19a]

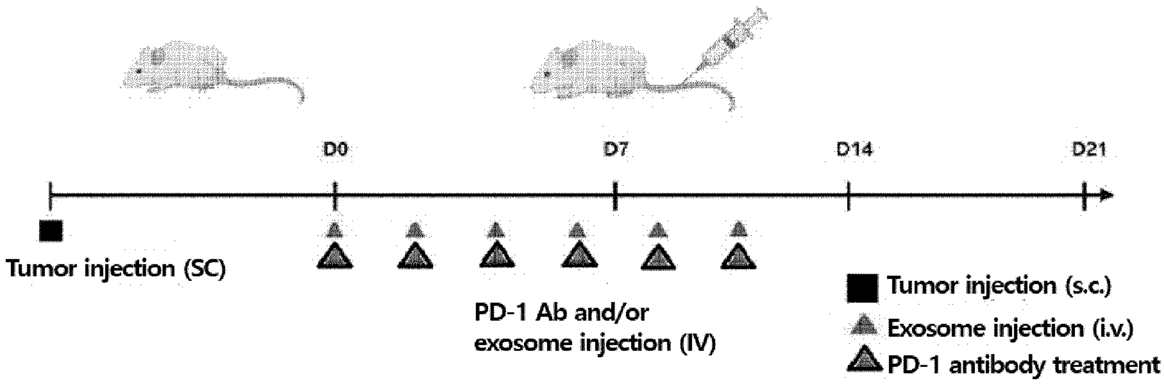


[Figure 19b]

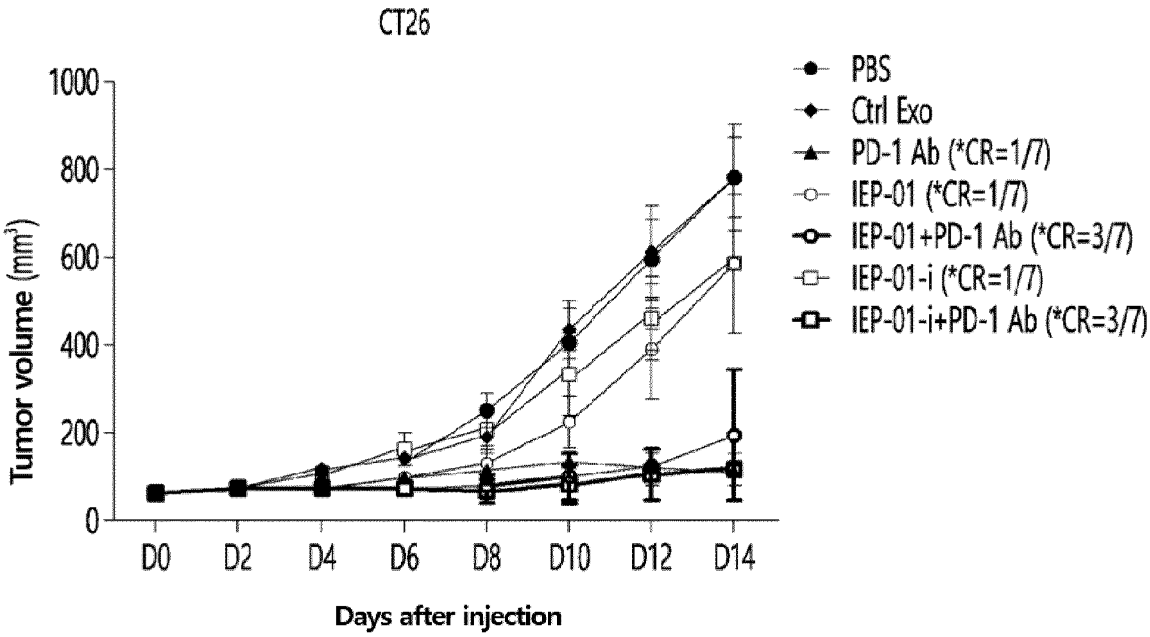


[Figure 19c]

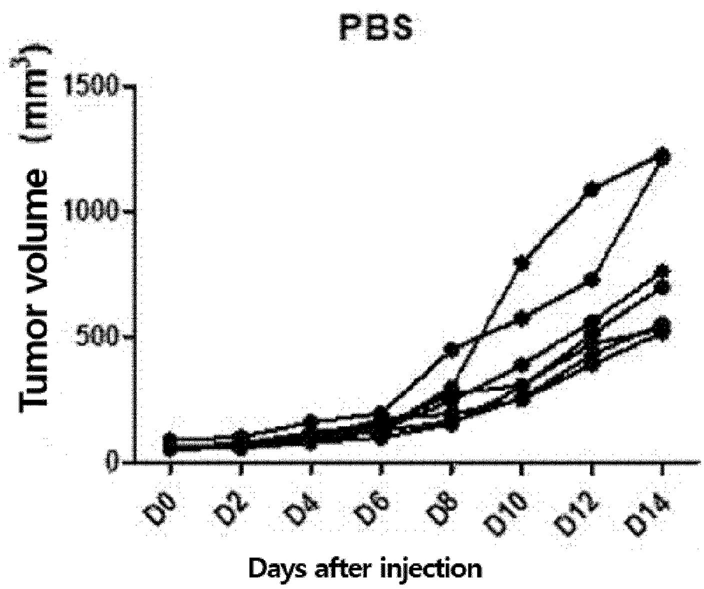

[Figure 19d]

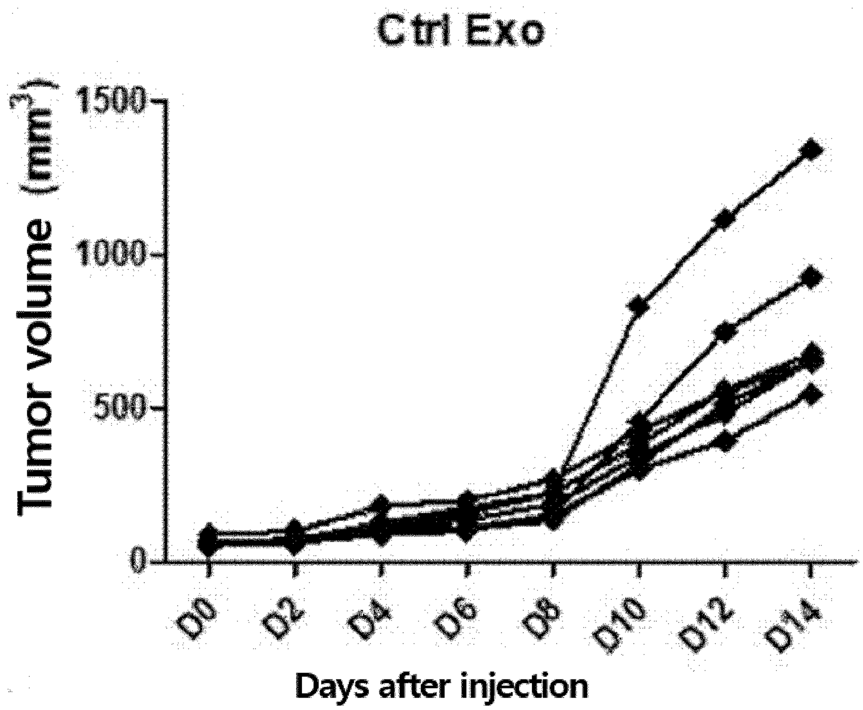


[Figure 19e]

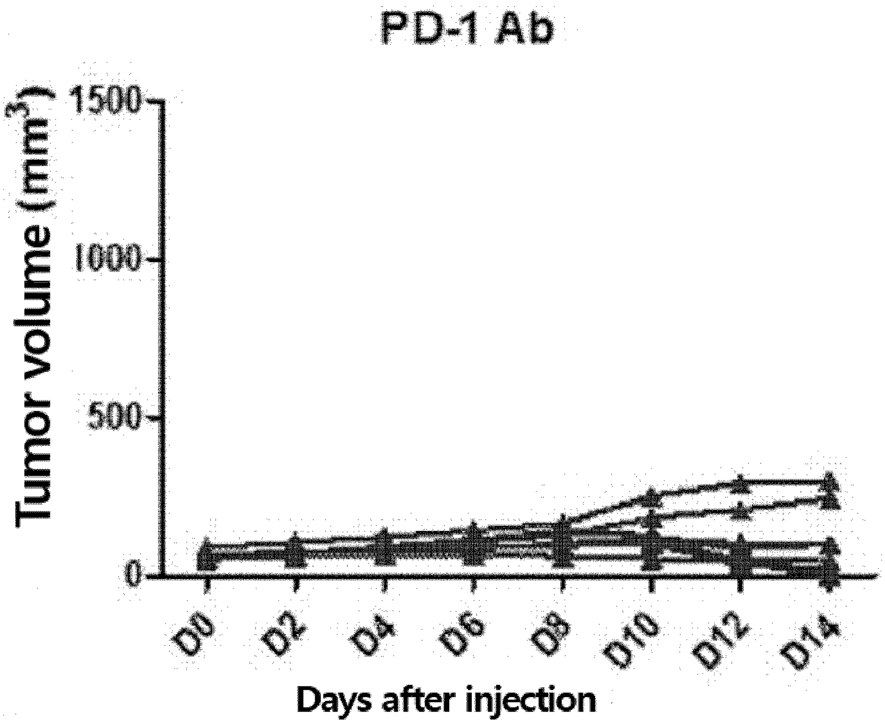

[Figure 19f]

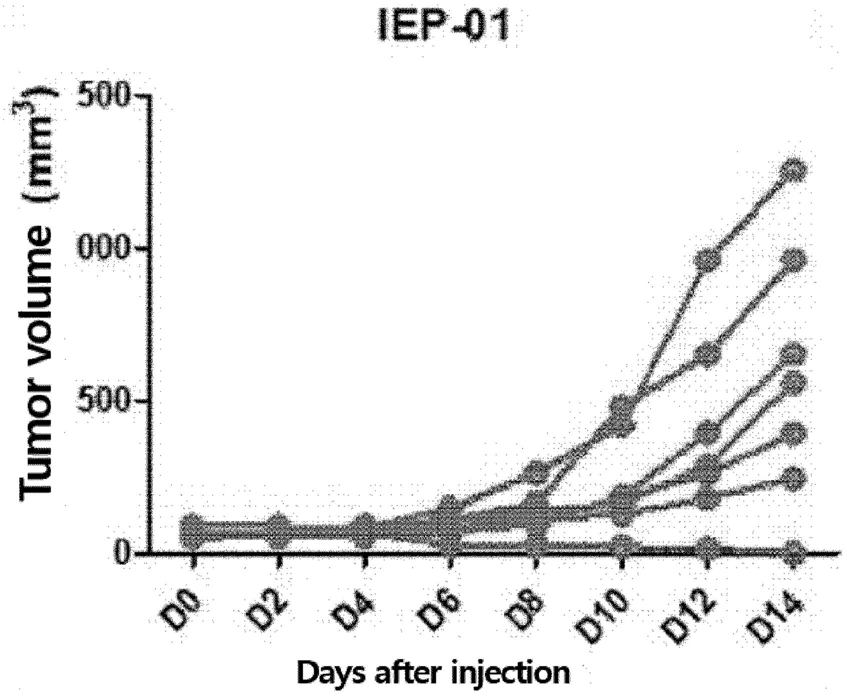


[Figure 19g]

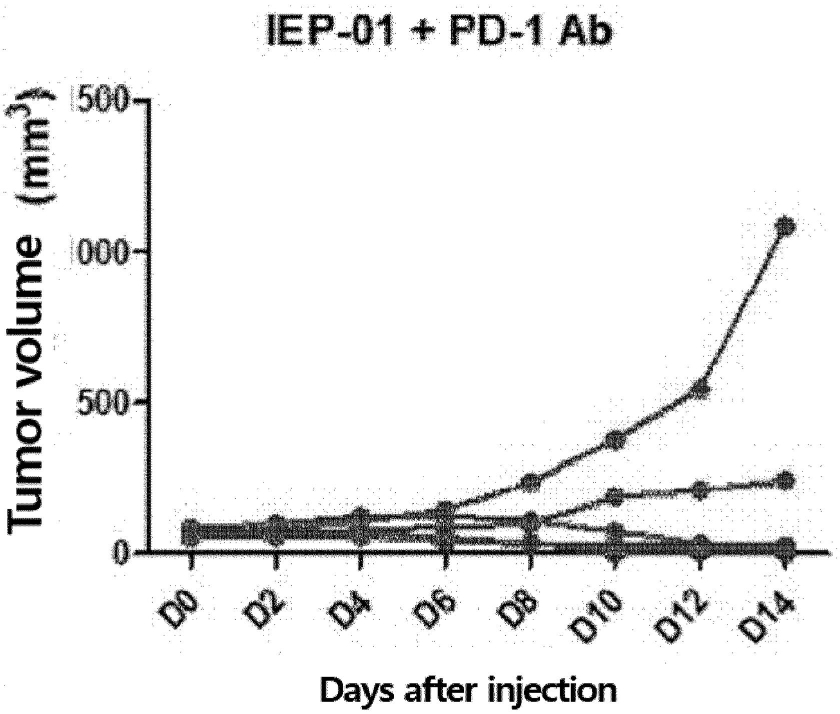

[Figure 19h]

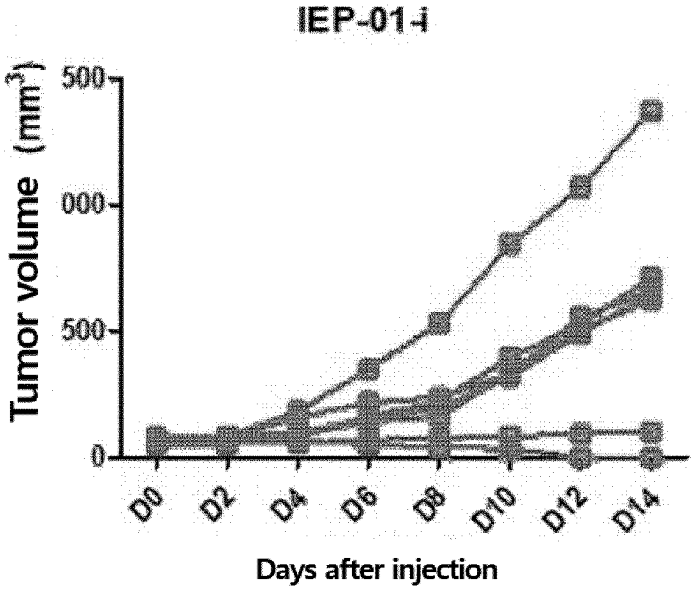


[Figure 19i]

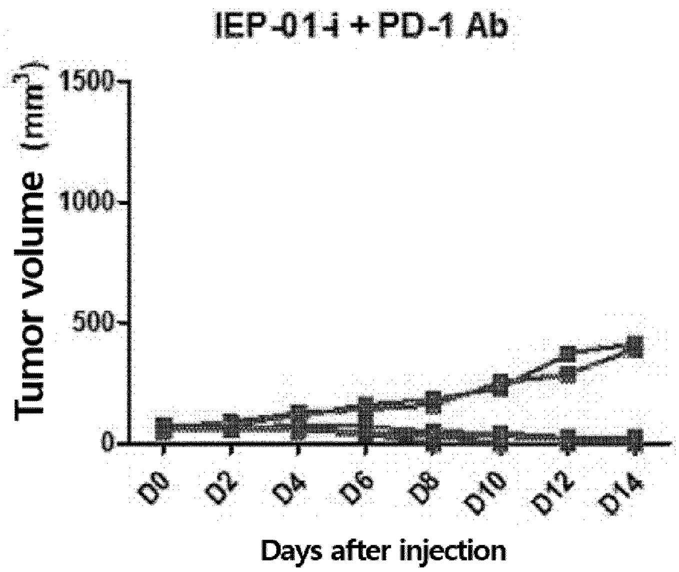


[Figure 20a]

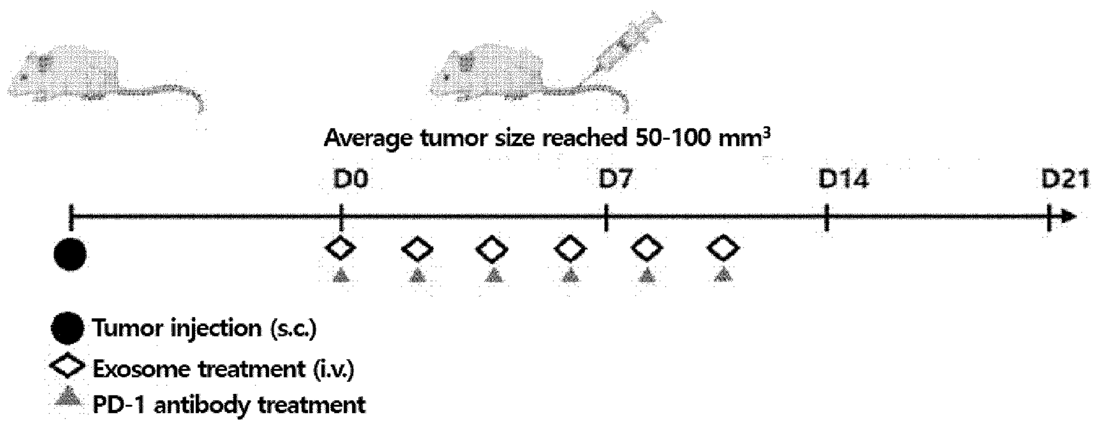

[Figure 20b]

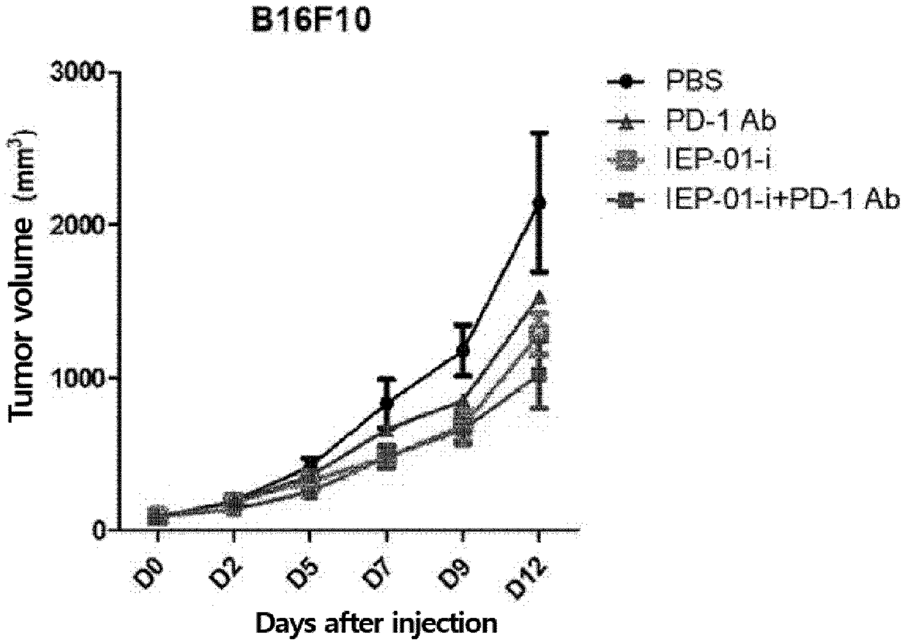


[Figure 20c]

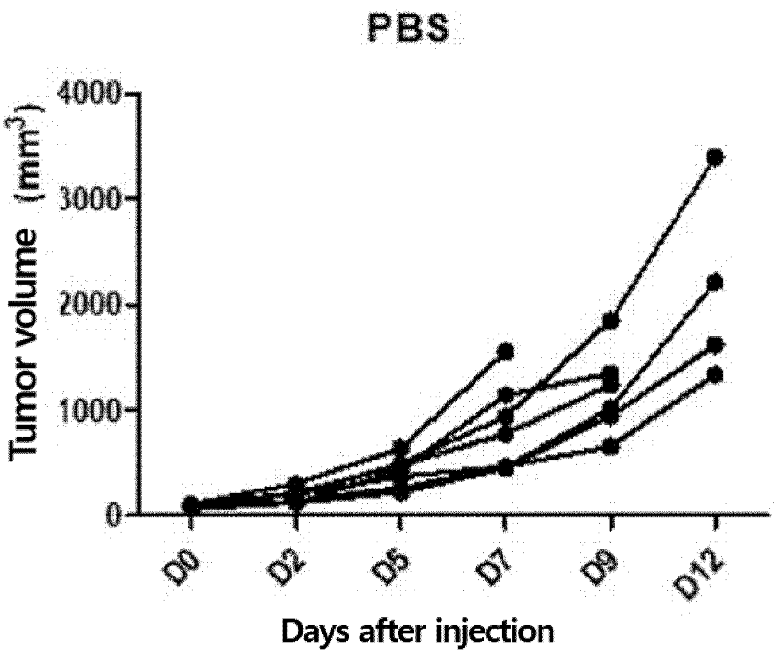

[Figure 20d]

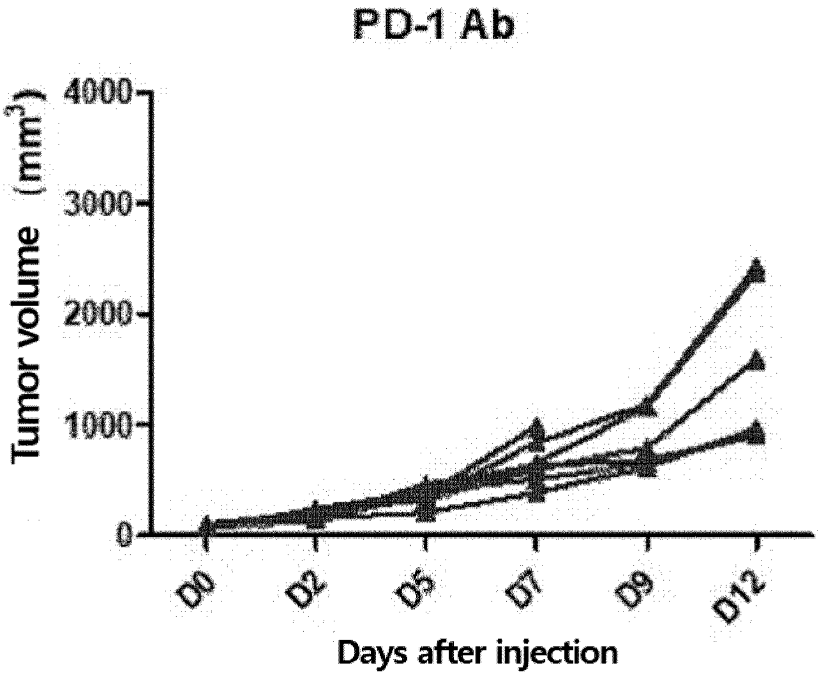


[Figure 20e]

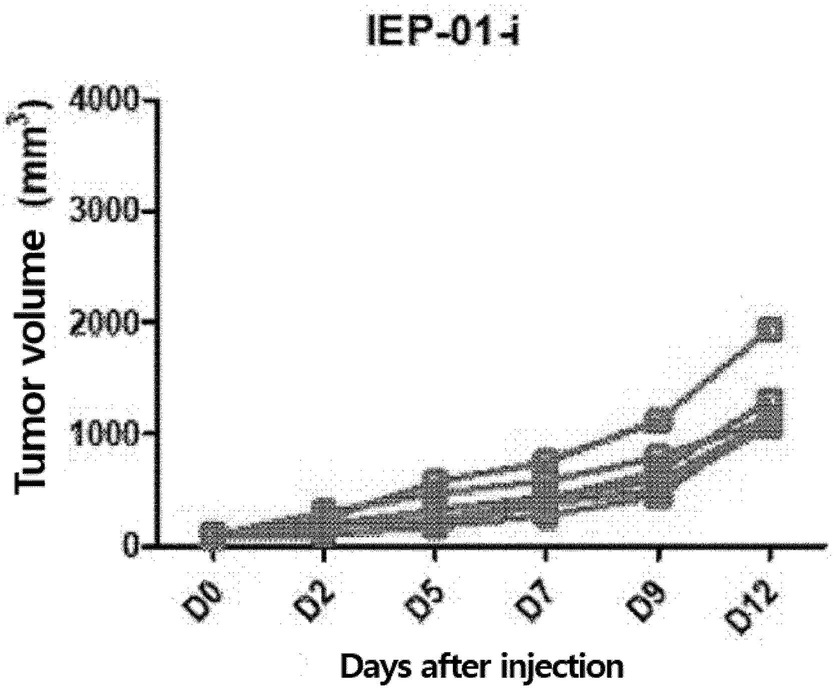

[Figure 20f]

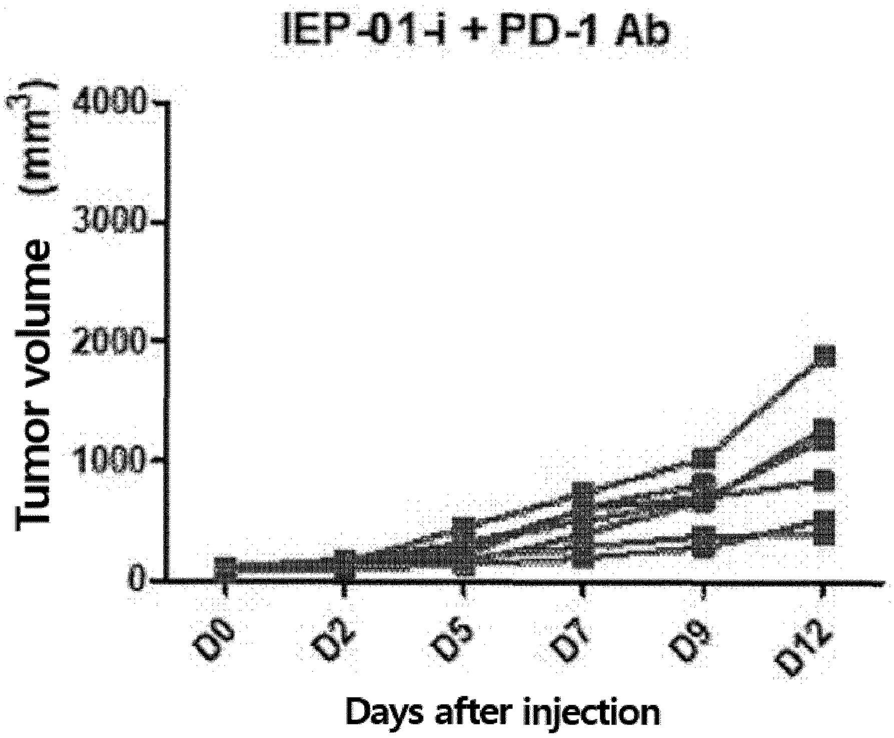


[Figure 21a]

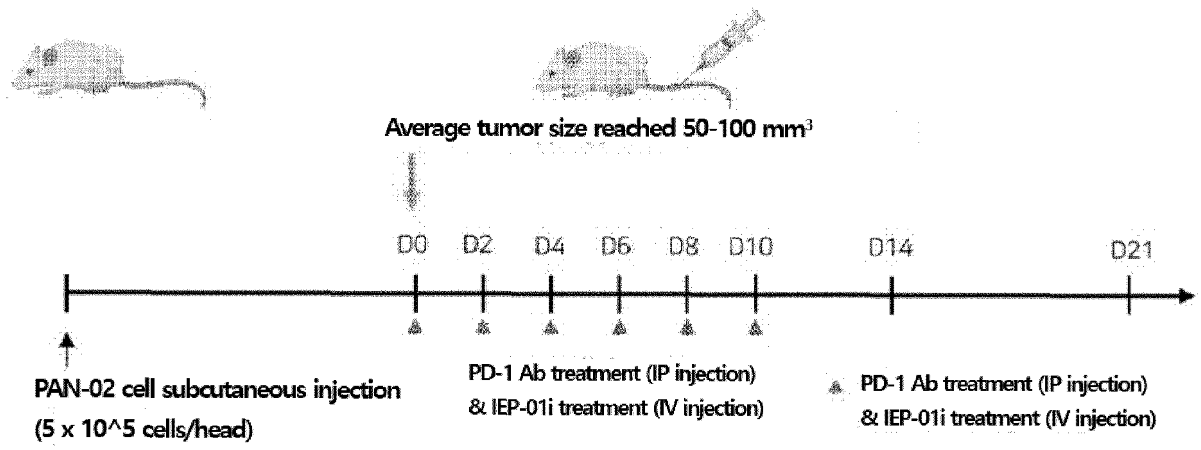

[Figure 21b]

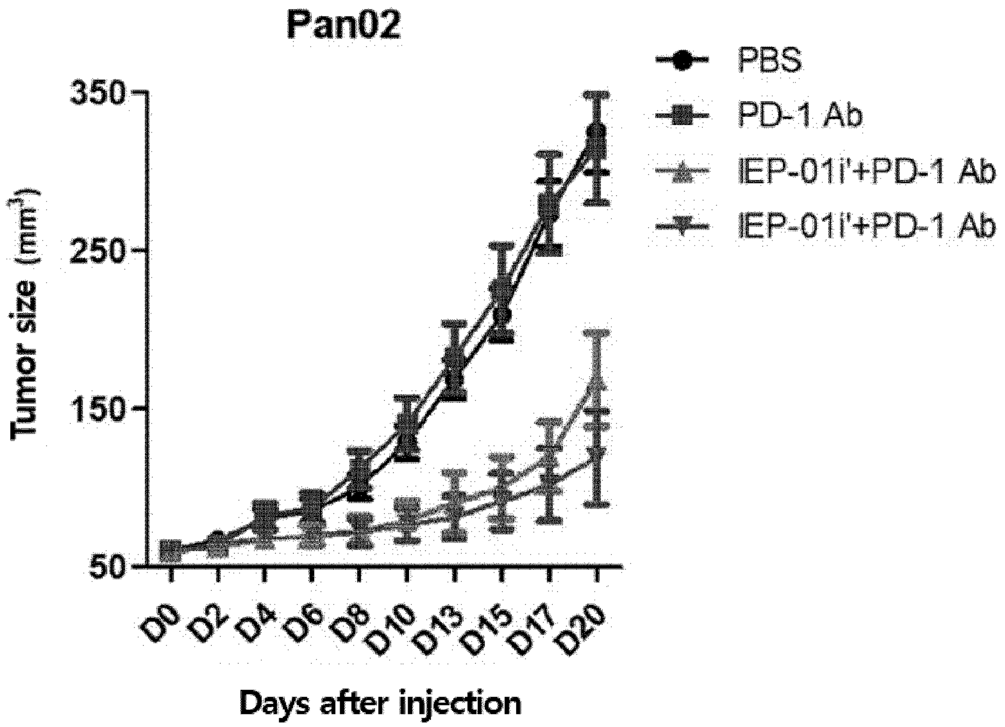


[Figure 21c]

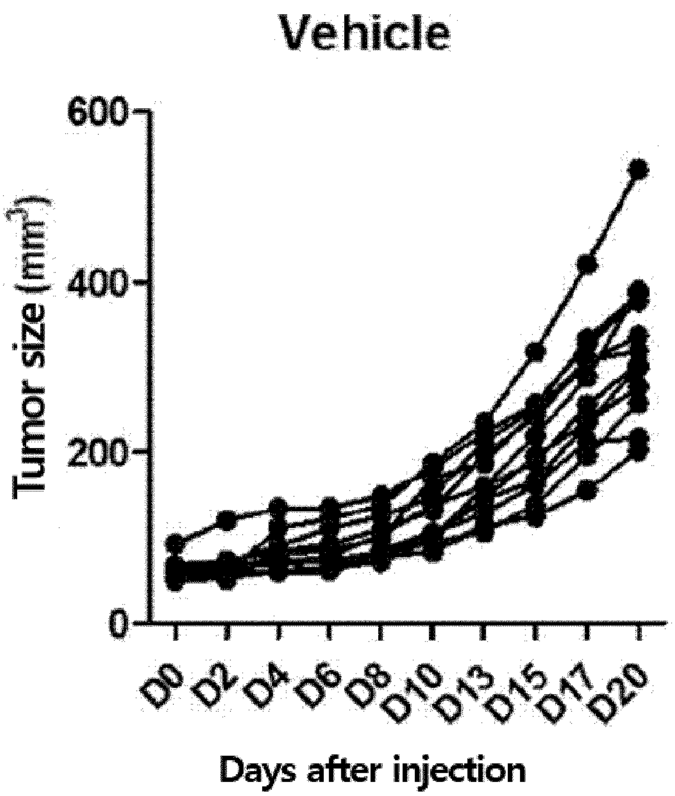

[Figure 21d]

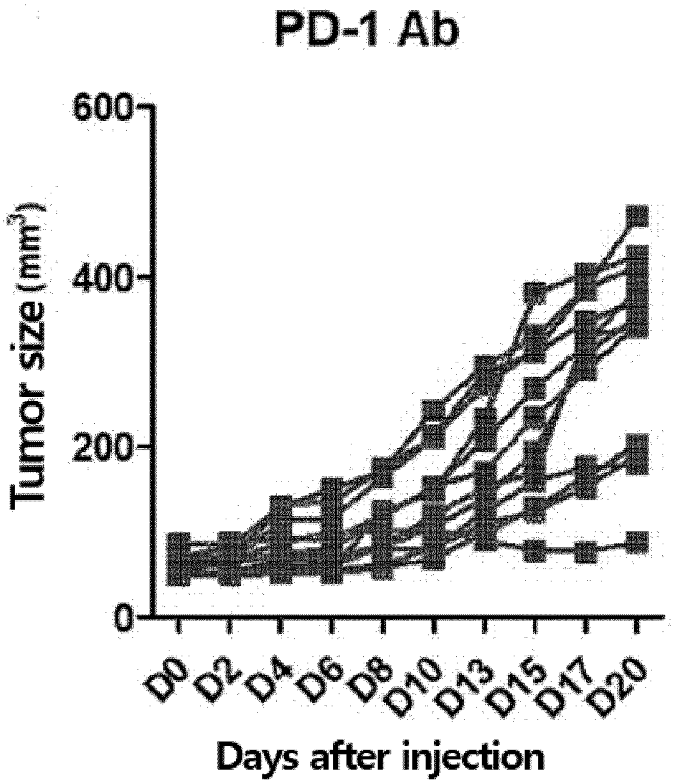


[Figure 21e]

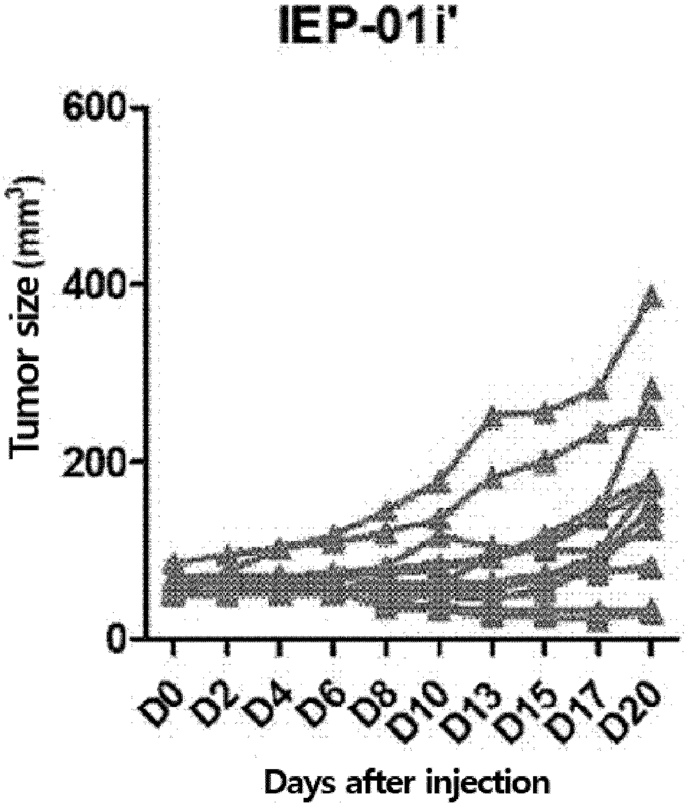

[Figure 21f]

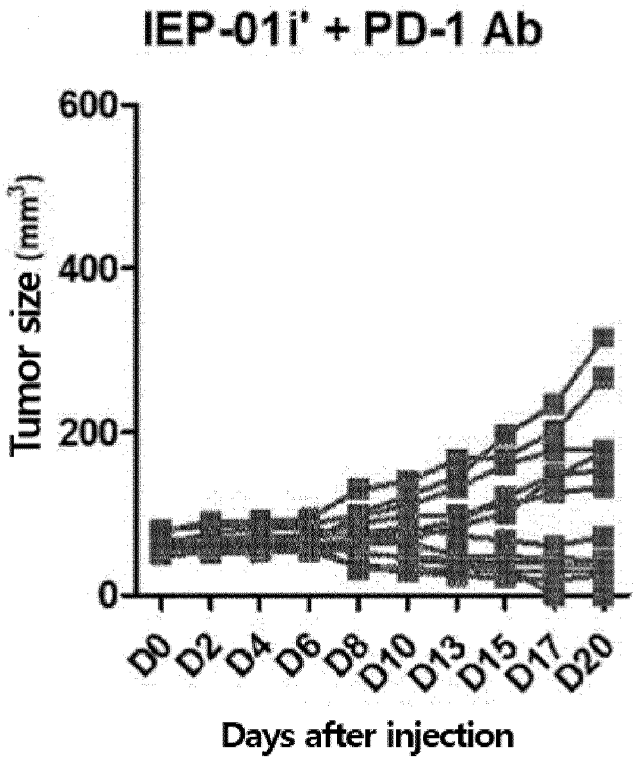
IEP-01i' + PD-1 Ab
600
400
200
0
Tumor size (mm3)
D0
D2
D4
D6
D8
D10
D13
D15
D17
D20
Days after injection

[Figure 22a]

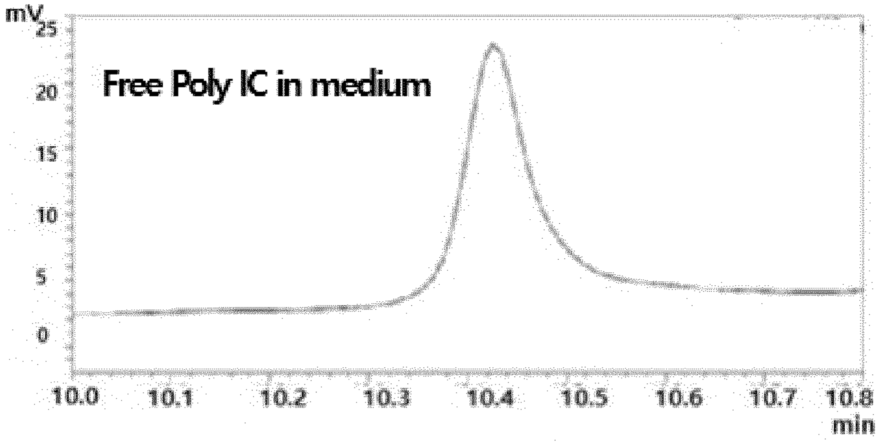


Before purification

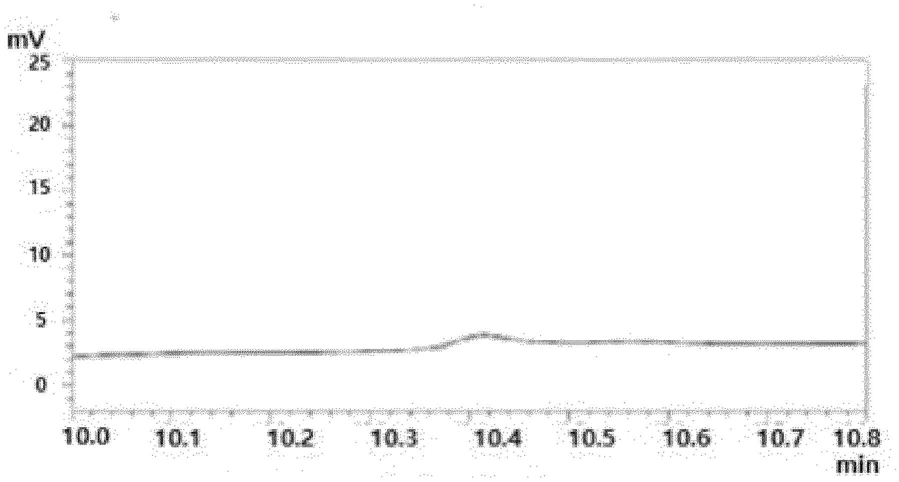


After purification

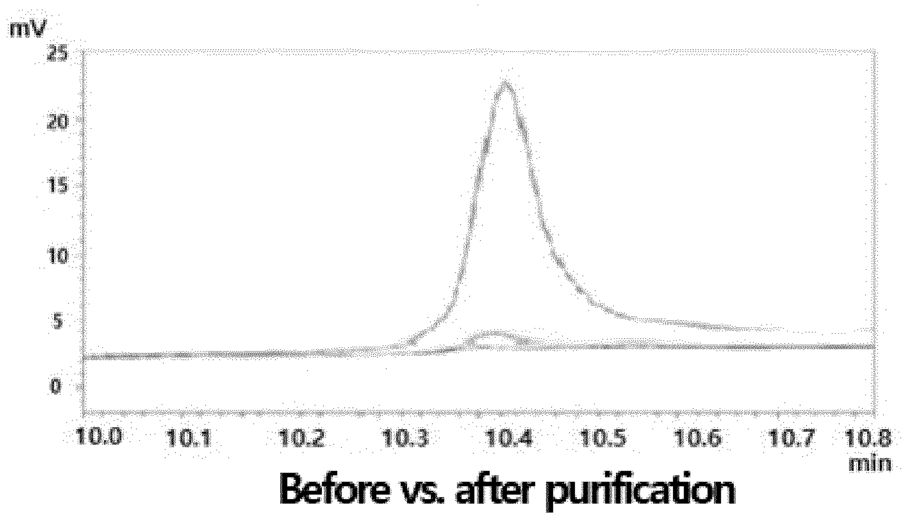


Before vs. after purification

[Figure 22b]

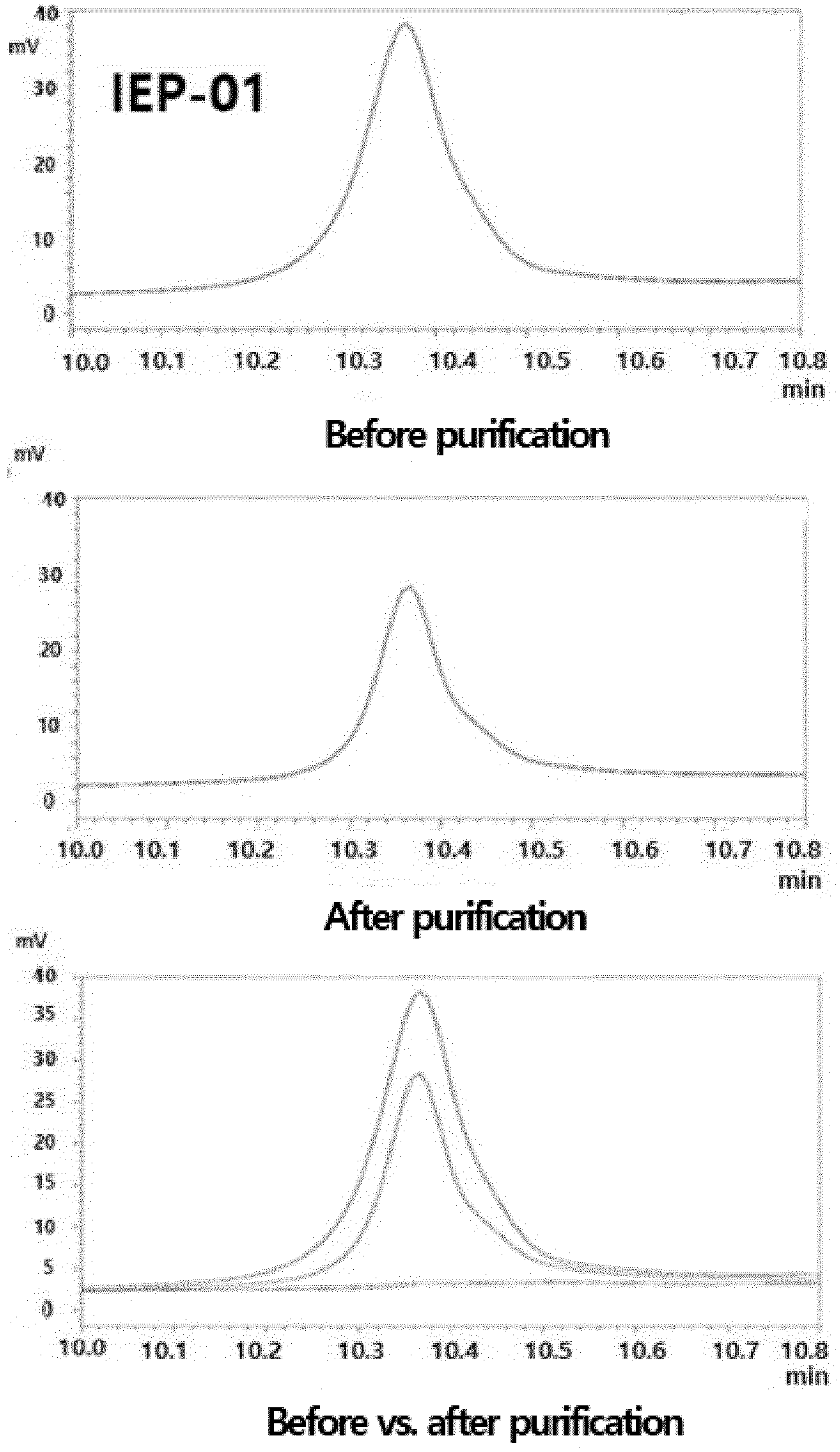
IEP-01
mV
40
30
20
10
0
10.0 10.1 10.2 10.3 10.4 10.5 10.6 10.7 10.8
min
Before purification
mV
40
30
20
10
0
10.0 10.1 10.2 10.3 10.4 10.5 10.6 10.7 10.8
min
After purification
mV
40
35
30
25
20
15
10
5
0
10.0 10.1 10.2 10.3 10.4 10.5 10.6 10.7 10.8
min
Before vs. after purification

[Figure 22c]

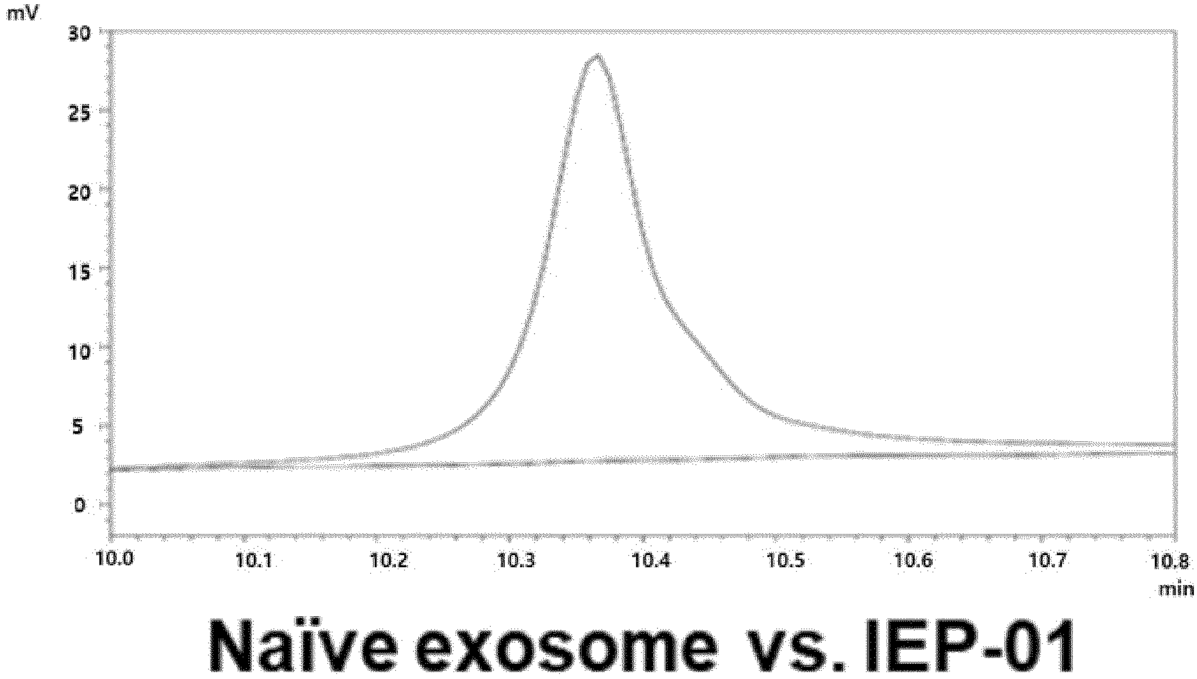
mV
30
25
20
15
10
5
0
10.0
10.1
10.2
10.3
10.4
10.5
10.6
10.7
10.8
min
Naïve exosome vs. IEP-01

[Figure 23]

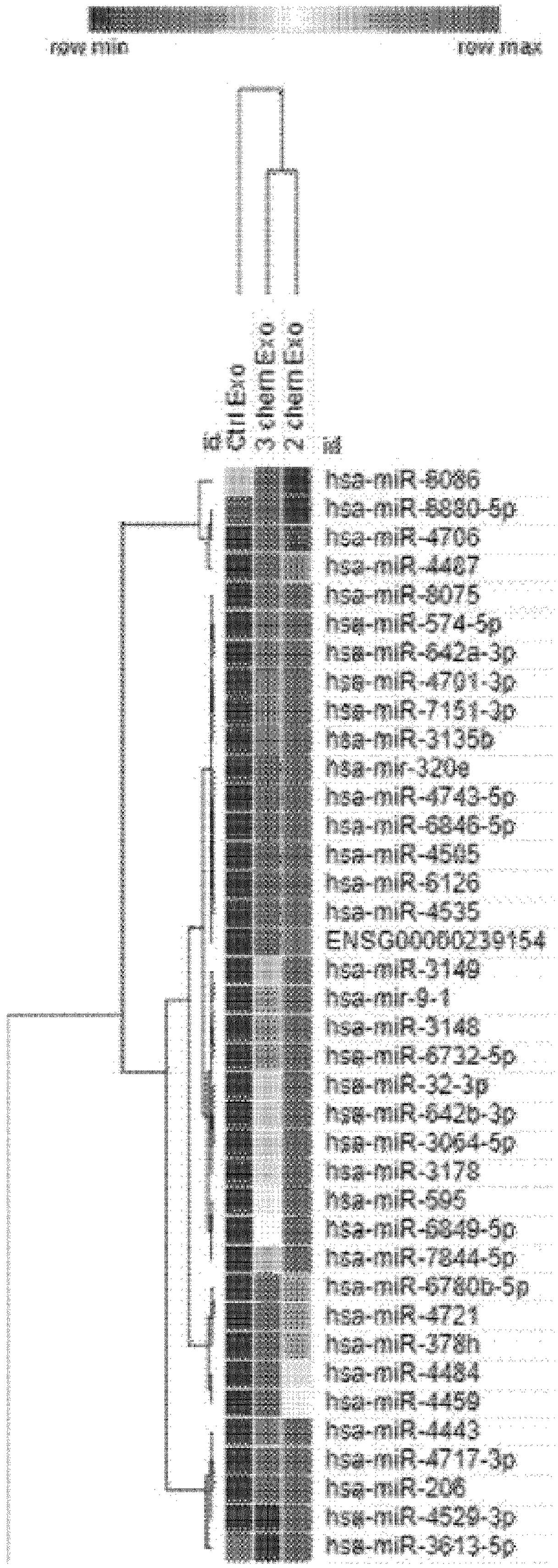
row min
row max
id
Ctrl Exo
3 chem Exo
2 chem Exo
id
hsa-miR-6086
hsa-miR-6880-5p
hsa-miR-4706
hsa-miR-4487
hsa-miR-8075
hsa-miR-574-5p
hsa-miR-642a-3p
hsa-miR-4701-3p
hsa-miR-7151-3p
hsa-miR-3135b
hsa-mir-320e
hsa-miR-4743-5p
hsa-miR-6846-5p
hsa-miR-4505
hsa-miR-6126
hsa-miR-4535
ENSG00000239154
hsa-miR-3149
hsa-mir-9-1
hsa-miR-3148
hsa-miR-6732-5p
hsa-miR-32-3p
hsa-miR-642b-3p
hsa-miR-3064-5p
hsa-miR-3178
hsa-miR-595
hsa-miR-6849-5p
hsa-miR-7844-5p
hsa-miR-6780b-5p
hsa-miR-4721
hsa-miR-378h
hsa-miR-4484
hsa-miR-4459
hsa-miR-4443
hsa-miR-4717-3p
hsa-miR-208
hsa-miR-4529-3p
hsa-miR-3613-5p

**INTERNATIONAL SEARCH REPORT**

International application No.
**PCT/KR2023/004969**

**A. CLASSIFICATION OF SUBJECT MATTER**

**A61K 35/28**(2006.01)i; **A61K 31/7105**(2006.01)i; **A61K 38/19**(2006.01)i; **A61K 38/20**(2006.01)i; **A61K 45/06**(2006.01)i; **A61P 35/00**(2006.01)i; **C12N 5/073**(2010.01)i

According to International Patent Classification (IPC) or to both national classification and IPC

**B. FIELDS SEARCHED**

Minimum documentation searched (classification system followed by classification symbols)

A61K 35/28(2006.01); A23K 10/20(2016.01); A23K 50/40(2016.01); C12N 5/071(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 세포외 소포체(extracellular vesicles), 엑소좀(exosome), 줄기세포(stem cell), miRNA, mRNA, 사이토카인(cytokine), 지모산(zymosan), Polyinosinic-polycytidylic acid(Poly I:C), monophosphoryl lipid A(MPLA)

**C. DOCUMENTS CONSIDERED TO BE RELEVANT**

| Category* | Citation of document, with indication, where appropriate, of the relevant passages | Relevant to claim No. |
|---|---|---|
| | FITZGERALD, W. et al. A System of Cytokines Encapsulated in ExtraCellular Vesicles. Scientific Reports. 2018, vol. 8, document 8973, pp. 1-11. | |
| X | See abstract; pages 5 and 8; and figure 4. | 7-10 |
| Y | | 11,12,19 |
| A | | 1-6,16 |
| | ASGARPOUR, K. et al. Exosomal microRNAs derived from mesenchymal stem cells: cell-to-cell messages. Cell Communication and Signaling. 2020, vol. 18, document 149, pp. 1-16. | |
| Y | See abstract; page 7; and table 1. | 11,12,19 |
| | KR 10-2022-0004035 A (CODIAK BIOSCIENCES, INC.) 11 January 2022 (2022-01-11) | |
| A | See entire document. | 1-12,16,19 |
| | N`DIAYE, E. R. et al. Chemically Modified Extracellular Vesicles and Applications in Radiolabeling and Drug Delivery. Pharmaceutics. electronic publication 16 March 2022, vol. 14, document 653, pp. 1-15. | |
| A | See entire document. | 1-12,16,19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 August 2023** | **17 August 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
PCT/KR2023/004969

C. DOCUMENTS CONSIDERED TO BE RELEVANT

| Category* | Citation of document, with indication, where appropriate, of the relevant passages | Relevant to claim No. |
|---|---|---|
| A | MUNIR, J. et al. Therapeutic miRNA-Enriched Extracellular Vesicles: Current Approaches and Future Prospects. Cells. 2020, vol. 9, document 2271, pp. 1-18. See entire document. | 1-12,16,19 |
| A | KR 10-2022-0040092 A (REPUBLIC OF KOREA(ANIMAL AND PLANT QUARANTINE AGENCY)) 30 March 2022 (2022-03-30) See entire document. | 1-12,16,19 |

Form PCT/ISA/210 (second sheet) (July 2022)

**INTERNATIONAL SEARCH REPORT**

International application No.
**PCT/KR2023/004969**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/KR2023/004969**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18**
because they relate to subject matter not required to be searched by this Authority, namely:

Claim 18 pertains to a method for treatment of the human body, and thus pertains to a subject matter on which the International Searching Authority is not required to carry out an international search under the provisions of PCT Article 17(2)(a)(i) and PCT Rule 39.1(iv).

2. ☑ Claims Nos.: **14,15,17**
because they relate to parts of the international application that do not comply with the prescribed requirements to such an extent that no meaningful international search can be carried out, specifically:

Claims 14, 15 and 17 refer to multiple dependent claims not meeting the requirement of PCT Rule 6.4(a), and thus are unclear.

3. ☑ Claims Nos.: **13**
because they are dependent claims and are not drafted in accordance with the second and third sentences of Rule 6.4(a).

**INTERNATIONAL SEARCH REPORT**
**Information on patent family members**

International application No.
**PCT/KR2023/004969**

| Patent document cited in search report | Publication date (day/month/year) | Patent family member(s) | Publication date (day/month/year) |
|---|---|---|---|
| KR 10-2022-0004035 A | 11 January 2022 | AR 118484 A1 | 06 October 2021 |
| | | AU 2020-240135 A1 | 24 September 2020 |
| | | AU 2021-236763 A1 | 23 September 2021 |
| | | CA 3133311 A1 | 24 September 2020 |
| | | CA 3171623 A1 | 23 September 2021 |
| | | CN 114080232 A | 22 February 2022 |
| | | CO 2021013895 A2 | 17 January 2022 |
| | | EA 202192504 A1 | 18 February 2022 |
| | | EP 3941937 A2 | 26 January 2022 |
| | | EP 4121450 A2 | 25 January 2023 |
| | | IL 286556 A | 31 October 2021 |
| | | JP 2022-526127 A | 23 May 2022 |
| | | JP 2023-518414 A | 01 May 2023 |
| | | MX 2021011241 A | 19 January 2022 |
| | | SG 11202109702 QA | 28 October 2021 |
| | | TW 202100164 A | 01 January 2021 |
| | | US 2022-0031921 A1 | 03 February 2022 |
| | | US 2022-0168415 A1 | 02 June 2022 |
| | | WO 2020-191361 A2 | 24 September 2020 |
| | | WO 2020-191361 A3 | 29 October 2020 |
| | | WO 2021-189047 A2 | 23 September 2021 |
| | | WO 2021-189047 A3 | 04 November 2021 |
| | | WO 2022-040376 A2 | 24 February 2022 |
| | | WO 2022-040376 A3 | 14 April 2022 |
| KR 10-2022-0040092 A | 30 March 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SUN et al.** *Mol. Ther.*, 2010, vol. 18 (9), 1606-1614
- Remington's Pharmaceutical Sciences. 1995